# EUROPÄISCHE PATENTSCHRIFT

(11) **EP 3 338 756 B1**
(45) Veröffentlichungstag und Bekanntmachung des Hinweises auf die Patenterteilung: **26.02.2020**
(21) Anmeldenummer: 16205915.8
(22) Anmeldetag: 21.12.2016
(51) Int. Cl.: A61K 6/00, A61K 6/083

(54) **LAGERSTABILER KUNSTSTOFFMODIFIZIERTER GLASIONOMERZEMENT**
STORAGE-STABLE RESIN-MODIFIED GLASS IONOMER CEMENT
CIMENT AU VERRE IONOMÈRE STABLE AU STOCKAGE, MODIFIÉ PAR UNE RÉSINE

(43) Veröffentlichungstag der Anmeldung: 27.06.2018
(73) Patentinhaber: VOCO GmbH, 27472 Cuxhaven (DE)
(72) Erfinder: Schmücker, Simon, 27472 Cuxhaven (DE); Plaumann, Manfred Thomas, 27472 Cuxhaven (DE)
(74) Vertreter: Eisenführ Speiser

(56) Entgegenhaltungen:
- US-A- 5 501 727
- US-A1- 2007 203 257
- US-A1- 2010 048 628
- US-A1- 2014 213 686
- US-B2- 7 488 762

## Beschreibung

Die vorliegende Erfindung betrifft eine Zusammensetzung zur Verwendung als Komponente eines mehrkomponentigen kunststoffmodifizierten Glasionomerzements, einen mehrkomponentigen kunststoffmodifizierten Glasionomerzement sowie ein Verfahren zur Herstellung entsprechender Zusammensetzungen und gehärteter Dentalmaterialien. Die Erfindung betrifft ferner die entsprechende Verwendung einer oder mehrerer Verbindungen (S) ausgewählt aus der Gruppe bestehend aus Sulfinsäuren, Salzen der Sulfinsäuren und Salzen bororganischer Verbindungen.

Mehrkomponentige kunststoffmodifizierte Glasionomerzemente werden zur Füllung restaurationsbedürftiger permanenter Zähne, insbesondere zur Füllung von Milchzähnen, zur Unterfüllung, zum Aufbau von Stümpfen, zur Überkappung der Pulpa, zur Versiegelung, in der Endodontie, zur Herstellung künstlicher Zahnkronen, in der Alterszahnheilkunde und speziell auch zur Befestigung dentaler und kieferorthopädischer Vorrichtungen eingesetzt. Die Erfindung betrifft derartige mehrkomponentigen kunststoffmodifizierten Glasionomerzemente sowie Verfahren zur Herstellung und zur Verwendung dieser dentalen Massen sowie die aus den Massen hergestellten Polymerisate (d.h. gehärteten Dentalmaterialien).

Mehrkomponentige kunststoffmodifizierte Glasionomerzemente (nachfolgend auch als "kunststoffmodifizierte Glasionomerzemente" bezeichnet) sind aus dem Stand der Technik bekannt; sie werden z.B. durch Beigabe von Kunststoffkomponenten zu konventionellen Glasionomerzementen dargestellt.

Ursprüngliches Ziel bei der Entwicklung kunststoffmodifizierter Glasionomerzemente war die Verbesserung der physikalischen Eigenschaften und eine Vereinfachung der Handhabung herkömmlicher Glasionomerzemente. Konventionelle Glasionomerzemente weisen gegenüber Kompositmaterialien niedrigere Abrasions- und Zugfestigkeiten, ein schlechteres ästhetisches Erscheinungsbild sowie eine hohe Anfälligkeit für Verarbeitungsfehler auf. Insbesondere sind Glasionomerzemente während ihrer Anwendung sehr empfindlich gegenüber Feuchtigkeit: Gelangt der Zement in einer frühen Stufe seiner Härtung in Kontakt mit einem Übermaß an Feuchtigkeit, wie beispielsweise mit Speichel, dann wird der Fortgang der Abbindung gestört, so dass die physikalischen Eigenschaften des gehärteten Zements verschlechtert sind. Insbesondere können die abgebundenen Produkte dann eine erhöhte Löslichkeit im feuchten Milieu des Mundes aufweisen. Um diese Nachteile auszugleichen, wurden Mischformen entwickelt, bei denen die klassischen Inhaltsstoffe der bekannten Glasionomerzemente um Elemente dentaler Kunststoffe ergänzt wurden.

Die so entstandenen kunststoffmodifizierten Glasionomerzemente besitzen eine deutlich reduzierte Löslichkeit und bessere mechanische Eigenschaften als herkömmliche Glasionomerzemente. Ein wesentlicher Aspekt bei dieser Verbesserung ist die rasche Härtung und die hydrophobere Natur des Dentalmaterials.

Bei der Härtung der kunststoffmodifizierten Glasionomerzemente reagiert zunächst die Monomermischung in einer schnellen Vernetzung durch eine radikalische Reaktion. Auf diesem Weg wird die Löslichkeit der verbleibenden Zementbestandteile erheblich gemindert, da diese in die Polymermatrix eingeschlossen werden, und das Material erhält eine erste "Härtestufe". Durch die beginnende Verfestigung des Materials wird das Glasionomersystem feuchtigkeitsunempfindlicher und kann dann, kinetisch langsamer, ungestört weiter durch die Säure-Base Reaktion des Glasionomers abbinden. Hierbei reagiert (analog zu den Vorgängen in konventionellen Glasionomerzementen) ein carbonsäuregruppenhaltiges Polymer mit einer basischen Glaszusammensetzung. Diese basische Glaszusammensetzung wird auch als "reaktives Glaspulver" bezeichnet. Der Begriff "reaktives Glaspulver" bedeutet, dass das basische Glas "reaktiv" gegenüber einer Säurefunktion ist. Die Säure-Base Reaktion erfordert immer die Anwesenheit von Wasser. Bei kunststoffmodifizierten Glasionomerzementen liegt der kunststoffmodifizierte Anteil im Gesamtsystem quantitativ in sehr viel geringerer Menge vor als der Glasionomerzementanteil. Die Eigenschaften dieser hybriden Systeme werden somit in erster Linie vom eingesetzten Glasionomer dominiert.

Mehrkomponentige kunststoffmodifizierte Glasionomerzemente sind üblicherweise zweikomponentig formuliert und werden beispielsweise als Paste/Paste Systeme oder in der Form Flüssigkeit/Paste angewendet. Dies trifft auch für erfindungsgemäße mehrkomponentige kunststoffmodifizierte Glasionomerzemente zu.

Die Anwendung der Zemente in der Form Paste/Paste, speziell im Verhältnis 1:1, aber auch in weiter abgestuften Verhältnissen (z.B. 1:2, 1:3, 1:4 oder 1:10) stellt an die Formulierung dieser Systeme höchste Anforderungen, insbesondere an die Aufteilung der unterschiedlichen Bestandteile des zweikomponentigen kunststoffmodifizierten Glasionomerzements auf die beiden Komponenten. Anders als in den Systemen Pulver/Flüssigkeit, Pulver/Paste oder Paste/Flüssigkeit ermöglichen Paste/Paste Systeme, ein Höchstmaß an chemischen Wechselwirkungen zwischen den Bestandteilen der jeweiligen Pasten, die in der Regel zu einer Destabilisierung der Systeme mit der Zeit führen, so dass die Gebrauchsdauer der reinen Paste/Paste Systeme gegenüber anderen Anwendungsformen oftmals stark eingeschränkt ist. Die enorme Komplexität dieser Zusammensetzungen wird u.a. durch die Initiatorkomponenten, die oftmals eine Vielzahl an Bestandteilen aufweisen, mitverursacht.

Die Härtungsreaktion des wasserlöslichen carbonsäuregruppenhaltigen Polymers mit der basischen Glaszusammensetzung wird in dem Moment eingeleitet, in dem die Komponenten miteinander vermischt werden. Die Reaktion (auch als GIZ-Reaktion oder GIZ-Abbindung bezeichnet, wobei "GIZ" für Glasionomerzement steht) erfolgt dabei formal als Neutralisation der Säuregruppen des wasserlöslichen carbonsäuregruppenhaltigen Polymers mit der basischen Glaszusammensetzung. Das Glas kann beispielsweise ein Strontiumaluminiumfluorsilikatglas sein.

Für den Fall, dass die radikalische Polymerisation kunststoffmodifizierter Glasionomerzemente chemisch erfolgt, beginnen der GIZ-Abbindemechanismus und die redoxinitiierte Polymerisation zeitlich parallel, wobei die radikalische Reaktion kinetisch sehr viel schneller abläuft.

Für den Fall, dass die radikalische Polymerisation kunststoffmodifizierter Glasionomerzemente durch Licht induziert wird, beginnt die Polymerisation zeitlich in dem Moment, in dem eine geeignete Lichtquelle die Monomermischung aktiviert. Auch hierbei läuft die radikalische Vernetzung sehr viel schneller als die GIZ-Abbindung ab.

Für den Fall, dass die radikalische Polymerisation kunststoffmodifizierter Glasionomerzemente sowohl durch Licht als auch chemisch erfolgt, weist das System drei unterschiedliche Härtungsmechanismen mit drei unterschiedlichen kinetischen Abläufen auf.

Die WO 88/05651 beschreibt einen kunststoffmodifizierten radioopaken Glasionomerzement als Unterfüllungsmaterial vom Typ Paste/Paste, der entweder chemisch oder mittels Licht gehärtet wird.

In der US 5,154,762 wird ein kunststoffmodifizierter Glasionomerzement offenbart, der 3-fach in einer Glasionomerzementreaktion, in einer chemisch induzierten Vernetzung und in einer Photopolymerisation härtet.

In der DE 39 41 629 C2 wird über einen kunststoffmodifizierten Glasionomerzement vom Typ Pulver/Flüssigkeit, Paste/Flüssigkeit oder Paste/Paste berichtet, der chemisch oder photochemisch härtet und grenzflächenaktive Mittel enthält.

Die US 7,173,074 B2 beschreibt chemisch härtbare, zweikomponentige, kunststoffmodifizierte Glasionomerzemente vom Typ Pulver/Flüssigkeit und Paste/Paste.

Die US 2006/0030637 A1 schlägt chemisch härtende, zweikomponentige, kunststoffmodifizierte Glasionomerzemente vor, denen auch Photoinitiatoren zugegeben werden können. Die Zemente können vom Typ Pulver/Flüssigkeit, Paste/Flüssigkeit oder Paste/Paste sein. Durch das Vorhandensein von Salzen sollen sie bessere Lagerstabilitäten aufweisen.

Die DE 10 2006 019 092 A1 betrifft chemisch härtende, zweikomponentige, kunststoffmodifizierte Glasionomerzemente vom Typ Paste/Paste.

In der US 2005/0252414 A1 findet sich der Vorschlag, die Ästhetik eines kunststoffmodifizierten Glasionomerzements, der chemisch und/oder photochemisch härtbar ist, durch Beigabe eines Nanofüllstoffs zu verbessern. Durch die Gegenwart des Nanofüllstoffs soll der Brechungsindex der flüssigen Phase erhöht werden und so zu einem besseren Angleich der Brechungsindices der Matrixphase mit der festen Phase führen. Dieser Angleich soll dazu führen, dass das gehärtete Material eine verbesserte optische Transluzenz aufweist.

Die DE 602 09 714 T2 beschreibt ebenfalls kunststoffmodifizierte Glasionomerzemente.

Die DE 195 26 224 B4 offenbart kunststoffmodifizierte Glasionomerzemente vom Typ Paste/Paste und Pulver/Flüssigkeit, die sowohl chemisch als auch photochemisch härtbar sind. Die Systeme sollen stark verbesserte physikalische Eigenschaften wie Anfangshärte, Biegefestigkeit und Adhäsionsfestigkeit an Dentin im Vergleich zu konventionellen Glasionomerzementen aufweisen. Lichtgehärtete Zementzusammensetzungen sollen selektiv für Füllungszwecke und chemisch härtende Zusammensetzungen selektiv für Befestigungszwecke einsetzbar sein.

In US 5,965,632 werden kunststoffverstärkte Glasionomerzemente vom Typ Paste/Paste offenbart.

Die oben beschriebenen kunststoffmodifizierten Glasionomerzemente weisen gegenüber den klassischen Glasionomerzementen einige verbesserte Eigenschaften wie Druckhärte, Biegefestigkeit und Löslichkeit auf.

Von besonderer Bedeutung bei einem kunststoffmodifizierten Glasionomerzement ist sein Initiatorsystem zur chemischen Härtung. Die spezielle Herausforderung hierbei wird durch den Umstand begründet, dass kunststoffmodifizierte Glasionomerzemente ein saures Milieu aufweisen und eine Vielzahl chemischer Stoffe aus den Redoxsystemen im Sauren nicht stabil sind und ihre Funktionsfähigkeit verlieren. Eine ganz spezielle Herausforderung stellen dabei wässrige saure Systeme dar.

In der Regel benutzt man in der Dentalchemie für chemisch härtende Massen das Initiatorsystem "Benzoylperoxid/aromatisches tertiäres Amin". Soll dieses Initiatorsystem jedoch im sauren Milieu eingesetzt werden, wie es bei kunststoffmodifizierten Glasionomerzementen vorliegt, versagt die Wirksamkeit der Redoxpartner. Kommt das aromatische tertiäre Amin in Kontakt mit leichten oder starken Säurefunktionen, wird die Verbindung protoniert und verliert ihre Funktionstüchtigkeit als Reduktionsmittel. Zudem wird das Benzoylperoxid unter sauren Bedingungen leicht abgebaut. Dies sind die Gründe, warum man für chemisch härtende dentale Zusammensetzungen schon früh auf die sogenannten "Bredereck"-Systeme zurückgriff.

Das klassische "Bredereck-System" wurde bereits 1964 angemeldet (DE 1495520), nachdem weitgehende Untersuchungen in der Arbeitsgruppe von Bredereck zur redoxinitiierten Vernetzung ungesättigter Verbindungen in den 40er und 50er Jahren (US 2,779,751, US 2,776,952, US 2,846,418, US 2,935,489 und US 2,894,932) erfolgreich abgeschlossen wurden.

Seit diesen Anfängen wurden Redoxsysteme, die unter sauren Bedingungen stabil sind, immer konsequenter erweitert.

Die DE 101 24 029 A1 offenbart ein Initiatorsystem für saure Dentalformulierungen, umfassend eine Barbitursäure oder Thiobarbitursäure bzw. ein Barbitursäure- oder Thiobarbitursäurederivat, eine Peroxodisulfatverbindung und/oder Peroxodiphosphatverbindung, eine Sulfinsäureverbindung und eine Kupferverbindung.

Die DE 197 57 277 A1 beschreibt ebenfalls ein dentales Initiatorsystem zur Härtung saurer, zweikomponentiger Massen. Der Initiator setzt sich hier aus einem Kupfersalz, einer Sulfinsäureverbindung und einem, Barbitur- bzw. Thiobarbitursäurederivat zusammen. In diesem System aus Pulver/Flüssigkeit befinden sich Reduktions- und Oxidationsmittel in der Pulverkomponente, während das Kupfersalz Bestandteil der flüssigen Phase ist.

Die DE 100 17 188 A1 beansprucht zweikomponentige Dentalmassen mit niedriger Abbindetemperatur, wobei die Komponente I: a.) mindestens einen Vinylether, b.) mindestens ein ethylenisch ungesättigtes Monomer, das kein Vinylether ist, c.) mindestens einen Beschleuniger, d.) Füllstoffe, Thixotropie-Hilfsmittel und andere Hilfsstoffe und Komponente II: e.) mindestens ein Barbitursäurederivat und/oder Malonylsulfamid, das die radikalische Polymerisation auslösen kann, f.) optional Füllstoffe, Thixotropie-Hilfsmittel, Verzögerer und anderer Hilfsstoffe sowie g.) übliche Weichmacher enthält.

Als Beschleuniger gemäß Bestandteil c.) ist der Einsatz von Schwermetallverbindungen und insbesondere von Kupferkomplexen sowie ionogen gebundenen Halogenen oder Pseudohalogenen wie organische Ammoniumchloride vorgesehen. In den Beispielen wird die 1-Benzyl-5-phenylbarbitursäure als Reduktionsmittel, das β-(Phenylethyl)-dibutylammoniumchlorid und der (Bis-(1-Phenylpentan-1,3-dionato)-kupfer(II)) Komplex als Beschleuniger in dem Paste-Paste System eingesetzt. Das Reduktionsmittel ist Teil der einen Paste, während Schwermetallkomplex und ionogene Halogenverbindung Teile der anderen Paste sind. Diese erfindungsgemäße Zusammensetzung härtet ohne Peroxid.

In der DE 199 28 238 A1 wird eine polymerisierbare Dentalmasse vorgeschlagen, die enthält a.) mindestens ein bi- oder höherfunktionelles ethylenisch ungesättigtes Monomer, b.) optional mindestens ein monofunktionelles ethylenisch ungesättigtes Monomer, c.) optional ein Beschleuniger, d.) ein Redoxinitiatorsystem, das die radikalische Polymerisation auslösen kann, e.) optional Füllstoffe, Thixotropie-Hilfsmittel, Verzögerer und andere Hilfsstoffe, f.) einen üblichen Weichmacher und dadurch gekennzeichnet ist, dass das Redoxinitiatorsystem ein Barbitursäurederivat und/oder ein Malonylsulfamid und ein organisches Peroxid, ausgewählt aus der Gruppe der ein- oder mehrfunktionellen Carbonsäureperoxyester, umfasst.

Auch hier wird in den Beispielen die 1-Benzyl-5-phenylbarbitursäure als Reduktionsmittel, das β-(Phenylethyl)-dibutylammoniumchlorid und der (Bis-(1-Phenylpentan-1,3-dionato)-kupfer(II)) Komplex als Beschleuniger in dem Paste-Paste System eingesetzt. Zum Reduktionsmittel kann hier der 3,5,5-Trimethylhexansäure-tert. butylperoxyester als Oxidationsmittel zugefügt werden, ohne dass das System seine Stabilität verliert. Die ionogene Halogenverbindung und der Schwermetallkomplex als Teile des Redoxsystems sind Bestandteile der anderen Paste. Diese Dentalmassen sollen sich als Füllungsmaterialien, Stumpfaufbaumaterialien, Befestigungszemente sowie als provisorische Kronen- und Brückenmaterialien eignen.

Um zu verbesserten Stabilitäten der Initiatorsysteme zu gelangen, wurden in den Bredereck Systemen statt der Säuren die entsprechenden Salze verwendet. Gemäß der alten Chemikerregel, dass "das Salz einer schwachen Säure bei Zusatz einer starken Säure das Salz der starken Säure ergibt und die schwache Säure freigesetzt wird", sollen mit diesem Konzept insbesondere lagerstabile 1:1 Paste/Paste Systeme auf der Grundlage CH-acider Verbindungen als Initiatoren in chemisch härtenden Dentalmaterialien erhalten werden. Die Salze CH-acider Verbindungen sollen in Gegenwart polymerisationsfähiger monomerer Bausteine beständig sein.

Beispiele hierfür finden sich in EP 1 872 767 A1, EP 2 070 506 A1, EP 2 055 324 A2, EP 2 198 824 A1, EP 0 923 924 A2, DE 11 2006 001 049 T5, EP 1 502 569 A1, EP 2 070 935 A1 oder in EP 1 881 010 A1.

Trotz dieser großen Anzahl an Schutzrechtsanmeldungen gibt es - unseres Wissens - bis heute kein entsprechendes Produkt im Markt.

Die EP 1 269 968 A1 offenbart einen dentalen Zement, der umfasst a.) ein polymerisierbares Monomer mit Säuregruppe, b.) eine Polyalkensäure, c.) einen Ionen abgebenden Füllstoff, d.) ein polymerisierbares Monomer ohne Säurefunktion, e.) Wasser, f.) ein Peroxid, g.) ein Salz einer aromatischen Sulfinsäure und h.) ein aromatisches sekundäres oder tertiäres Amin. Oxidations- und Reduktionsmittel liegen in diesen zweikomponentigen Systemen getrennt vor, wobei das Benzoylperoxid als Oxidationsmittel in der flüssigen, wässrigen Phase zusammen mit der sauren Polyacrylsäure formuliert wurde. Es ist daher zu erwarten, dass das System keine große Lagerstabilität aufweist.

Die US 2003/0166740 A1 beschreibt ebenfalls chemisch härtende, zweikomponentige, kunststoffmodifizierte Glasionomerzemente in der Form Pulver/Flüssigkeit und Paste/Paste, die ohne Konditionierung an der Zahnhartsubstanz aufgebracht werden können. Die Palette tauglicher Reduktionsmittel wird hier um polymerisationsfähige Harnstoff- und Thioharnstoffverbindungen erweitert. Es wird ausgeführt (vgl. Paragraph [0077]), dass in solchen Systemen Reduktions- und Oxidationsmittel in getrennten Teilen zu formulieren sind. Man kann sie gemeinsam in einen Teil geben, falls man die Technik der Mikroverkapselung anwendet (siehe hierzu auch die US 5,154,762).

Die EP 2 371 346 A1 offenbart ein Paste/Paste System zur Zahnwiederherstellung, das besonders gute Lagerstabilität aufweisen soll. Das Redoxsystem umfasst eine Peroxid- und eine Ascorbinsäureverbindung.

Die WO 95/22955 A1 beschreibt dentale Zusammensetzungen vom Paste/Paste Typ, umfassend ethylenisch ungesättigte Funktionen mit verbesserter Farbstabilität. Die Zusammensetzungen umfassen ein Oxidationsmittel sowie metallkomplexierte Ascorbinsäure.

Die WO 2016/007453 A1 ist auf dentale Zusammensetzungen gerichtet und offenbart ein Redoxsystem, umfassend eine Ascorbinsäureverbindung, ein Übergangsmetall (bevorzugt Kupfer oder Eisen) und organische Peroxide (bevorzugt Hydroperoxide oder Diperoxide).

Die EP 2 712 888 A1 zielt auf dentale härtbare Massen, die auch in sauren, nichtwässrigen Zusammensetzungen vorliegen können. Das hier verwendete Redoxsystem umfasst ein organisches Peroxid als Oxidationsmittel, ein Cystein als Reduktionsmittel sowie einen Polymerisationsbeschleuniger. Letzteres können Kupferverbindungen, beispielsweise Kupfersulfat, Kupferacetylaceton, Kupferchlorid, Kupfergluconat, Kupferoleat oder Vanadiumverbindungen wie Vanadiumbenzoylacetonat, Vanadiumnaphthenat, Vanadylacetylacetonat sein.

Die EP 2 433 612 A1 beansprucht kunststoffmodifizierte Glasionomerzement- zusammensetzungen, die als Redoxsystem die Kombination Cystein - Sulfinsäurederivat verwenden.

Vielfach beansprucht wird die Kombination Peroxid - Thioharnstoff, gegebenenfalls in Verbindung mit einem Polymerisationsbeschleuniger, in der Regel einer organischen Übergangsmetallverbindung.

So wird in der EP 1 458 831 A2 eine dentale Zusammensetzung beansprucht, die ein polymerisierbares Reduktionsmittel mit einer Allylthioharnstoffgruppe (beispielsweise 1-Allylthioharnstoff), ein Oxidationsmittel (beispielsweise Peroxid) und ein sekundäres Reduktionsmittel (beispielsweise ein Amin, ein(e) Ascorbinsäure(derivat), ein(e) Sulfinsäure(derivat) oder ein Metallsalz als Redoxsystem umfasst. Das System kann auch ein kunststoffmodifizierter Glasionomerzement sein und in der Form eines Paste/Paste-, Paste/Flüssigkeit- oder Pulver/Flüssigkeit-Systems verwendet werden.

In der EP 2 233 544 A1 wird ein lagerstabiler kunststoffmodifizierter Glasionomerzement als Paste/Paste-System offenbart, der ein Redoxsystem auf der Basis eines Hydroperoxids, eines Thioharnstoffderivats und einer Vanadiumverbindung umfasst.

Die EP 1 754 465 A1 trägt den Titel "2-Komponenten-Initiatorsystem (aminfrei) mit Lagerstabilität und besonderer Eignung für acide Systeme". Das Redoxinitiatorsystem dieser aminfreien Zusammensetzung umfasst eine Hydroperoxid-Verbindung mit einer oder mehreren Hydroperoxidgruppen, die an einen tertiären Kohlenstoff gebunden sind, ein Thioharnstoff-Derivat sowie eine in der Zusammensetzung lösliche Kupfer-Verbindung. Die Dentalzusammensetzung kann als System Flüssigkeit/Flüssigkeit, Paste/Paste, Paste/Flüssigkeit oder Pulver/Flüssigkeit formuliert werden und findet Verwendung zur Herstellung einer restorativ, orthodontisch oder endodontisch nutzbaren Zubereitung.

Das Dokument WO 95/22956 offenbart einen lichthärtbaren kunststoffmodifizierten Glasionomerzement in der Form Paste/Paste. Die offenbarten Glasionomerzemente können zusätzlich zu der Lichthärtung auch auf anderen Wegen gehärtet werden, nämlich insbesondere mit Hilfe eines Redoxkatalysatorsystems. WO 95/22956 offenbart insoweit eine Liste bevorzugter Reduktionsmittel, die Bestandteil des Redoxkatalysatorsystems sein können (vgl. Seite 19, erster Absatz, sowie Anspruch 15).

Eine primäre Aufgabe der vorliegenden Erfindung war es, eine Zusammensetzung zur Verwendung als Komponente eines mehrkomponentigen kunststoffmodifizierten Glasionomerzements sowie einen entsprechenden mehrkomponentigen kunststoffmodifizierten Glasionomerzement zur Verfügung zu stellen, die gegenüber aus dem Stand der Technik bekannten Systemen eine höhere Lagerstabilität aufweisen und vorzugsweise auch nach mehrwöchiger Lagerung mit allenfalls geringfügig veränderten Abbindezeiten (auch Aushärtezeiten genannt) zu gehärteten Dentalmaterialien verarbeitet werden können, welche gute mechanische Eigenschaften besitzen, insbesondere eine hohe Biegefestigkeit.

Insbesondere war es die Aufgabe der vorliegenden Erfindung, eine Zusammensetzung zur Verwendung als Komponente eines mehrkomponentigen kunststoffmodifizierten Glasionomerzements anzugeben, die im Vergleich mit entsprechenden Zusammensetzungen, die als Reduktionsmittel eine oder mehrere Verbindungen (A) ausgewählt aus der Gruppe bestehend aus den Isomeren der Ascorbinsäure, den Salzen der Isomere der Ascorbinsäure, den Estern der Isomere der Ascorbinsäure und den Ethern der Isomere der Ascorbinsäure umfassen, eine erhöhte Lagerstabilität besitzt. Die genannten Verbindungen (A) sind nämlich zwar als wirksame und in der Praxis besonders relevante Bestandteile von Redox-Initiatorsystemen in Komponenten kunststoffmodifizierter Glasionomerzemente weit verbreitet, führen aber nachteiligerweise regelmäßig zu vergleichsweise niedrigen Lagerstabilitäten dieser Komponenten.

Darüber hinaus war es eine weitere Aufgabe der vorliegenden Erfindung, ein Verfahren zur Herstellung solcher erfindungsgemäß anzugebenden Zusammensetzungen anzugeben. Weitere (Teil-)Aufgaben der vorliegenden Erfindung ergeben sich aus den beigefügten Patentansprüchen und der vorliegenden Beschreibung.

Die vorstehend genannten Aufgaben werden durch Zusammensetzungen, mehrkomponentige kunststoffmodifizierte Glasionomerzemente, Verfahren und Verwendungen gelöst, wie sie in den beigefügten Ansprüchen definiert sind. Bevorzugte erfindungsgemäße Ausgestaltungen ergeben sich aus den Unteransprüchen.

Insbesondere betrifft die vorliegende Erfindung eine Zusammensetzung zur Verwendung als Komponente eines mehrkomponentigen kunststoffmodifizierten Glasionomerzements, wobei die Zusammensetzung umfasst
- ein oder mehrere radikalisch polymerisierbare organische Monomere (M)
   sowie als Bestandteil eines Polymerisationsinitiatorsystems dafür
- eine oder mehrere Verbindungen (A) ausgewählt aus der Gruppe bestehend aus den Isomeren der Ascorbinsäure, den Salzen der Isomere der Ascorbinsäure, den Estern der Isomere der Ascorbinsäure und den Ethern der Isomere der Ascorbinsäure
   und
- eine oder mehrere Verbindungen (S) ausgewählt aus der Gruppe bestehend aus Sulfinsäuren, Salzen der Sulfinsäuren und Salzen bororganischer Verbindungen, zusätzlich umfassend eine basische Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C).

Die erfindungsgemäße Zusammensetzung umfasst radikalisch polymerisierbare organische Monomere (M) und zudem eine spezifische Kombination von Verbindungen (A) und (S), die gemeinsam als Reduktionsmittel eines Polymerisationsinitiatorsystems fungieren können und gemeinsam für eine hohe Lagerstabilität der erfindungsgemäßen Zusammensetzung verantwortlich sind. Die in der Zusammensetzung vorhandenen radikalisch polymerisierbaren organischen Monomere (M) polymerisieren in Gegenwart vieler herkömmlicher Reduktionsmittel regelmäßig schon während der Lagerung und somit schon vor dem Vermischen mit dem Oxidationsmittel des Polymerisationsinitiatorsystems (wie es üblicherweise in einer weiteren Komponente des mehrkomponentigen kunststoffmodifizierten Glasionomerzements vorliegt).

In umfangreichen eigenen Untersuchungen wurde festgestellt, dass die erfindungsgemäß vorgesehene Kombination von Verbindung (A) und (S) als Bestandteil eines Polymerisationsinitiatorsystems sowohl gegenüber konventionell etablierten als auch eher exotischen Initiatorsystemen überraschenderweise Vorteile hinsichtlich der Stabilitätseigenschaften bewirkt. Die erfindungsgemäßen Zusammensetzungen sind nicht nur selbst nach einer mehrwöchigen Lagerung noch überraschend stabil, sondern können auch dann noch zu Dentalmaterialien mit überraschend guten mechanischen Eigenschaften gehärtet werden, wobei die Abbindezeit (Aushärtezeit) gegenüber den initial hergestellten Zusammensetzungen nur in vergleichsweise geringem Maße verändert wird.

So erweist sich die erfindungsgemäße Zusammensetzung beispielsweise als vorteilhaft im Vergleich zu Zusammensetzungen, die neben radikalisch polymerisierbaren organischen Monomeren (M) ein tertiäres Amin, ein Sulfinat, eine Barbitursäure, ein tertiäres Amin in Kombination mit einem Sulfinat oder ein tertiäres Amin in Kombination mit einem Borat enthalten.

Diese überraschende Erkenntnis ist insbesondere für mehrkomponentige kunststoffmodifizierte Glasionomerzemente relevant, deren die Reduktionsmittel umfassende Komponente bislang regelmäßig eine unzureichende Lagerstabilität besitzt. Das Problem der geringen Lagerstabilität und das Bedürfnis, auch aus länger gelagerten Zusammensetzungen Dentalmaterialien mit guten mechanischen Eigenschaften herstellen zu können, besteht aber auch für andere dem Fachmann bekannte Dentalmaterialien. Die in erfindungsgemäßen Zusammensetzungen enthaltenen Kombinationen von Monomeren (M) mit Verbindungen (A) und (S) sind damit z.B. auch für solche anderen Mehrkomponentensysteme relevant, bei denen es sich nicht um Glasionomerzemente handelt, die aber zumindest teilweise chemisch, d.h. mit Hilfe eines Redox- Polymerisationsinitiatorsystems, gehärtet werden. Insbesondere sind hierbei Infiltranten, Fissurenversiegeler, Dentallacke, Dental-Kompositmaterialien und Dental-Compomermaterialien zu nennen.

Die "Komponenten" eines mehrkomponentigen kunststoffmodifizierten Glasionomerzements liegen separat, d.h. räumlich getrennt, von der oder den weiteren Komponenten vor.

Der Ausdruck "Isomere der Ascorbinsäure" umfasst L-Ascorbinsäure, D-Ascorbinsäure, L-Isoascorbinsäure und D-Isoascorbinsäure.

"Ester der Isomere der Ascorbinsäure" lassen sich erhalten durch die Reaktionsprodukte eines Isomers der Ascorbinsäure mit einer organischen Säure, insbesondere einer linearen oder verzweigten, gesättigten oder ungesättigten Carbonsäure mit 2 bis 26 Kohlenstoffatomen.

"Ether der Isomere der Ascorbinsäure" lassen sich erhalten durch die Reaktion eines Isomers der Ascorbinsäure mit einem Halogenkohlenwasserstoff, insbesondere eines linearen oder verzweigten, gesättigten oder ungesättigten Halogenkohlenwasserstoffs mit 1 bis 26 Kohlenstoffatomen.

Eine erfindungsgemäße Zusammensetzung umfasst zusätzlich eine basische Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C).

In eigenen Untersuchungen hat sich gezeigt, dass sich die Gegenwart der basischen Glaszusammensetzung in der erfindungsgemäßen Zusammensetzung nicht nachteilig auf die Lagerstabilität und Haltbarkeit der erfindungsgemäßen Zusammensetzung auswirkt. Diese Erkenntnis ist besonders relevant für die Konzeption eines kunststoffmodifizierten Glasionomerzements mit lediglich zwei separaten Komponenten; sie ermöglicht eine besonders günstige und einfache Handhabung und Verarbeitung der erfindungsgemäßen Zusammensetzung und eines entsprechenden kunststoffmodifizierten Glasionomerzements insgesamt. Zu entsprechenden erfindungsgemäßen kunststoffmodifizierten Glasionomerzementen sei auf die Ansprüche und die Ausführungen weiter unten verwiesen.

Der Fachmann auf dem Gebiet der mehrkomponentigen kunststoffmodifizierten Glasionomerzemente definiert die basische Glaszusammensetzung üblicherweise funktional über ihre Eignung bzw. Fähigkeit, carbonsäuregruppenhaltige Polymere (P) ausgewählt aus der Gruppe bestehend aus Homo- und Copolymeren einer α,β-ungesättigten Carbonsäure (C) in der GIZ-Abbindung zu vernetzen. Entsprechend umfasst die erfindungsgemäße Zusammensetzung eine "basische Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C)", die basische Glaszusammensetzung ist also als Vernetzer für diese Polymere geeignet. Hierbei ist die α,β-ungesättigte Carbonsäure (C) vorzugsweise ausgewählt aus der Gruppe bestehend aus Acrylsäure, Methacrylsäure, Itaconsäure, Fumarsäure, Maleinsäure, Chlormethacrylsäure, Cyanomethacrylsäure, Aconitsäure, Mesaconsäure, Glutaconsäure und Citraconsäure, besonders vorzugsweise ausgewählt aus der Gruppe bestehend aus Acrylsäure und Maleinsäure.

Bevorzugt ist eine erfindungsgemäße Zusammensetzung, wobei die basische Glaszusammensetzung (G) ein oder mehrere Kationen von Elementen umfasst, die ausgewählt sind aus der Gruppe bestehend aus den Metallen der Hauptgruppen I, II und III des Periodensystems, vorzugsweise ausgewählt sind aus der Gruppe bestehend aus Natrium, Kalium, Calcium und Aluminium und die besonders bevorzugt vorliegen in Form einer oder mehrerer Verbindungen ausgewählt aus der Gruppe bestehend aus Oxiden, Hydroxiden, Sulfaten, Nitraten, Phosphaten, Carbonaten, Silikaten, Fluoriden und Nitriden. Dies bedeutet, dass die basische Glaszusammensetzung (G) bevorzugt beispielsweise Kaliumnitrat, Natriumphosphat oder Aluminiumfluorid umfasst. Entsprechende basische Glaszusammensetzungen (G) sind insbesondere bevorzugt, weil diese säureaktiven Elemente sich relativ leicht in Gläser einbauen lassen und eine solche basischen Glaszusammensetzung bei der GIZ-Abbindung zu guten mechanischen Eigenschaften führen.

Bezüglich der einzusetzenden Mengenverhältnisse wird auf die Angaben in der DE 39 41 629, der DE 195 26 224 B4 und ähnlichen Dokumenten verwiesen, in denen auch gezeigt wird, wie die Eigenschaften des gehärteten Werkstoffs von Art und Menge der säurereaktiven Elemente (insbesondere der genannten Metallkationen) und ihrer Gegenionen abhängen. Die Freisetzung der säurereaktiven Elemente aus dem Glas, also die Reaktivität des Glases gegenüber den Säurefunktionen, wird durch den gezielten Einbau von Fehlstellen im Gitter des Glases herbeigeführt. Dies kann beispielsweise durch die Variation der eingesetzten Mengen an dreiwertigen Kationen gegenüber Si⁴⁺ erreicht werden. Eine andere Möglichkeit zur bewussten Generierung von Gitterfehlstellen im Glas stellt der Einsatz von Fluoridionen dar, da F⁻ gegenüber O²⁻ den Aufbau eines regulären Gitters stört. Ansonsten ist der Gehalt der säurereaktiven Elemente im Glas nicht spezifisch beschränkt.

Bevorzugt ist eine erfindungsgemäße Zusammensetzung (vorzugsweise wie vorstehend als bevorzugt bezeichnet), wobei die basische Glaszusammensetzung (G) ein Aluminiumfluorosilikatglas umfasst.

Vorzugsweise umfasst die basische Glaszusammensetzung (G) ein Aluminiumfluorosilikatglas, da sich in der Praxis mit diesem Glas besonders gute mechanische Eigenschaften für einen entsprechenden gehärteten kunststoffmodifizierten Glasionomerzement erhalten lassen, während gleichzeitig die Lagerstabilität einer entsprechenden erfindungsgemäßen Zusammensetzung (als separate Komponente) besonders wenig beeinträchtigt wird. Hauptkomponenten dieses Glases sind Al³⁺, Si⁴⁺, F⁻, O²⁻ sowie Ca²⁺ und/oder Sr²⁺- Ionen. Das Glas wird nach den bekannten Verfahren hergestellt. Beispielsweise verwendet man Siliziumdioxid, Aluminiumoxid, Aluminiumhydroxid, Aluminiumsilikat, Strontiumsilikat, Natriumfluorid, Aluminiumfluorid, Aluminiumphosphat, Natriumaluminiumfluorid und Strontiumphosphat als Ausgangsmaterialien. Das Rohmaterial wird gemischt und bei über 1000 °C geschmolzen, abgekühlt, gemahlen und nach Korngrößenfraktionen getrennt.

Bevorzugt ist eine erfindungsgemäße Zusammensetzung (vorzugsweise wie vorstehend als bevorzugt bezeichnet), wobei die basische Glaszusammensetzung (G) ein oder mehrere Gläser mit organisch modifizierter Oberfläche, vorzugsweise mit silanisierter Oberfläche, umfasst.

Bevorzugt kann die Oberfläche eines oder mehrerer Gläser in der basischen Glaszusammensetzung (G) organisch modifiziert sein. Durch eine geeignete Modifizierung wird die Kompatibilität des reaktiven Glaspulvers mit den anderen Bestandteilen der Zusammensetzung verbessert. Eine organische Oberflächenmodifizierung eines, mehrerer oder sämtlicher Gläser in der basischen Glaszusammensetzung (G) soll jedoch die Freisetzung der reaktiven Elemente nicht behindern. Bevorzugt ist für den Fall einer Oberflächenmodifizierung die Durchführung einer Silanisierung. Hierbei wird die Oberfläche des reaktiven Glaspulvers beispielsweise mit einer organischen Silanverbindung, die über eine polymerisierbare, ethylenisch ungesättigte Doppelbindung verfügt, überzogen. Bevorzugt eingesetzt wird Vinyltrimethoxysilan oder insbesondere Methacryloxypropyltrialkoxyysilan. Die Organosilanverbindungen können allein oder in Gemischen verwendet werden. Weitere Beispiele umfassen Silane, die keine ethylenisch ungesättigte Doppelbindung aufweisen, wie gamma-Aminopropyltrialkoxysilan, Propyltrialkoxysilan, etc.

Andere Oberflächenmodifizierungsmittel können Fettsäuren, organische Säuren, Tenside, anorganische Säuren, Polysiloxane, etc. sein.

Bevorzugt ist zusammenfassend eine erfindungsgemäße Zusammensetzung (vorzugsweise wie vorstehend als bevorzugt bezeichnet),
wobei die basische Glaszusammensetzung (G) ein oder mehrere Kationen von Elementen umfasst, die ausgewählt sind aus der Gruppe bestehend aus den Metallen der Hauptgruppen I, II und III des Periodensystems, vorzugsweise ausgewählt sind aus der Gruppe bestehend aus Natrium, Kalium, Calcium und Aluminium,
und die besonders bevorzugt vorliegen in Form einer oder mehrerer Verbindungen ausgewählt aus der Gruppe bestehend aus Oxiden, Hydroxiden, Sulfaten, Nitraten, Phosphaten, Carbonaten, Silikaten, Fluoriden und Nitriden
und/oder
wobei die basische Glaszusammensetzung (G) ein Aluminiumfluorosilikatglas umfasst
und/oder
wobei die basische Glaszusammensetzung (G) ein oder mehrere Gläser mit organisch modifizierter Oberfläche, vorzugsweise mit silanisierter Oberfläche, umfasst.

Bevorzugt ist eine erfindungsgemäße Zusammensetzung (vorzugsweise wie vorstehend als bevorzugt bezeichnet), zusätzlich umfassend ein oder mehrere Verbindungen (Z) ausgewählt aus der Gruppe bestehend aus Barbitursäuren, tertiären Aminen und sekundären Aminen.

Im Vergleich mit Zusammensetzungen, die als Reduktionsmittel lediglich ein oder mehrere Verbindungen (Z) umfassen, hat sich überraschenderweise gezeigt, dass die Lagerstabilität erfindungsgemäßer Zusammensetzungen, die, bei gleicher Gesamtstoffmenge des Reduktionsmittels, neben ein oder mehrere Verbindungen (Z) auch die Verbindungen (A) und (S) umfassen, verbessert ist. Dies ist besonders vorteilhaft, weil sich mit Hilfe der Verbindungen (Z) die Härtungskinetik eines erfindungsgemäßen kunststoffmodifizierten Glasionomerzements besonders gut an die jeweiligen Anforderungen anpassen lässt. Insbesondere werden die Verbindungen (Z) regelmäßig als sogenannte Beschleuniger für Lichthärtungsinitiatorsysteme in Kombination mit Photoinitiatoren eingesetzt, d.h. in Kombination mit Katalysatoren, die photosensibilisierend wirken.

Bevorzugt ist eine erfindungsgemäße Zusammensetzung (vorzugsweise wie vorstehend als bevorzugt bezeichnet), zusätzlich umfassend einen oder mehrere Photoinitiatoren, wobei der eine oder die mehreren Photoinitiatoren vorzugsweise ausgewählt sind aus der Gruppe bestehend aus Benzoinalkylether, Benzoinalkylester, Benzilmonoketale, Acylphosphinoxide, Benzophenone, Acetophenone, Ketalen, Thioxanthone, Titanocene, aliphatische 1,2-Diketoverbindungen und aromatische 1,2-Diketoverbindungen, besonders vorzugsweise ausgewählt ist aus der Gruppe umfassend 2,2-Diethoxyacetophenon, 9,10-Phenanthrenchinon, Diacetyl, Furil, Anisil, 4,4'-Dichlorbenzil und 4,4'-Dialkoxybenzil und Campherchinon.

Entsprechende erfindungsgemäße Zusammensetzungen sind insbesondere deshalb bevorzugt, weil für diese Zusammensetzungen neben der chemischen Härtung ein weiterer Härtungsmechanismus zur Verfügung steht, mit dem die Zusammensetzung gegebenenfalls auch allein, d.h. ohne das Vermischen mit einer weiteren Komponente, gehärtet werden kann. Besonders vorteilhaft ist, dass sich die Anwesenheit eines Photoinitiators nicht negativ auf die Lagerstabilität der erfindungsgemäßen Zusammensetzung auswirkt, sofern diese unter Lichtausschluss gelagert wird, es somit also nicht zu einer ungünstigen und unerwünschten Wechselwirkung zwischen den Verbindungen (A) und (S) mit dem Photoinititator kommt. Vorteilhaft ist, dass die Verbindungen (A) und (S) in synergistischer Art und Weise als Beschleuniger für die Photoinitiatoren wirken können und die Effektivität der bevorzugt in der erfindungsgemäßen Zusammensetzung enthaltenen Photoinitiatoren somit erhöhen.

Bevorzugt ist eine erfindungsgemäße Zusammensetzung (vorzugsweise wie vorstehend als bevorzugt bezeichnet), zusätzlich umfassend einen oder mehrere Inhibitoren, wobei der eine oder die mehreren Inhibitoren vorzugsweise ausgewählt sind aus der Gruppe bestehend aus substituierten Phenolen, Phenothiazin, stabile organische Radikale und Hydrochinonmonomethylether, besonders bevorzugt ausgewählt sind aus der Gruppe bestehend aus 2,6-Di-tert.-butyl-4-methylphenol, Hydrochinonmonomethylether, dem 2,2-Diphenyl-1-picrylhydrazylradikal, dem Galvinoxylradikal, dem Triphenylmethylradikal und dem 2,2,6,6-Tetramethylpiperidinyl-1-oxylradikal.

Die bevorzugt eingesetzten Inhibitoren sind dazu geeignet, eine vorzeitige radikalische Polymerisation der radikalisch polymerisierbaren organische Monomere (M) zu verhindern. Die Inhibitoren wirken somit unterstützend in Richtung der gleichen technischen Wirkung, die mit erfindungsgemäßen Zusammensetzungen erzielt wird. Vorteilhaft ist dabei, dass die Wirkung der Inhibitoren in erfindungsgemäßen Zusammensetzungen nicht vermindert ist, sondern die technische Wirkung der vorliegenden Erfindung mit dem Effekt der Inhibitoren additiv ist. Damit lassen sich besonders vorteilhafte und besonders lagerstabile Zusammensetzungen herstellen. Insbesondere bei Anwesenheit von Photoinitiatoren in der erfindungsgemäßen Zusammensetzung erlaubt es der Einsatz von Inhibitoren, die Lagerstabilität der Zusammensetzung weiter zu erhöhen.

Bevorzugt ist eine erfindungsgemäße Zusammensetzung (vorzugsweise wie vorstehend als bevorzugt bezeichnet), zusätzlich umfassend ein oder mehrere Additive ausgewählt aus der Gruppe bestehend aus Farbmitteln, Weinsäure, Kieselsäure, Stabilisatoren, Modifikatoren, bakterizid wirkenden Substanzen und inerten Füllstoffen.

Die erfindungsgemäßen Zusammensetzungen können als Farbmittel beispielsweise anorganische und organische Pigmente oder Farbstoffe umfassen. Beispiele hierfür werden in der DE 196 05 272 gegeben.

Die erfindungsgemäßen Zusammensetzungen können Weinsäure, insbesondere (+)Weinsäure, enthalten. In kunststoffmodifizierten Glasionomerzementen ist Weinsäure ein geeignetes Mittel zur Steuerung der Abbindereaktion zwischen der basischen Glaszusammensetzung (G) und dem carbonsäuregruppenhaltigen Polymer (P), das die Verarbeitungszeit verlängert, die Abbindungsgeschwindigkeit jedoch gleichzeitig beschleunigt.

Die erfindungsgemäßen Zusammensetzungen, insbesondere solche, die als Paste vorliegen, können Modifikatoren umfassen, die insbesondere bei der Herstellung der Zusammensetzung dazu beitragen, dass sich die verschiedenen Bestandteile sehr gut zu einer Paste verarbeiten lassen. Als vorteilhafte Modifikatoren können beispielsweise wasserlösliche Substanzen hohen Molekulargewichts wie Stärkederivate, Carboxyalkylzellulose, Polyvinylalkohol, Polyethylenglykol, Polyacrylamid, Polyvinylpyrrolidon, Xanthan Gum, Alginat, etc. eingesetzt werden. Durch den Einsatz von (vorteilhaften) Modifikatoren können die rheologischen Eigenschaften erfindungsgemäßer Zusammensetzungen (insbesondere Pasten) gezielt eingestellt und an die jeweiligen praktischen Anforderungen angepasst werden, ohne die Lagerstabilität negativ zu beeinflussen. Einen ähnlichen Zweck erfüllen die Stabilisatoren, bei denen es sich beispielsweise um Tenside handelt.

Die erfindungsgemäße Zusammensetzung umfasst vorteilhafterweise bakterizid wirkende Substanzen. Solche dem Fachmann geläufigen und käuflich zu erwerbenden Substanzen beeinträchtigen den für erfindungsgemäße Zusammensetzungen beobachteten technischen Effekt vorteilhafterweise nicht, sondern erhöhen im Gegenteil die Beständigkeit erfindungsgemäßer Zusammensetzungen gegenüber biologischen Einflussfaktoren, die die Lagerstabilität vermindern könnten.

Die erfindungsgemäße Zusammensetzung umfasst vorzugsweise bekannte anorganische Füllstoffe, die in breitem Umfang in dentalen Kompositwerkstoffen Verwendung finden und die dem Fachmann gut bekannt sind. Dies sind beispielsweise Barium-Aluminium-Borsilikatgläser unterschiedlicher Teilchengrößen, die nicht (wie die basische Glaszusammensetzung (G)) gegenüber einem carbonsäuregruppenhaltigen Polymer (P) ausgewählt aus der Gruppe bestehend aus Homo- und Copolymeren einer α,β-ungesättigten Carbonsäure (C) reaktiv sind. Solche Gläser lassen sich folglich als inerte anorganische Füllstoffe bezeichnen. Auch Quarz, Feldspat, Titandioxid und/oder Bariumsulfat können vorzugsweise als inerte anorganische Füllstoffe eingesetzt werden. Vorteilhaft an diesen inerten anorganischen Füllstoffen ist, dass sich der Feststoffanteil der Zusammensetzung erhöhen lässt, ohne die Kinetik der GIZ-Abbindung signifikant zu verändern. Darüber hinaus lassen sich mit den inerten anorganischen Füllstoffen die mechanischen Eigenschaften der Materialien einstellen, die durch Härtung aus den erfindungsgemäßen Zusammensetzungen erhalten werden. Vorteilhafterweise wirkt sich die Anwesenheit inerter anorganischer Füllstoffe nicht nachteilig auf die Lagerstabilität erfindungsgemäßer Zusammensetzungen aus.

Der Einsatz von Kieselsäure, insbesondere nanoskaliger nicht agglomerierter Kieselsäure Partikel mit Teilchengrößen von 1 bis 200 nm, die aus dem Sol-Gel Verfahren stammen, führt zu einer noch besseren Raumerfüllung der Füllkörper, so dass die Eigenschaften der durch Härtung aus den erfindungsgemäßen Zusammensetzungen resultierenden Werkstoffe im Vergleich zu den Produkten aus dem Stand der Technik noch vorteilhafter werden. Nanoskalige, nicht aggregierte Sole sind in einer großen Bandbreite käuflich zu erwerben. Bevorzugt können auch Füllstoffe bestimmter Geometrie, wie sie beispielsweise durch Sprühtrocknung von Solen erhalten werden, verwendet werden. Beispiele hierfür sind aggregierte Kieselsäureteilchen in der Form von Tori, wie sie in der WO 00/25729 oder in der DE 102 53 481 beschrieben sind. Weitere Beispiele für geeignete Kieselsäuren sind Aerosile, wie Aerosil 200, Aerosil OX 50, Aerosil R 972, Aerosil R 974, Aerosil R 8200, Aerosil R 711, Aerosil DT 4, etc.. Zu den Aerosilen gehören auch Aluminiumoxid C sowie Titandioxid P 25. Darüber hinaus können organische und/ oder organisch-anorganische Hybridfüllstoffe zum Einsatz kommen.

Die inerten anorganischen Füllstoffe und/oder die Kieselsäureteilchen können vorzugsweise oberflächenmodifiziert sein, so wie es vorstehend für die Gläser der basischen Glaszusammensetzung beschrieben ist.

Bevorzugt ist zusammenfassend demnach eine erfindungsgemäße Zusammensetzung (vorzugsweise wie vorstehend als bevorzugt bezeichnet),
- zusätzlich umfassend ein oder mehrere Verbindungen (Z) ausgewählt aus der Gruppe bestehend aus Barbitursäuren, tertiären Aminen und sekundären Aminen und/oder
- zusätzlich umfassend einen oder mehrere Photoinitiatoren,
   wobei der eine oder die mehreren Photoinitiatoren vorzugsweise ausgewählt sind aus der Gruppe bestehend aus Benzoinalkylether, Benzoinalkylester, Benzilmonoketale, Acylphosphinoxide, Benzophenone, Acetophenone, Ketalen, Thioxanthone, Titanocene, aliphatische 1,2-Diketoverbindungen und aromatische 1,2-Diketoverbindungen, besonders vorzugsweise ausgewählt ist aus der Gruppe umfas-send 2,2-Diethoxyacetophenon, 9,10-Phenanthrenchinon, Diacetyl, Furil, Anisil, 4,4'-Dichlorbenzil und 4,4'-Dialkoxybenzil und Campherchinon
   und/oder
- zusätzlich umfassend einen oder mehrere Inhibitoren,
   wobei der eine oder die mehreren Inhibitoren vorzugsweise ausgewählt sind aus der Gruppe bestehend aus substituierten Phenolen, Phenothiazin, stabile organische Radikale und Hydrochinonmonomethylether, besonders bevorzugt ausgewählt sind aus der Gruppe bestehend aus 2,6-Di-tert.-butyl-4-methylphenol, Hydrochinonmonomethylether, dem 2,2-Diphenyl-1-picrylhydrazylradikal, dem Galvinoxylradikal, dem Triphenylmethylradikal und dem 2,2,6,6-Tetramethylpiperidinyl-1-oxylradikal
   und/oder
- zusätzlich umfassend ein oder mehrere Additive ausgewählt aus der Gruppe bestehend aus Farbmitteln, Weinsäure, Kieselsäure, Stabilisatoren, Modifikatoren, bakterizid wirkenden Substanzen und inerten Füllstoffen.

Bevorzugt ist eine erfindungsgemäße Zusammensetzung (vorzugsweise wie vorstehend als bevorzugt bezeichnet), wobei das eine oder zumindest eines der mehreren radikalisch polymerisierbaren organischen Monomere (M)
- ausgewählt ist aus der Gruppe bestehend aus Monomeren mit mindestens zwei ethylenischen Gruppen (M1),
   wobei die Monomere mit mindestens zwei ethylenischen Gruppen (M1) vorzugsweise ausgewählt sind aus der Gruppe bestehend aus Diacrylaten, Triacrylaten, Dimethacrylaten, Trimethacrylaten, besonders vorzugsweise ausgewählt sind aus der Gruppe bestehend aus Ethylenglykoldimethacrylat, 1,6-Hexandioldimethacrylat, Triethylenglykoldimethacrylat, 1,12-Dodecandioldimethacrylat, ethoxyliertes Bisphenol-A-dimethacrylat, Polyethylenglykoldimethacrylat, 7,7,9-Trimethyl-4,13-dioxo-3,14-dioxa-5,12-diazahexadecan-1,16-dioxydimethacrylat, Butandioldimethacrylat, Trimethylolpropantrimethacrylat, Urethandimethacrylat, Glycerin-1,3-dimethacrylat, Tetraethylenglykoldimethacrylat, Neopentylglykoldimethacrylat, 2,2-Bis[4-[3-methacryloyloxy-2-hydroxypropoxy]phenyl]propan und den Di(meth)acrylaten des Dihydroxymethyltricyclo[5.2.1.0^{2,6}]decans
   und/oder
   wobei das eine oder zumindest eines der mehreren oder zumindest ein weiteres der mehreren radikalisch polymerisierbaren organischen Monomere (M)
- ausgewählt ist aus der Gruppe bestehend aus Hydroxylverbindungen mit mindestens einer ethylenischen Gruppe (M2),
   wobei die Hydroxylverbindungen mit mindestens einer ethylenischen Gruppe (M2) vorzugsweise ausgewählt sind aus der Gruppe bestehend aus 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat und 2,2-Bis[4-[3-methacryloyloxy-2-hydroxypropoxy]phenyl]propan.

Dies bedeutet für den Fall der "oder"-Verknüpfung, dass das eine oder zumindest eines der mehreren radikalisch polymerisierbaren organischen Monomere (M) ausgewählt ist aus der Gruppe bestehend aus Monomeren mit mindestens zwei ethylenischen Gruppen (M1) und Hydroxylverbindungen mit mindestens einer ethylenischen Gruppe (M2).
Vorzugsweise ist in diesem Fall somit das eine oder zumindest eines der mehreren radikalisch polymerisierbaren organischen Monomere (M) ausgewählt aus der Gruppe bestehend aus Ethylenglykoldimethacrylat, 1,6-Hexandioldimethacrylat, Triethylenglykoldimethacrylat, 1,12-Dodecandioldimethacrylat, ethoxyliertes Bisphenol-A-dimethacrylat, Polyethylenglykoldimethacrylat, 7,7,9-Trimethyl-4,13-dioxo-3,14-dioxa-5,12-diazahexadecan-1,16-dioxydimethacrylat, Butandioldimethacrylat, Trimethylolpropantrimethacrylat, Urethandimethacrylat, Tetraethylenglykoldimethacrylat, Neopentylglykoldimethacrylat, und den Di(meth)acrylaten des Dihydroxymethyltricyclo[5.2.1.0^{2,6}]decans, 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat und 2,2-Bis[4-[3-methacryloyloxy-2-hydroxypropoxy]phenyl]propan.
Ganz besonders bevorzugt sind solche erfindungsgemäßen Zusammensetzungen, in denen sämtliche der radikalisch polymerisierbaren organischen Monomere (M) ausgewählt sind aus der vorstehenden Liste.

Derartige erfindungsgemäße Zusammensetzungen sind bevorzugt, weil die entsprechenden radikalisch polymerisierbaren organischen Monomere (M) in der Praxis eine besondere Verträglichkeit mit den Verbindungen (A) und (S) zeigen und sich mit diesen Monomeren besonders lagerstabile Zusammensetzungen erhalten lassen. Darüber hinaus lassen sich entsprechende radikalisch polymerisierbare organische Monomere (M) mit einem Polymerisationsinitiatorsystem umfassend die Verbindungen (A) und (S) besonders effizient polymerisieren, ohne dass chemische Inkompatibilitäten auftreten. Somit führen entsprechende erfindungsgemäße Zusammensetzungen nach der Härtung vorteilhafterweise zu (erfindungsgemäßen, siehe die Ansprüche und die Ausführungen weiter unten) gehärteten Dentalmaterialien mit besonders guten mechanischen Eigenschaften.

Die obige Definition einer bevorzugten Ausgestaltung einer erfindungsgemäßen Zusammensetzung bedeutet für den Fall der "und"-Verknüpfung, die gegenüber der vorstehend beschriebenen "oder"-Verknüpfung bevorzugt ist, dass zumindest eines der mehreren radikalisch polymerisierbaren organischen Monomere (M) ausgewählt ist aus der Gruppe bestehend aus Monomeren mit mindestens zwei ethylenischen Gruppen (M1) oder der entsprechend bevorzugten Liste und zumindest eines der mehreren radikalisch polymerisierbaren organischen Monomere (M) ausgewählt ist aus der Gruppe bestehend aus Hydroxylverbindungen mit mindestens einer ethylenischen Gruppe (M2) oder der entsprechend bevorzugten Liste.

Während bestimmte Monomere beide Merkmale erfüllen, nämlich die Hydroxylverbindungen mit mindestens zwei ethylenischen Gruppen wie z.B. 2,2-Bis[4-[3-methacryloyloxy-2-hydroxypropoxy]phenyl]propan, ist es bevorzugt, dass zumindest eines der radikalisch polymerisierbaren organischen Monomere (M) ausgewählt ist aus der Gruppe bestehend aus Monomeren mit mindestens zwei ethylenischen Gruppen (M1) oder der entsprechend bevorzugten Liste und zumindest ein weiteres der radikalisch polymerisierbaren organischen Monomere (M) ausgewählt ist aus der Gruppe bestehend aus Hydroxylverbindungen mit mindestens einer ethylenischen Gruppe (M2) oder der entsprechend bevorzugten Liste, mit der Maßgabe, dass es sich bei den ausgewählten Verbindungen um verschiedene radikalisch polymerisierbare organische Monomere (M) handelt. Ganz besonders bevorzugt sind dabei solche erfindungsgemäßen Zusammensetzungen, in denen sämtliche der radikalisch polymerisierbaren organischen Monomere (M) ausgewählt sind aus der Gruppe bestehend aus Monomeren mit mindestens zwei ethylenischen Gruppen (M1) und Hydroxylverbindungen mit mindestens einer ethylenischen Gruppe (M2), oder den entsprechend bevorzugten Listen.

Derartige erfindungsgemäße Zusammensetzungen sind zunächst wiederum bevorzugt, weil die entsprechenden radikalisch polymerisierbaren organischen Monomere (M) der Gruppen (M1) und (M2) in der Praxis eine besondere Verträglichkeit mit den Verbindungen (A) und (S) zeigen und sich mit diesen Monomeren besonders lagerstabile Zusammensetzungen erhalten lassen.

Darüber hinaus weisen derartig bevorzugte erfindungsgemäße Zusammensetzungen mit zwei unterschiedlichen radikalisch polymerisierbaren organischen Monomeren (M), von denen zumindest eines ausgewählt ist aus der Gruppe bestehend aus Monomeren mit mindestens zwei ethylenischen Gruppen (M1) und zumindest eines ausgewählt ist aus der Gruppe bestehend aus Hydroxylverbindungen mit mindestens einer ethylenischen Gruppe (M2), oder den entsprechend bevorzugten Listen, eine überlegene Härtungskinetik auf und lassen sich auch nach mehrwöchiger Lagerung noch zu Dentalmaterialien mit guten mechanischen Eigenschaften härten. Dabei wurde überraschenderweise gefunden, dass die Abbindezeit (Aushärtezeit) derart bevorzugter erfindungsgemäßer Zusammensetzungen besonders wenig von der Lagerung beeinflusst wird.

Neben den vorstehend bereits als bevorzugt bezeichneten Monomeren mit mindestens zwei ethylenischen Gruppen (M1) sind in der Patentliteratur eine Vielzahl weiterer Verbindungen genannt, die allesamt Diester der Acryl- oder Methacrylsäure sind und sich zum Einsatz in einer erfindungsgemäßen Zusammensetzung eignen. Bevorzugt verwendet werden können die entsprechenden Dimethacrylate bzw. Diacrylate des Dihydroxymethyltricyclo[5.2.1.0^{2.6}]decans, wie sie in den Druckschriften DE 2816823, DE 2419887, DE 2406557, DE 2931926, DE 3522005, DE 3522006, DE 3703120, DE 102005021322, DE 102005053775, DE 102006060983, DE 69935794 und DE 102007034457 beschrieben sind.

Bevorzugt ist eine erfindungsgemäße Zusammensetzung (vorzugsweise wie vorstehend als bevorzugt bezeichnet), wobei die eine oder zumindest eine der mehreren Verbindungen (A) ausgewählt ist aus der Gruppe bestehend aus Ascorbinsäure, Salzen der Ascorbinsäure, Isoascorbinsäure und Salzen der Isoascorbinsäure, vorzugsweise ausgewählt ist aus der Gruppe bestehend aus Ascorbinsäure, Natriumascorbat, Isoascorbinsäure und Natriumisoascorbat
und/oder
wobei die eine oder zumindest eine der mehreren Verbindungen (S) ausgewählt ist aus der Gruppe bestehend aus aromatischen Sulfinsäuren, Salzen der aromatischen Sulfinsäuren und Salzen aromatischer bororganischer Verbindungen, vorzugsweise ausgewählt ist aus der Gruppe bestehend aus Toluolsulfinsäure, Natriumtoluolsulfinat, Kaliumtoluolsulfinat, Benzolsulfinsäure, Natriumbenzolsulfinat, Kaliumbenzolsulfinat, Natriumtetraphenylborat und Lithiumtetraphenylborat, besonders bevorzugt ausgewählt ist aus der Gruppe bestehend aus Toluolsulfinsäure, Natriumtoluolsulfinat, Kaliumtoluolsulfinat, Benzolsulfinsäure, Natriumbenzolsulfinat und Kaliumbenzolsulfinat.

Für derart bevorzugte erfindungsgemäße Zusammensetzungen werden besonders hohe Lagerstabilitäten sowie ein günstiges Abbindeverhalten, d.h. möglichst unveränderte Abbindezeiten (Aushärtezeiten), auch nach mehrwöchiger Lagerung erzielt. Die vorstehend definierten Verbindungen (A) sind dabei besonders effektive und leistungsstarke Reduktionsmittel, für die sich in eigenen Versuchen eine besonders vorteilhafte Wirkung in Kombination mit den Verbindungen (S) gezeigt hat, wobei die vorstehend definierten Verbindungen (S), die alleine häufig nicht als ausreichend leistungsstarke Reduktionsmittel angesehen werden, bei Mischung mit den Verbindungen (A) eine besonders ausgeprägte technische Wirkung zeigten.

Diese technische Wirkung besteht, wie vorstehend erläutert, insbesondere darin, dass die Kombination der Verbindungen (A) und (S) zu einem Bestandteil eines Polymerisationsinitiatorsystems in einer erfindungsgemäßen Zusammensetzung führt, der eine hohe Leistungsfähigkeit besitzt, aber in synergistischer Art und Weise weniger stark ein vorzeitiges Polymerisieren der in der Zusammensetzung enthaltenen radikalisch polymerisierbaren organischen Monomere (M) veranlasst als dies ein Vergleichsbestandteil eines Polymerisationsinitiatorsystems einer nicht erfindungsgemäßen Zusammensetzung tut, welche lediglich die Verbindung (A) (in gleicher Gesamtstoffmenge) enthält.

Besonders bevorzugt ist eine erfindungsgemäße Zusammensetzung (vorzugsweise wie vorstehend als bevorzugt bezeichnet), wobei die Verbindung (A) ausgewählt ist aus der Gruppe bestehend aus Ascorbinsäure, Natriumascorbat, Isoascorbinsäure und Natriumisoascorbat und die Verbindung (S) ausgewählt ist aus der Gruppe bestehend aus Toluolsulfinsäure, Natriumtoluolsulfinat, Kaliumtoluolsulfinat, Benzolsulfinsäure, Natriumbenzolsulfinat, Kaliumbenzolsulfinat, Natriumtetraphenylborat und Lithiumtetraphenylborat, besonders bevorzugt ausgewählt ist aus der Gruppe bestehend aus Toluolsulfinsäure, Natriumtoluolsulfinat, Kaliumtoluolsulfinat, Benzolsulfinsäure, Natriumbenzolsulfinat und Kaliumbenzolsulfinat.

Besonders bevorzugt ist eine erfindungsgemäße Zusammensetzung (vorzugsweise wie vorstehend als bevorzugt bezeichnet), wobei sämtliche Verbindungen (A) ausgewählt sind aus der Gruppe bestehend aus Ascorbinsäure, Natriumascorbat, Isoascorbinsäure und Natriumisoascorbat und sämtliche Verbindungen (S) ausgewählt sind aus der Gruppe bestehend aus Toluolsulfinsäure, Natriumtoluolsulfinat, Kaliumtoluolsulfinat, Benzolsulfinsäure, Natriumbenzolsulfinat und Kaliumbenzolsulfinat.

Für derart bevorzugte erfindungsgemäße Zusammensetzungen werden die vorteilhaftesten Lagerstabilitäten beobachtet.

Bevorzugt ist eine erfindungsgemäße Zusammensetzung (vorzugsweise wie vorstehend als bevorzugt bezeichnet), wobei das Verhältnis der Gesamtstoffmenge sämtlicher Verbindungen (A) zu der Gesamtstoffmenge sämtlicher Verbindungen (S)
größer als 0,2, bevorzugt größer als 0,3, besonders bevorzugt größer als 1 ist und/oder
im Bereich von 0,2 bis 50, bevorzugt im Bereich von 0,3 bis 20, besonders bevorzugt im Bereich von 1 bis 10 liegt.

In derart bevorzugten erfindungsgemäßen Zusammensetzungen ergibt sich ein besonders ausgeprägter vorteilhafter Effekt der Kombination der Verbindungen (A) und (S) für die Lagerstabilität der erfindungsgemäßen Zusammensetzung und auf die mechanischen Eigenschaften des durch Härtung aus der Zusammensetzung herstellbaren Dentalmaterials.

Bevorzugt ist eine erfindungsgemäße Zusammensetzung (vorzugsweise wie vorstehend als bevorzugt bezeichnet),
wobei das Verhältnis der kombinierten Gesamtmasse sämtlicher Verbindungen (A) und sämtlicher Verbindungen (S) zu der Gesamtmasse sämtlicher radikalisch polymerisierbarer organischer Monomere (M) im Bereich von 0,05 bis 10 Gew.-% liegt, vorzugsweise im Bereich von 0,5 bis 7 Gew.-%
und/oder
wobei der Gesamtanteil radikalisch polymerisierbarer organischer Monomere (M) im Bereich von 15 bis 95 Gew.-% liegt, vorzugsweise im Bereich von 25 bis 40 Gew.-%, bezogen auf die Gesamtmasse der Zusammensetzung
und/oder
wobei der kombinierte Gesamtanteil sämtlicher Verbindungen (A) und sämtlicher Verbindungen (S) im Bereich von 0,05 bis 7 Gew.-% liegt, vorzugsweise im Bereich von 0,1 bis 2,5 Gew.-%, bezogen auf die Gesamtmasse der Zusammensetzung.

Jede der vorstehenden erfindungsgemäßen Zusammensetzungen ist bevorzugt, da sich für die definierten Massenverhältnisse, bzw. bei den angegebenen Gesamtanteilen die technische Wirkung am besten beobachten und messen lässt.

Bei sehr geringen Gehalten an Reduktionsmittel zeigen sich beispielsweise signifikante Unterschiede zu herkömmlichen Systemen erst nach längeren Lagerzeiten, wohingegen besonders große Anteile an Reduktionsmittel (und insbesondere von Verbindungen (A)) auch in erfindungsgemäßen Zusammensetzungen zu einer recht schnellen Polymerisation führen können, die nicht immer akzeptabel ist. Ganz entsprechend sind die überraschenden technischen Effekte dann besonders ausgeprägt, wenn der Gesamtanteil radikalisch polymerisierbarer organischer Monomere (M) einen gewissen Mindestwert übersteigt, da eine zu geringe Konzentration der Monomere (M) die Polymerisationskinetik hemmt.

Bevorzugt ist eine erfindungsgemäße Zusammensetzung (vorzugsweise wie vorstehend als bevorzugt bezeichnet),
wobei die Zusammensetzung
- flüssig oder eine Paste ist
   und
- nach einer Lagerung bei 37 °C von 6 Wochen, bevorzugt 8 Wochen, besonders bevorzugt 10 Wochen und ganz besonders bevorzugt 12 Wochen, eine Viskosität besitzt, die kleiner ist als 10000 Pa s, vorzugsweise kleiner ist als 8000 Pa s
und/oder
wobei die Zusammensetzung direkt nach der Herstellung eine Viskosität aufweist, die höchstens um den Faktor 5, bevorzugt höchstens um den Faktor 3, kleiner ist als die Viskosität der entsprechenden Komponente nach 6 Wochen der Lagerung bei 37 °C.

In Flüssigkeiten und Pasten zeigt sich der negative Effekt von Reduktionsmitteln (insbesondere Verbindungen (A)) auf die Lagerstabilität regelmäßig am deutlichsten, so dass für sie auch die größten absolut erzielbaren Verbesserungen der Lagerstabilität beobachtet werden können. Die vorstehend definierten bevorzugten erfindungsgemäßen Zusammensetzungen sind besonders vorteilhaft, weil sie sich auch nach 6 Wochen der Lagerung bei 37 °C noch hervorragend verarbeiten lassen und insoweit den entsprechenden Zusammensetzungen direkt nach der Herstellung für praktische Zwecke noch ausreichend ähnlich sind.

Die Bestimmung der Viskosität erfolgt (sofern nicht anders angegeben) mittels eines Rheometers (Physica MCR 301) der Firma Anton Paar (Graz, Österreich). Die Messung erfolgt bei 23 °C (bei einem Umgebungsdruck von 1 bar) im Rotationsversuch mit Platte/Platte-Anordnung (Durchmesser 25 mm, Spaltabstand 1 mm). Die Viskosität wird für drei Scherraten von 10 s⁻¹ (15 s), 1 s⁻¹ (30 s) und 0,1 s⁻¹ (60 s) gemessen. Hierbei gibt der Wert in Klammern die Intervallzeit an. Es wird 1 Messwert je Scherrate aufgenommen. Es liegen also 3 Messwerte in einem Bereich von 0,1 bis 10 s⁻¹ vor. Als Viskosität wird der Wert, der bei einer Scherrate von 0,1 s⁻¹ gemessen wird, angegeben. Die Messung wird wie beschrieben durchgeführt (von hoher zu niedriger Scherrate), damit eine immer gleiche Vorscherung des Materials gewährleistet wird. Insbesondere bei niedrigen Scherraten wäre die Vorscherung durch das Aufbringen des Materials auf die Messplatte und das Zusammenfahren der Messplatten andernfalls höher als die Scherung während der Messung und die Messung somit kaum reproduzierbar.

Bevorzugt ist eine erfindungsgemäße Zusammensetzung (vorzugsweise wie vorstehend als bevorzugt bezeichnet), umfassend
- 0,01 bis 5 Gew.-%, vorzugsweise 0,05 bis 1 Gew.-%, einer oder mehrerer Verbindungen ausgewählt aus der Gruppe bestehend aus Ascorbinsäure, Natriumascorbat, Isoascorbinsäure und Natriumisoascorbat,
- 0,01 bis 5 Gew.-%, vorzugsweise 0,05 bis 1 Gew.-%, einer oder mehrerer Verbindungen ausgewählt aus der Gruppe bestehend aus Toluolsulfinsäure, Natriumtoluolsulfinat, Kaliumtoluolsulfinat, Benzolsulfinsäure, Natriumbenzolsulfinat, Kaliumbenzolsulfinat, Natriumtetraphenylborat und Lithiumtetraphenylborat,
- 2 bis 40 Gew.-%, vorzugsweise 3 bis 30 Gew.-%, einer oder mehrerer Verbindungen ausgewählt aus der Gruppe bestehend aus Ethylenglykoldimethacrylat, 1,6-Hexandioldimethacrylat, Triethylenglykoldimethacrylat, 1,12-Dodecandioldimethacrylat, ethoxyliertes Bisphenol-A-dimethacrylat, Polyethylenglykoldimethacrylat, 7,7,9-Trimethyl-4,13-dioxo-3,14-dioxa-5,12-diazahexadecan-1,16-dioxydimethacrylat, Butandioldimethacrylat, Trimethylolpropantrimethacrylat, Urethandimethacrylat, Glycerin-1,3-dimethacrylat, Tetraethylenglykoldimethacrylat, Neopentylglykoldimethacrylat, 2,2-Bis[4-[3-methacryloyloxy-2-hydroxypropoxy]phenyl]propan und den Di(meth)acrylaten des Dihydroxymethyltricyclo[5.2.1.0^{2.6}]decans,
- 3 bis 60 Gew.-%, vorzugsweise 6 bis 40 Gew.-%, einer oder mehrerer Verbindungen ausgewählt aus der Gruppe bestehend aus 2- Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-Bis[4-[3-methacryloyloxy-2-hydroxypropoxy]phenyl]propan,
   und
- 5 bis 85 Gew.-%, vorzugsweise 20 bis 80 Gew.-%, Aluminiumfluorosilikatglas,
wobei die Prozentangaben auf die Gesamtmasse der Zusammensetzung bezogen sind.

Solche bevorzugten Zusammensetzungen haben sich in Praxistests als besonders vorteilhaft erwiesen, weil sie über eine besonders hohe Lagerstabilität verfügen und sich ihre Eigenschaften auch nach mehrwöchiger Lagerung bei 37 °C nur in relativ geringem Maße verändern. Werden entsprechende erfindungsgemäße Zusammensetzungen als Komponente eines mehrkomponentigen kunststoffmodifizierten Glasionomerzements eingesetzt, kann auch nach mehrwöchiger Lagerung bei 37 °C durch Vermischen der Komponenten ein gehärteter kunststoffmodifizierter Glasionomerzement erhalten werden, dessen mechanische Eigenschaften, insbesondere dessen Biegefestigkeit, von der vorangehenden Lagerung der separaten Komponenten nur unwesentlich beeinflusst sind.

Bevorzugt ist eine erfindungsgemäße Zusammensetzung (vorzugsweise wie vorstehend als bevorzugt bezeichnet), umfassend
- Ascorbinsäure und/oder Natriumisoascorbat in einer Gesamtmenge von 0,1 bis 1 Gew.-%,
- Natriumtoluolsulfinat und/oder Natriumtetraphenylborat in einer Gesamtmenge von 0,01 bis 1 Gew.-%,
- Urethandimethacrylat und/oder Triethylenglycoldimethacrylat und/oder Glycerin-1,3-dimethacrylat in einer Gesamtmenge von 5 bis 20 Gew.-%,
- 2-Hydroxyethylmethacrylat und/oder 2-Hydroxypropylmethacrylat in einer Gesamtmenge von 10 bis 30 Gew.-%,
- 0,01 bis 1 Gew.-% 2,6-Di(tert-butyl)hydroxytoluol
   und
   - 50 bis 75 Gew.-%, Aluminiumfluorosilikatglas,
   wobei die Prozentangaben auf die Gesamtmasse der Zusammensetzung bezogen sind.

Für derart bevorzugte Zusammensetzungen konnten in eigenen Versuchen die vorteilhaftesten Gesamteigenschaften erzielt werden. Insbesondere die für die Verbindungen (A) und (S) sowie für die radikalisch polymerisierbare organische Monomere (M) und die basische Glaszusammensetzung (G) gewählten Substanzen wurden dabei in umfassenden Entwicklungsschritten iterativ aufeinander abgestimmt und angepasst. Für die bevorzugten Zusammensetzungen sind die hohe Lagerstabilität der erfindungsgemäßen Zusammensetzung mit besonders günstigen Verarbeitungseigenschaften und einer hohen Leistungsfähigkeit des hergestellten gehärteten kunststoffmodifizierten Glasionomerzements kombiniert.

Bevorzugt ist eine erfindungsgemäße Zusammensetzung (vorzugsweise wie vorstehend als bevorzugt bezeichnet),
wobei die Zusammensetzung wasserfrei ist
und/oder
wobei die Zusammensetzung kein carbonsäuregruppenhaltiges Polymer (P) ausgewählt aus der Gruppe bestehend aus Homo- und Copolymeren einer α,β-ungesättigten Carbonsäure (C) umfasst
und/oder
wobei die Zusammensetzung kein Oxidationsmittel (O) ausgewählt aus der Gruppe bestehend aus Persulfaten, Permanganaten, Perboraten, Peroxiden und Hydroperoxiden umfasst.

Solche erfindungsgemäßen Zusammensetzungen sind insbesondere deshalb bevorzugt, weil die Lagerstabilität und/oder die Fähigkeit zur Härtung von erfindungsgemäßen Zusammensetzungen bei der bevorzugten Abwesenheit der vorstehend definierten Bestandteile regelmäßig besser sind als bei ihrer Anwesenheit. Die Verbindungen (A) und (S) sind z.B. in wasserhaltigen und/oder sauren Zusammensetzungen regelmäßig nicht besonders beständig. Die Gegenwart eines Oxidationsmittels (O) in der erfindungsgemäßen Zusammensetzung könnte in Gegenwart der Verbindungen (A) und/oder (S) die Polymerisation der radikalisch polymerisierbare organische Monomere (M) initiieren und ist daher unerwünscht. Da die erfindungsgemäße Zusammensetzung auch eine basische Glaszusammensetzung (G) enthält, würde das gleichzeitige Vorliegen eines carbonsäuregruppenhaltigen Polymers (P) ebenfalls zur direkten Härtung der Zusammensetzung durch GIZ-Abbindung führen.

Der Fachmann weiß, dass in erfindungsgemäßen Zusammensetzungen, beispielsweise bedingt durch Verunreinigungen von Ausgangssubstanzen, durch Luftfeuchtigkeit oder durch Substanzrückstände auf den verwendeten Produktionsgeräten, in Einzelfällen durchaus kleinste Mengen der vorstehenden Substanzen vorhanden sein können, selbst wenn dies nicht gewünscht ist. Der Einfluss dieser Verunreinigungen ist in diesem Fall jedoch in der Regel vernachlässigbar bzw. noch akzeptabel.

Aus den oben genannten Gründen bevorzugt ist daher auch eine erfindungsgemäße Zusammensetzung (vorzugsweise wie vorstehend als bevorzugt bezeichnet),
umfassend weniger als 0,5 Gew.-%, bevorzugt weniger als 0,01 Gew.-% Wasser
und/oder
umfassend weniger als 0,5 Gew.-%, bevorzugt weniger als 0,01 Gew.-% an carbonsäuregruppenhaltigen Polymeren (P)
und/oder
umfassend weniger als 0,5 Gew.-%, bevorzugt weniger als 0,01 Gew.-% an Oxidationsmitteln (O) ausgewählt aus der Gruppe bestehend aus Persulfaten, Permanganaten, Perboraten, Peroxiden und Hydroperoxiden,
wobei die Prozentangaben auf die Gesamtmasse der Zusammensetzung bezogen sind.

Die vorliegende Erfindung betrifft gemäß einem weiteren Aspekt auch einen mehrkomponentigen kunststoffmodifizierten Glasionomerzement umfassend
- eine erfindungsgemäße Zusammensetzung als erste Komponente (es gelten insoweit die vorstehenden Angaben zu bevorzugten Ausgestaltungen)
   und
- in ein oder mehreren weiteren Komponenten zumindest
   - ein oder mehrere carbonsäuregruppenhaltige Polymere (P) ausgewählt aus der Gruppe bestehend aus Homo- und Copolymeren einer α,β-ungesättigten Carbonsäure (C),
   - Wasser
      und
   - ein oder mehrere Oxidationsmittel (O) ausgewählt aus der Gruppe bestehend aus Persulfaten, Permanganaten, Perboraten, Peroxiden und Hydroperoxiden,
      wobei der mehrkomponentige kunststoffmodifizierte Glasionomerzement
   - in der ersten und/oder einer weiteren Komponente eine basische Glaszusammensetzung (G) als Vernetzer für die Polymere (P) umfasst,
      mit der Maßgabe, dass die basische Glaszusammensetzung (G) nicht in einer Komponente gemeinsam mit einem carbonsäuregruppenhaltigen Polymer (P) und/oder gemeinsam mit Wasser vorliegt.

Ein solcher mehrkomponentiger kunststoffmodifizierter Glasionomerzement ist als Kit zu verstehen. Das erfindungsgemäße Kit umfasst zwei, drei oder mehr separate, d.h. räumlich voneinander getrennt vorliegende, Komponenten, vorzugsweise besteht er aus zwei separaten Komponenten. Das erfindungsgemäße Kit verfügt über eine hohe Lagerstabilität und kann auch nach mehrwöchiger Lagerung durch Vermischen der Komponenten zu einem gehärteten kunststoffmodifizierten Glasionomerzement gehärtet werden, dessen mechanische Eigenschaften nur geringfügig von der vorangehenden Lagerung beeinflusst sind.

Der Fachmann versteht, dass somit ein mehrkomponentiger kunststoffmodifizierter Glasionomerzement bevorzugt ist umfassend
- eine erfindungsgemäße Zusammensetzung als erste Komponente
   und
- in ein oder mehreren weiteren Komponenten zumindest
   - ein oder mehrere carbonsäuregruppenhaltige Polymere (P) ausgewählt aus der Gruppe bestehend aus Homo- und Copolymeren einer α,β-ungesättigten Carbonsäure (C),
   - Wasser
      und
   - ein oder mehrere Oxidationsmittel (O) ausgewählt aus der Gruppe bestehend aus Persulfaten, Permanganaten, Perboraten, Peroxiden und Hydroperoxiden,
   wobei der mehrkomponentige kunststoffmodifizierte Glasionomerzement
- in der ersten und/oder einer weiteren Komponente eine basische Glaszusammensetzung (G) als Vernetzer für die Polymere (P) umfasst,
   wobei die basische Glaszusammensetzung (G) und das carbonsäuregruppenhaltige Polymer (P) in separaten Komponenten vorliegen und/oder
   wobei die basische Glaszusammensetzung (G) und das Wasser in separaten Komponenten vorliegen.

Vorzugsweise liegen in einem erfindungsgemäßen mehrkomponentigen kunststoffmodifizierten Glasionomerzement sämtliche Verbindungen (A) und (S) nur in der ersten Komponente vor, wobei das eine oder die mehreren radikalisch polymerisierbare organische Monomere (M) in der ersten Komponente und zumindest einer weiteren Komponente vorliegen.

Die vorstehend beschriebenen erfindungsgemäßen mehrkomponentigen kunststoffmodifizierten Glasionomerzemente sind insbesondere deshalb besonders lagerstabil, weil zum einen eine erfindungsgemäße Zusammensetzung als Komponente eingesetzt wird und weil zum anderen die Auswirkungen chemischer Unverträglichkeiten vermieden werden.

Bevorzugt sollten das Wasser und das carbonsäuregruppenhaltige Polymer (P) nicht in der gleichen Komponente wie die erfindungsgemäße Zusammensetzung vorliegen, d.h. das Wasser und das carbonsäuregruppenhaltige Polymer (P) sollten ausschließlich in den weiteren Komponenten vorliegen. Lägen Wasser und/oder das carbonsäuregruppenhaltige Polymer (P) in der gleichen Komponente wie die erfindungsgemäße Zusammensetzung vor, dann wären die Verbindungen (A) und (S) möglicherweise nicht ausreichend beständig.

Bevorzugt sollte das Oxidationsmittel (O) nicht in der gleichen Komponente wie die erfindungsgemäße Zusammensetzung vorliegen, d.h. das Oxidationsmittel (O) sollte ausschließlich in den weiteren Komponenten vorliegen. Wären das Oxidationsmittel (O) Bestandteil der gleichen Komponente wie die erfindungsgemäße Zusammensetzung, könnte es durch die Wechselwirkung mit den Verbindungen (A) und (S) zu einer ungewollten Polymerisation der radikalisch polymerisierbare organische Monomere (M) kommen.

Wären die basische Glaszusammensetzung (G) und das carbonsäuregruppenhaltige Polymer (P) Bestandteil der gleichen Komponente, käme es durch die GIZ-Abbindung zu einer ungewollten Härtung.

Wären die basische Glaszusammensetzung (G) und das Wasser Bestandteil der gleichen Komponente, könnte es, zumindest teilweise, zu einer nachteiligen Reaktion zwischen Wasser und den Bestandteilen des Glases kommen, die in einer verminderten Fähigkeit des Glases resultieren könnte, als Vernetzer für carbonsäuregruppenhaltige Polymere (P) ausgewählt aus der Gruppe bestehend aus Homo- und Copolymeren einer α,β-ungesättigten Carbonsäure (C)zu reagieren.

Carbonsäuregruppenhaltige Polymere (P) ausgewählt aus der Gruppe bestehend aus Homo- und Copolymeren einer α,β-ungesättigten Carbonsäure (C) entstehen z.B. nach einer radikalischen Polymerisation beispielsweise einer wässrigen, α,β-ungesättigten Acryl-, Methacryl-, Itacon-, Fumar-, Malein-, Chlormethacryl-, Cyanomethacryl-, Aconit-, Mesacon-, Glutacon-, Citraconsäure, etc.. Werden organische α,β-ungesättigte Mono- oder Dicarbonsäuren (bzw. deren Anhydride) einzeln polymerisiert, so erhält man Homopolymere. Wird die Polymerisation in Gegenwart von zwei oder mehreren Säuren durchgeführt, so gelangt man zu Copolymeren. Erfindungsgemäß kann ein einziges (Homo- oder Co-)Polymer oder ein Gemisch unterschiedlicher Polymere eingesetzt werden.

Die α,β-ungesättigten Carbonsäuren (C) (die zur Herstellung des carbonsäuregruppenhaltigen Polymers (P) eingesetzt werden) sind strukturell nicht besonders beschränkt und können verwendet werden unabhängig von der Anzahl von Carbonsäuregruppen im Molekül oder der Existenz einer Carbonsäureanhydridgruppe oder anderer Substituenten.

Der Einsatz der Homo- oder Copolymere bzw. der Gemische kann in Form ihrer wässrigen Lösungen erfolgen.

Wasser kann demineralisiert oder auch einfach als Leitungswasser verwendet werden.

Das erfindungsgemäße Kit kann als zweikomponentiges Kit in der Form Paste/Flüssigkeit oder, ganz besonders bevorzugt als Paste/Paste vorliegen.

Bevorzugt ist ein erfindungsgemäßer mehrkomponentiger kunststoffmodifizierter Glasionomerzement (vorzugsweise wie vorstehend als bevorzugt bezeichnet),
wobei die α,β-ungesättigte Carbonsäure (C) (die zur Herstellung des carbonsäuregruppenhaltigen Polymers (P) eingesetzt wird) ausgewählt ist aus der Gruppe bestehend aus Acrylsäure, Methacrylsäure, Itaconsäure, Fumarsäure, Maleinsäure, Chlormethacrylsäure, Cyanomethacrylsäure, Aconitsäure, Mesaconsäure, Glutaconsäure und Citraconsäure, vorzugsweise ausgewählt ist aus der Gruppe bestehend aus Acrylsäure und Maleinsäure
und/oder
wobei das gewichtsmittlere Molekulargewicht M_{w} der Gesamtmenge der carbonsäuregruppenhaltigen Polymere (P) im Bereich von 1000 bis 150000 g/mol, vorzugsweise zwischen 40000 und 100000 g/mol, liegt.

Entsprechende erfindungsgemäße mehrkomponentige kunststoffmodifizierte Glasionomerzemente weisen eine besonders gute Härtungskinetik auf und resultieren nach der Härtung in gehärteten kunststoffmodifizierten Glasionomerzementen mit besonders vorteilhaften mechanischen Eigenschaften. Ganz besonders vorteilhaft ist, dass sich entsprechende erfindungsgemäße mehrkomponentige kunststoffmodifizierte Glasionomerzemente besonders leicht verarbeiten lassen, insbesondere lassen sich ihre Komponenten leicht vermischen.

Ist das gewichtsmittlere Molekulargewicht M_{w} der Gesamtmenge der carbonsäuregruppenhaltigen Polymere (P) kleiner als 1.000 g/mol, so sind in manchen Fällen die mechanischen Eigenschaften der resultierenden dentalen Massen ungenügend, die Haftung des Materials an der Zahnsubstanz ist schlecht und der Patient verspürt einen schlechten Geschmack und vernimmt einen unangenehmen Geruch. Übersteigt das gewichtsmittlere Molekulargewicht M_{w} der Gesamtmenge der carbonsäuregruppenhaltigen Polymere (P) einen Wert von 150000 g/mol, so steigt die Viskosität in manchen Fällen so stark, dass ein homogenes Vermischen der Komponenten erschwert oder gar unmöglich wird.

Weiterhin ist es möglich, die carbonsäuregruppenhaltigen Polymere (P) in fester Form einzusetzen. Hierzu werden die Polymerlösungen beispielsweise gefriergetrocknet oder anderen geeigneten Methoden unterworfen.

Bevorzugt ist ein erfindungsgemäßer mehrkomponentiger kunststoffmodifizierter Glasionomerzement (vorzugsweise wie vorstehend als bevorzugt bezeichnet),
wobei die basische Glaszusammensetzung (G) ein oder mehrere Kationen von Elementen umfasst, die ausgewählt sind aus der Gruppe bestehend aus den Metallen der Hauptgruppen I, II und III des Periodensystems, vorzugsweise ausgewählt sind aus der Gruppe bestehend aus Natrium, Kalium, Calcium und Aluminium,
und die besonders bevorzugt vorliegen in Form einer oder mehrerer Verbindungen ausgewählt aus der Gruppe bestehend aus Oxiden, Hydroxiden, Sulfaten, Nitraten, Phosphaten, Carbonaten, Silikaten, Fluoriden und Nitriden
und/oder
wobei die basische Glaszusammensetzung (G) ein Aluminiumfluorosilikatglas umfasst
und/oder
wobei die basische Glaszusammensetzung (G) ein oder mehrere Gläser mit organisch modifizierter Oberfläche, vorzugsweise mit silanisierter Oberfläche, umfasst.

Die Vorteile entsprechend erfindungsgemäßer mehrkomponentiger kunststoffmodifizierter Glasionomerzemente entsprechen denen, die vorstehend für die erfindungsgemäßen Zusammensetzungen dargelegt sind.

Bevorzugt ist ein erfindungsgemäßer mehrkomponentiger kunststoffmodifizierter Glasionomerzement (vorzugsweise wie vorstehend als bevorzugt bezeichnet),
wobei das eine oder zumindest eines der mehreren Oxidationsmittel (O) ausgewählt ist aus der Gruppe bestehend aus Peroxiden und Hydroperoxiden, vorzugsweise ausgewählt ist aus der Gruppe bestehend aus Hydroperoxiden.

Entsprechende erfindungsgemäße mehrkomponentige kunststoffmodifizierte Glasionomerzemente sind bevorzugt, weil sich die angegebenen bevorzugten Oxidationsmittel in der Praxis als besonders kompatibel mit den Verbindungen (A) und (S) erwiesen haben. Das bedeutet, dass durch das Vermischen entsprechender Oxidationsmittel (O) mit erfindungsgemäßen Zusammensetzungen als Bestandteile mehrkomponentiger kunststoffmodifizierter Glasionomerzemente, auch nach längeren Lagerzeiten, besonders günstige, d.h. möglichst wenig veränderte, Abbindezeiten erhalten werden.

Bevorzugt ist ein erfindungsgemäßer mehrkomponentiger kunststoffmodifizierter Glasionomerzement (vorzugsweise wie vorstehend als bevorzugt bezeichnet), in der ersten und/oder einer weiteren Komponente zusätzlich umfassend
- ein oder mehrere Verbindungen (Z) ausgewählt aus der Gruppe bestehend aus Barbitursäuren, tertiären Aminen und sekundären Aminen,
   und/oder
- einen oder mehrere Photoinitiatoren,
   wobei der eine oder die mehreren Photoinitiatoren vorzugsweise ausgewählt sind aus der Gruppe bestehend aus Benzoinalkylether, Benzoinalkylester, Benzilmonoketale, Acylphosphinoxide, Benzophenone, Acetophenone, Ketalen, Thioxanthone, Titanocene, aliphatische 1,2-Diketoverbindungen und aromatische 1,2-Diketoverbindungen, vorzugsweise ausgewählt ist aus der Gruppe umfassend 2,2-Diethoxyacetophenon, 9,10-Phenanthrenchinon, Diacetyl, Furil, Anisil, 4,4'-Dichlorbenzil und 4,4'-Dialkoxybenzil und Campherchinon
   und/oder
- einen oder mehrere Inhibitoren,
   wobei der eine oder die mehreren Inhibitoren vorzugsweise ausgewählt sind aus der Gruppe bestehend aus substituierten Phenolen, Phenothiazin, stabile organische Radikale und Hydrochinonmonomethylether, besonders bevorzugt ausgewählt sind aus der Gruppe bestehend aus 2,6-Di-tert.-butyl-4-methylphenol, Hydrochinonmonomethylether, dem 2,2-Diphenyl-1-picrylhydrazylradikal, dem Galvinoxylradikal, dem Triphenylmethylradikal und dem 2,2,6,6-Tetramethylpiperidinyl-1-oxylradikal
   und/oder
- ein oder mehrere Additive ausgewählt aus der Gruppe bestehend aus Farbmitteln, Weinsäure, Kieselsäure, Stabilisatoren, Modifikatoren, bakterizid wirkenden Substanzen und inerten Füllstoffen.

Die Vorteile dieser bevorzugten erfindungsgemäßen mehrkomponentigen kunststoffmodifizierten Glasionomerzemente entsprechen denen, die vorstehend für die bevorzugten erfindungsgemäßen Zusammensetzungen und deren Inhaltsstoffe dargelegt sind.

Die Erfindung betrifft gemäß einem weiteren Aspekt ein Verfahren zur Herstellung einer erfindungsgemäßen Zusammensetzung (wie vorstehend definiert, vorzugsweise wie vorstehend als bevorzugt bezeichnet) oder eines gehärteten Dentalmaterials, wobei eine erfindungsgemäße Zusammensetzung (wie vorstehend definiert, vorzugsweise wie vorstehend als bevorzugt bezeichnet) als Zwischenprodukt hergestellt wird, umfassend die folgenden Schritte:

| | |
|---|---|
| Schritt (a) | Herstellen oder Bereitstellen eines oder mehrerer radikalisch polymerisierbarer organischer Monomere (M), |
| Schritt (b) | Herstellen oder Bereitstellen einer oder mehrerer Verbindungen (A), ausgewählt aus der Gruppe bestehend aus den Isomeren der Ascorbinsäure, den Salzen der Isomere der Ascorbinsäure, den Estern der Isomere der Ascorbinsäure und den Ethern der Isomere der Ascorbinsäure, |
| Schritt (c) | Herstellen oder Bereitstellen einer oder mehrerer Verbindungen (S), ausgewählt aus der Gruppe bestehend aus Sulfinsäuren, Salzen der Sulfinsäuren und Salzen bororganischer Verbindungen und |
| Schritt (d) | nach den Schritten (a), (b) und (c) Vermischen der Monomere (M) mit den Verbindungen (A) und (S) |
| Schritt (e) | Herstellen oder Bereitstellen einer basischen Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C), |

wobei Schritt (d) das Vermischen der Monomere (M) mit den Verbindungen (A) und (S) sowie mit der in Schritt (e) hergestellten oder bereitgestellten Glaszusammensetzung (G) umfasst.

Hinsichtlich der in den Schritten (a) bis (d) eingesetzten Substanzen (M), (A) und (S) gelten jeweils die vorstehend im Zusammenhang mit anderen Aspekten der Erfindung gemachten Ausführungen entsprechend.

Die Reihenfolge der Schritte (a), (b) und (c) ist beliebig. Schritt (d) kann Teilschritte umfassen, z.B. wenn ein Vormischen einzelner Bestandteile gewünscht ist.

Das erfindungsgemäße Verfahren ist apparativ besonders leicht durchzuführen und der Fachmann hat die Möglichkeit, das Vermischen in Schritt (d) mit zahlreichen verschiedenen Methoden durchzuführen. Bevorzugt erfolgt das Vermischen der Monomere (M) mit sämtlichen Verbindungen (A) und das Vermischen der Monomere (M) mit sämtlichen Verbindungen (S) gleichzeitig, d.h. nicht nacheinander. Somit umfasst das Verfahren vorzugsweise als Bestandteil des Schrittes (d) einen (Teil-)Schritt, wobei im (Teil-)Schritt sämtliche Verbindungen (A) und (S) miteinander vermischt werden.

Der Begriff "gehärtetes Dentalmaterial" bezeichnet für den Einsatz im Dentalbereich geeignete Zusammensetzungen, unabhängig vom Grad der Härtung. Sobald also beispielsweise in einer dentalen Zusammensetzung die Polymerisation von Monomeren (M) (und damit eine Härtung) eingesetzt hat, liegt ein gehärtetes Dentalmaterial vor. Diese Definition ist notwendig, weil ein Endpunkt einer Härtung in Dentalmaterialien regelmäßig nicht scharf definiert werden kann und es auch in augenscheinlich ausgehärteten Materialien noch in geringem Maße zur Polymerisation kommen kann. Am Beispiel eines erfindungsgemäßen mehrkomponentigen kunststoffmodifizierten Glasionomerzements bedeutet dies, dass bereits unmittelbar nach dem Vermischen der Komponenten und damit dem Beginn der Polymerisation der Monomere (M) sowie dem Beginn der GIZ-Abbindung ein gehärtetes Dentalmaterial vorliegt. Ebenso handelt es sich beispielsweise bei einem verarbeiteten Glasionomerzement, mit dem beispielsweise ein Implantat befestigt ist, um ein gehärtetes Dentalmaterial.

Bevorzugt sind erfindungsgemäße Verfahren (vorzugsweise wie vorstehend als bevorzugt bezeichnet), mit denen bevorzugte oder besonders bevorzugte erfindungsgemäße Zusammensetzungen hergestellt werden, wobei sich die Vorteile dieser Verfahren jeweils aus den vorstehend offenbarten Vorteilen der hergestellten bevorzugten Zusammensetzungen ergeben. Nachfolgend werden entsprechende besonders bevorzugte Verfahren offenbart.

Bevorzugt ist ein erfindungsgemäßes Verfahren (vorzugsweise wie vorstehend als bevorzugt bezeichnet), wobei das eine oder zumindest eines der mehreren radikalisch polymerisierbaren organischen Monomere (M)
- ausgewählt ist aus der Gruppe bestehend aus Monomeren mit mindestens zwei ethylenischen Gruppen (M1),
   wobei die Monomere mit mindestens zwei ethylenischen Gruppen (M1) vorzugsweise ausgewählt sind aus der Gruppe bestehend aus Diacrylaten, Triacrylaten, Dimethacrylaten, Trimethacrylaten, besonders vorzugsweise ausgewählt sind aus der Gruppe bestehend aus Ethylenglykoldimethacrylat, 1,6-Hexandioldimethacrylat, Triethylenglykoldimethacrylat, 1,12-Dodecandioldimethacrylat, ethoxyliertes Bisphenol-A-dimethacrylat, Polyethylenglykoldimethacrylat, 7,7,9-Trimethyl-4,13-dioxo-3,14-dioxa-5,12-diazahexadecan-1,16-dioxydimethacrylat, Butandioldimethacrylat, Trimethylolpropantrimethacrylat, Urethandimethacrylat, Glycerin-1,3-dimethacrylat, Tetraethylenglykoldimethacrylat, Neopentylglykoldimethacrylat, 2,2-Bis[4-[3-methacryloyloxy-2-hydroxypropoxy]phenyl]propan und den Di(meth)acrylaten des Dihydroxymethyltricyclo[5.2.1.0^{2,6}]decans
   und/oder
   wobei das eine oder zumindest eines der mehreren oder zumindest ein weiteres der mehreren radikalisch polymerisierbaren organischen Monomere (M)
- ausgewählt ist aus der Gruppe bestehend aus Hydroxylverbindungen mit mindestens einer ethylenischen Gruppe (M2),
   wobei die Hydroxylverbindungen mit mindestens einer ethylenischen Gruppe (M2) vorzugsweise ausgewählt sind aus der Gruppe bestehend aus 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat und 2,2-Bis[4-[3-methacryloyloxy-2-hydroxypropoxy]phenyl]propan.

Besonders bevorzugt ist hierbei die und-Verknüpfung. Ebenfalls bevorzugt ist, dass sämtliche der radikalisch polymerisierbaren organischen Monomere (M) ausgewählt sind aus der Gruppe bestehend aus Monomeren mit mindestens zwei ethylenischen Gruppen (M1) und Hydroxylverbindungen mit mindestens einer ethylenischen Gruppe (M2).

Bevorzugt ist ein erfindungsgemäßes Verfahren (vorzugsweise wie vorstehend als bevorzugt bezeichnet), wobei die eine oder zumindest eine der mehreren Verbindungen (A) ausgewählt ist aus der Gruppe bestehend aus Ascorbinsäure, Salzen der Ascorbinsäure, Isoascorbinsäure und Salzen der Isoascorbinsäure, vorzugsweise ausgewählt ist aus der Gruppe bestehend aus Ascorbinsäure, Natriumascorbat, Isoascorbinsäure und Natriumisoascorbat
und/oder
wobei die eine oder zumindest eine der mehreren Verbindungen (S) ausgewählt ist aus der Gruppe bestehend aus aromatischen Sulfinsäuren, Salzen der aromatischen Sulfinsäuren und Salzen aromatischer bororganischer Verbindungen, vorzugsweise ausgewählt ist aus der Gruppe bestehend aus Toluolsulfinsäure, Natriumtoluolsulfinat, Kaliumtoluolsulfinat, Benzolsulfinsäure, Natriumbenzolsulfinat, Kaliumbenzolsulfinat, Natriumtetraphenylborat und Lithiumtetraphenylborat, besonders bevorzugt ausgewählt ist aus der Gruppe bestehend aus Toluolsulfinsäure, Natriumtoluolsulfinat, Kaliumtoluolsulfinat, Benzolsulfinsäure, Natriumbenzolsulfinat und Kaliumbenzolsulfinat.

Bevorzugt ist ein erfindungsgemäßes Verfahren (vorzugsweise wie vorstehend als bevorzugt bezeichnet), wobei das Verhältnis der Gesamtstoffmenge sämtlicher Verbindungen (A) zu der Gesamtstoffmenge sämtlicher Verbindungen (S)
größer als 0,2, bevorzugt größer als 0,3, besonders bevorzugt größer als 1 ist
und/oder
im Bereich von 0,2 bis 50, bevorzugt im Bereich von 0,3 bis 20, besonders bevorzugt im Bereich von 1 bis 10 liegt.

Bevorzugt ist ein erfindungsgemäßes Verfahren (vorzugsweise wie vorstehend als bevorzugt bezeichnet), wobei das Verhältnis der kombinierten Gesamtmasse sämtlicher Verbindungen (A) und sämtlicher Verbindungen (S) zu der Gesamtmasse sämtlicher radikalisch polymerisierbarer organischer Monomere (M) im Bereich von 0,05 bis 10 Gew.-% liegt, vorzugsweise im Bereich von 0,5 bis 7 Gew.-%
und/oder
wobei der Gesamtanteil radikalisch polymerisierbarer organischer Monomere (M) im Bereich von 15 bis 95 Gew.-% liegt, vorzugsweise im Bereich von 25 bis 40 Gew.-%, bezogen auf die Gesamtmasse der Zusammensetzung
und/oder
wobei der kombinierte Gesamtanteil sämtlicher Verbindungen (A) und sämtlicher Verbindungen (S) im Bereich von 0,05 bis 7 Gew.-% liegt, vorzugsweise im Bereich von 0,1 bis 2,5 Gew.-%, bezogen auf die Gesamtmasse der Zusammensetzung.

Besonders bevorzugt ist, unter Berücksichtigung der vorstehenden Ausführungen, ein erfindungsgemäßes Verfahren (vorzugsweise wie vorstehend als bevorzugt bezeichnet),
wobei das eine oder zumindest eines der mehreren radikalisch polymerisierbaren organischen Monomere (M)
- ausgewählt ist aus der Gruppe bestehend aus Monomeren mit mindestens zwei ethylenischen Gruppen (M1), wobei die Monomere mit mindestens zwei ethylenischen Gruppen (M1) ausgewählt sind aus der Gruppe bestehend aus Ethylenglykoldimethacrylat, 1,6-Hexandioldimethacrylat, Triethylenglykoldimethacrylat, 1,12-Dodecandioldimethacrylat, ethoxyliertes Bisphenol-A-dimethacrylat, Polyethylenglykoldimethacrylat, 7,7,9-Trimethyl-4,13-dioxo-3,14-dioxa-5,12-diazahexadecan-1,16-dioxydimethacrylat, Butandioldimethacrylat, Trimethylolpropantrimethacrylat, Urethandimethacrylat, Glycerin-1,3-dimethacrylat, Tetraethylenglykoldimethacrylat, Neopentylglykoldimethacrylat, 2,2-Bis[4-[3-methacryloyloxy-2-hydroxypropoxy]phenyl]propan und den Di(meth)acrylaten des Dihydroxymethyltricyclo[5.2.1.0^{2,6}]decans
   und/oder
   wobei das eine oder zumindest eines der mehreren oder zumindest ein weiteres der mehreren radikalisch polymerisierbaren organischen Monomere (M)
- ausgewählt ist aus der Gruppe bestehend aus Hydroxylverbindungen mit mindestens einer ethylenischen Gruppe (M2), wobei die Hydroxylverbindungen mit mindestens einer ethylenischen Gruppe (M2) ausgewählt sind aus der Gruppe bestehend aus 2- Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat und 2,2-Bis[4-[3-methacryloyloxy-2-hydroxypropoxy]phenyl]propan
und/oder
wobei die eine oder zumindest eine der mehreren Verbindungen (A) ausgewählt ist aus der Gruppe bestehend aus Ascorbinsäure, Natriumascorbat, Isoascorbinsäure und Natriumisoascorbat
und/oder
wobei die eine oder zumindest eine der mehreren Verbindungen (S) ausgewählt ist aus der Gruppe bestehend aus Toluolsulfinsäure, Natriumtoluolsulfinat, Kaliumtoluolsulfinat, Benzolsulfinsäure, Natriumbenzolsulfinat und Kaliumbenzolsulfinat
und/oder
wobei das Verhältnis der Gesamtstoffmenge sämtlicher Verbindungen (A) zu der Gesamtstoffmenge sämtlicher Verbindungen (S)
größer als 1 ist
   und/oder
im Bereich von 1 bis 10 liegt
und/oder
wobei das Verhältnis der kombinierten Gesamtmasse sämtlicher Verbindungen (A) und sämtlicher Verbindungen (S) zu der Gesamtmasse sämtlicher radikalisch polymerisierbarer organischer Monomere (M) im Bereich von 0,5 bis 7 Gew.-% liegt
und/oder
wobei der Gesamtanteil radikalisch polymerisierbarer organischer Monomere (M) im Bereich von 25 bis 40 Gew.-% liegt, bezogen auf die Gesamtmasse der Zusammensetzung
und/oder
wobei der kombinierte Gesamtanteil sämtlicher Verbindungen (A) und sämtlicher Verbindungen (S) im Bereich von 0,1 bis 2,5 Gew.-% liegt, bezogen auf die Gesamtmasse der Zusammensetzung.

Vorzugsweise gilt hierbei für jede "und/oder"-Verknüpfung das "und".

Ein erfindungsgemäßes Verfahren umfasst zusätzlich den Schritt:

| | |
|---|---|
| Schritt (e) | Herstellen oder Bereitstellen einer basischen Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C), |

wobei Schritt (d) das Vermischen der Monomere (M) mit den Verbindungen (A) und (S) sowie mit der in Schritt (e) hergestellten oder bereitgestellten Glaszusammensetzung (G) umfasst.

Schritt (e) erfolgt vorzugsweise vor Beginn des Schrittes (d). Schritt (d) kann Teilschritte umfassen.

Durch das erfindungsgemäße Verfahren können erfindungsgemäße Zusammensetzungen als Komponente eines mehrkomponentigen kunststoffmodifizierten Glasionomerzement hergestellt werden, die bereits eine basische Glaszusammensetzung (G) umfassen und demnach effektiv in zweikomponentigen kunststoffmodifizierten Glasionomerzementen eingesetzt werden können.

Bevorzugt ist ein erfindungsgemäßes Verfahren (vorzugsweise wie vorstehend als bevorzugt bezeichnet), wobei die basische Glaszusammensetzung (G) ein oder mehrere Kationen von Elementen umfasst, die ausgewählt sind aus der Gruppe bestehend aus den Metallen der Hauptgruppen I, II und III des Periodensystems, vorzugsweise ausgewählt sind aus der Gruppe bestehend aus Natrium, Kalium, Calcium und Aluminium,
und die besonders bevorzugt vorliegen in Form einer oder mehrerer Verbindungen ausgewählt aus der Gruppe bestehend aus Oxiden, Hydroxiden, Sulfaten, Nitraten, Phosphaten, Carbonaten, Silikaten, Fluoriden und Nitriden
und/oder
wobei die basische Glaszusammensetzung (G) ein Aluminiumfluorosilikatglas umfasst
und/oder
wobei die basische Glaszusammensetzung (G) ein oder mehrere Gläser mit organisch modifizierter Oberfläche, vorzugsweise mit silanisierter Oberfläche, umfasst.

Entsprechende Verfahren sind ebenfalls vorteilhaft, weil mit ihnen bevorzugte Zusammensetzungen erhalten werden.

Bevorzugt ist ein erfindungsgemäßes Verfahren (vorzugsweise wie vorstehend als bevorzugt bezeichnet), zusätzlich umfassend den Schritt:
Schritt (f) Herstellen oder Bereitstellen eines oder mehrerer weiterer Bestandteile (B) ausgewählt aus der Gruppe bestehend aus Photoinitiatoren, Inhibitoren, Verbindungen (Z) und Additiven,
wobei Schritt (d) das Vermischen der Monomere (M) mit den Verbindungen (A) und (S) sowie mit dem einen oder den mehreren weiteren Bestandteilen (B) umfasst.

Hierbei sind
- die Photoinitiatoren vorzugsweise ausgewählt aus der Gruppe bestehend aus Benzoinalkylether, Benzoinalkylester, Benzilmonoketale, Acylphosphinoxide, Benzophenone, Acetophenone, Ketalen, Thioxanthone, Titanocene, aliphatische 1,2-Diketoverbindungen und aromatische 1,2-Diketoverbindungen, vorzugsweise ausgewählt ist aus der Gruppe umfassend 2,2-Diethoxyacetophenon, 9,10-Phenanthrenchinon, Diacetyl, Furil, Anisil, 4,4'-Dichlorbenzil und 4,4'-Dialkoxybenzil und Campherchinon,
- die Inhibitoren vorzugsweise ausgewählt aus der Gruppe bestehend aus substituierten Phenolen, Phenothiazin, stabile organische Radikale und Hydrochinonmonomethylether, besonders bevorzugt ausgewählt sind aus der Gruppe bestehend aus 2,6-Di-tert.-butyl-4-methylphenol, Hydrochinonmonomethylether, dem 2,2-Diphenyl-1-picrylhydrazylradikal, dem Galvinoxylradikal, dem Triphenylmethylradikal und dem 2,2,6,6-Tetramethylpiperidinyl-1-oxylradikal,
- die Verbindungen (Z) ausgewählt aus der Gruppe bestehend aus Barbitursäuren, tertiären Aminen und sekundären Aminen
   und
- die Additive ausgewählt aus der Gruppe bestehend aus Farbmitteln, Weinsäure, Kieselsäure, Stabilisatoren, Modifikatoren, bakterizid wirkenden Substanzen und inerten Füllstoffen. (Bevorzugte Additive sind vorstehend im Zusammenhang mit erfindungsgemäßen Zusammensetzungen definiert.)

Dieses erfindungsgemäße Verfahren ist bevorzugt, weil damit bevorzugte erfindungsgemäße Zusammensetzungen erhalten werden. Schritt (f) erfolgt vorzugsweise vor Beginn des Schrittes (d). Schritt (d) kann Teilschritte umfassen.

Bevorzugt ist ein erfindungsgemäßes Verfahren zur Herstellung eines gehärteten Dentalmaterials (vorzugsweise wie vorstehend als bevorzugt bezeichnet), zusätzlich umfassend den Schritt:
Schritt (g) Härten einer die hergestellte Zusammensetzung umfassenden Mischung, wobei das Härten vorzugsweise umfasst:
das Umsetzen der in der Mischung enthaltenen Verbindungen (A) und (S) mit einem oder mehreren Oxidationsmitteln (O) ausgewählt aus der Gruppe bestehend aus Persulfaten, Permanganaten, Perboraten, Peroxiden und Hydroperoxiden
   und/oder
das Bestrahlen der Mischung mit Licht einer Wellenlänge im Bereich von 300 bis 700 nm, vorzugsweise im Bereich von 350 bis 600 nm
   und/oder
das Umsetzen von in der Mischung enthaltenen carbonsäuregruppenhaltigen Polymeren (P) ausgewählt aus der Gruppe bestehend aus Homo- und Copolymeren einer α,β-ungesättigten Carbonsäure (C) mit einer in der Mischung enthaltenen basischen Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C), wobei die α,β-ungesättigte Carbonsäure (C) vorzugsweise ausgewählt ist aus der Gruppe bestehend aus Acrylsäure, Methacrylsäure, Itaconsäure, Fumarsäure, Maleinsäure, Chlormethacrylsäure, Cyanomethacrylsäure, Aconitsäure, Mesaconsäure, Glutaconsäure und Citraconsäure, vorzugsweise ausgewählt ist aus der Gruppe bestehend aus Acrylsäure und Maleinsäure und/oder wobei das gewichtsmittlere Molekulargewicht M_{w} der Gesamtmenge der carbonsäuregruppenhaltigen Polymere (P) vorzugsweise im Bereich von 1000 bis 150000 g/mol, vorzugsweise zwischen 40000 und 100000 g/mol, liegt.

"Härten" bedeutet, dass zumindest einige der Bestandteile der die hergestellte Zusammensetzung umfassenden Mischung zur Polymerisation gebracht werden. Das Härten beginnt mit der Initiierung der Polymerisation. Ein vollständiges Abreagieren sämtlicher polymerisierbarer Bestandteile der Zusammensetzung ist nicht nötig und wird in der Praxis regelmäßig nicht erreicht. Für bevorzugte erfindungsgemäße mehrkomponentige kunststoffmodifizierte Glasionomerzemente gilt, dass bereits unmittelbar nach dem Vermischen der Komponenten und mit Beginn der Polymerisation der Monomere (M) oder dem Beginn der GIZ-Abbindung das Härten beginnt.

Die in einem erfindungsgemäßen Verfahren hergestellte erfindungsgemäße Zusammensetzung wird vorzugsweise mit weiteren Bestandteilen vermischt, um eine Mischung herzustellen. Diese Mischung wird erfindungsgemäß in Schritt (g) gehärtet. Eine entsprechende Mischung wird beispielsweise erhalten, wenn die erste Komponente eines erfindungsgemäßen mehrkomponentigen kunststoffmodifizierten Glasionomerzements, die die erfindungsgemäße Zusammensetzung umfasst, mit einer einzelnen oder mehreren weiteren Komponenten des erfindungsgemäßen mehrkomponentigen kunststoffmodifizierten Glasionomerzements vermischt wird.

Beispielsweise kann das Vermischen der Komponenten für den Fall eines erfindungsgemäßen zweikomponentigen kunststoffmodifizierten Glasionomerzements in der Form Paste/Paste maschinell und automatisch mit Hilfe eines entsprechenden Kartuschensystems mit statischem Mischrohr zur Direktapplikation erfolgen. Bei der Direktapplikation werden die Komponenten zunächst in entsprechend dimensionierte Doppelkammerkartuschen abgefüllt. Die Herstellung einer die erfindungsgemäße Zusammensetzung umfassenden Mischung bei Applikation erfolgt dann durch Auspressen der Pasten aus der Doppelkammerkartusche mit Hilfe eines Doppelstempels durch eine statische Mischkanüle auf den zu behandelnden Zahn im Mundinnenraum. Eine unterschiedliche Einfärbung der beiden ungemischten Komponenten erlaubt es nach dem Durchgang der Pasten durch die Mischkanüle, anhand des resultierenden Farbtons die Homogenität der Mischung zu überprüfen.

Die Verarbeitung einer erfindungsgemäßen Zusammensetzung zu einer die erfindungsgemäße Zusammensetzung umfassenden Mischung kann selbstverständlich auch per Handanmischung erfolgen. Für den Fall eines erfindungsgemäßen zweikomponentigen kunststoffmodifizierten Glasionomerzements in der Form Paste/Flüssigkeit wird in der Regel ein sukzessiver Mischprozess ausgeführt, bei dem eine zuvor bestimmte Menge der Paste stufenweise mit einer zuvor bestimmten Menge der Flüssigkeit mit Hilfe eines Anmischinstruments miteinander vermengt wird. Analog kann auch im Falle der Form Paste/Paste vorgegangen werden.

Für den Fall eines erfindungsgemäßen zweikomponentigen kunststoffmodifizierten Glasionomerzements kann das Mischungsverhältnis der beiden Komponenten zwischen 10:1 und 1:10 betragen und liegt bevorzugt zwischen 1:4 und 4:1 und beträgt ganz besonders bevorzugt 1:1, bezogen auf die eingesetzten Masse der beiden Komponenten.

Die die hergestellte erfindungsgemäße Zusammensetzung umfassende Mischung muss gehärtet werden. Dies erfolgt bevorzugt auf zumindest einem, vorzugsweise zumindest zwei von drei nachfolgend erläuterten Wegen:
Wenn das Härten durch das Umsetzen der in der Mischung enthaltenen Verbindungen (A) und (S) mit einem oder mehreren Oxidationsmitteln (O) ausgewählt aus der Gruppe bestehend aus Persulfaten, Permanganaten, Perboraten, Peroxiden und Hydroperoxiden initiiert wird, handelt es sich um eine chemische Härtung mittels eines Redox-Polymerisationsinitiatorsystems. Eine solche Härtung wird auch als Selbsthärtung bezeichnet.

Wenn das Härten durch das Bestrahlen der Mischung mit Licht einer Wellenlänge im Bereich von 300 bis 700 nm, vorzugsweise im Bereich von 350 bis 600 nm initiiert wird, spricht der Fachmann von einer lichtinduzierten Härtung. Diese Härtung setzt die Anwesenheit zumindest eines Photoinitiators in der die hergestellte erfindungsgemäße Zusammensetzung umfassenden Mischung voraus.

Wenn das Härten durch das Umsetzen von in der Mischung enthaltenen carbonsäuregruppenhaltigen Polymeren (P) ausgewählt aus der Gruppe bestehend aus Homo- und Copolymeren einer α,β-ungesättigten Carbonsäure (C) mit einer in der Mischung enthaltenen basischen Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C) erfolgt, spricht der Fachmann von einer GIZ-Abbindung.

Bevorzugt erfolgt das Härten der die hergestellte Zusammensetzung umfassenden Mischung durch zumindest zwei der oben genannten drei Härtungsmechanismen, wobei die GIZ-Abbindung (wie vorstehend definiert) einen dieser zumindest zwei Härtungsmechanismen darstellt, und ganz besonders bevorzugt nach sämtlichen der oben genannten drei Härtungsmechanismen.

Demnach besonders bevorzugt ist ein erfindungsgemäßes Verfahren zur Herstellung eines gehärteten Dentalmaterials (vorzugsweise wie vorstehend als bevorzugt bezeichnet), zusätzlich umfassend den Schritt:

| | |
|---|---|
| Schritt (g) | Härten einer die hergestellte Zusammensetzung umfassenden Mischung, wobei das Härten vorzugsweise umfasst: |
| | das Umsetzen der in der Mischung enthaltenen Verbindungen (A) und (S) mit einem oder mehreren Oxidationsmitteln (O) ausgewählt aus der Gruppe bestehend aus Persulfaten, Permanganaten, Perboraten, Peroxiden und Hydroperoxiden |
| | und |
| | das Bestrahlen der Mischung mit Licht einer Wellenlänge im Bereich von 300 bis 700 nm, vorzugsweise im Bereich von 350 bis 600 nm |
| | und |
| | das Umsetzen von in der Mischung enthaltenen carbonsäuregruppenhaltigen Polymeren (P) ausgewählt aus der Gruppe bestehend aus Homo- und Copolymeren einer α,β-ungesättigten Carbonsäure (C) mit einer in der Mischung enthaltenen basischen Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C), |
| | wobei die α,β-ungesättigte Carbonsäure (C) vorzugsweise ausgewählt ist aus der Gruppe bestehend aus Acrylsäure, Methacrylsäure, Itaconsäure, Fumarsäure, Maleinsäure, Chlormethacrylsäure, Cyanomethacrylsäure, Aconitsäure, Mesaconsäure, Glutaconsäure und Citraconsäure, vorzugsweise ausgewählt ist aus der Gruppe bestehend aus Acrylsäure und Maleinsäure |
| | und/oder |
| | wobei das gewichtsmittlere Molekulargewicht M_{w} der Gesamtmenge der carbonsäuregruppenhaltigen Polymere (P) vorzugsweise im Bereich von 1000 bis 150000 g/mol, vorzugsweise zwischen 40000 und 100000 g/mol, liegt. |

Derartig bevorzugte erfindungsgemäße Verfahren zeigen in der Praxis regelmäßig eine überlegene Abbindekinetik für die hergestellte Mischung und erlauben auch nach mehrwöchiger Lagerung der zur Herstellung der Mischung benötigten Komponenten noch eine zuverlässige und schnelle Härtung. Die Härtung mit drei verschiedenen Härtungsmechanismen macht das Verfahren besonders unanfällig für Störungen, die lediglich einen der Härtungsmechanismen betreffen, wie beispielsweise ein Zerfall des Photoinitiators. Die die hergestellte Zusammensetzung umfassenden Mischungen, beispielsweise ein erfindungsgemäßer kunststoffmodifizierter Glasionomerzement, dessen ursprünglich separate Komponenten bereits gemischt vorliegen, können direkt im Mund des Patienten am Zahn appliziert werden oder auch außerhalb des Mundes beispielsweise an eine Prothese angewendet werden, wobei die Prothese anschließend im Mund verklebt wird.

Die vorliegende Erfindung betrifft auch die Verwendung einer erfindungsgemäßen Zusammensetzung (wie vorstehend definiert, vorzugsweise wie vorstehend als bevorzugt bezeichnet) als Komponente eines mehrkomponentigen kunststoffmodifizierten Glasionomerzements, bevorzugt eines erfindungsgemäßen mehrkomponentigen kunststoffmodifizierten Glasionomerzements.

Zudem betrifft die vorliegende Erfindung die Verwendung einer oder mehrerer Verbindungen (S) ausgewählt aus der Gruppe bestehend aus Sulfinsäuren, Salzen der Sulfinsäuren und Salzen bororganischer Verbindungen oder von Mischungen umfassend eine oder mehrere Verbindungen (S) ausgewählt aus der Gruppe bestehend aus Sulfinsäuren, Salzen der Sulfinsäuren und Salzen bororganischer Verbindungen, zur
(i) Erhöhung der Lagerstabilität einer Zusammensetzung umfassend
   - eine oder mehrere Verbindungen (A) ausgewählt aus der Gruppe bestehend aus den Isomeren der Ascorbinsäure, den Salzen der Isomere der Ascorbinsäure, den Estern der Isomere der Ascorbinsäure und den Ethern der Isomere der Ascorbinsäure (wie vorstehend im Zusammenhang mit anderen Aspekten der Erfindung definiert, vorzugsweise wie vorstehend als bevorzugt bezeichnet),
   - ein oder mehrere radikalisch polymerisierbare organische Monomere (M) (wie vorstehend im Zusammenhang mit anderen Aspekten der Erfindung definiert, vorzugsweise wie vorstehend als bevorzugt bezeichnet),
      und
   - eine basische Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C)
   und/oder
(ii) Herstellung einer erfindungsgemäßen Zusammensetzung (wie vorstehend definiert, vorzugsweise wie vorstehend als bevorzugt bezeichnet).

Überraschenderweise hat sich gezeigt, dass sich die Lagerstabilität einer Zusammensetzung umfassend eine oder mehrere Verbindungen (A) und ein oder mehrere radikalisch polymerisierbare organische Monomere (M) erhöhen lässt, wenn der Zusammensetzung eine oder mehrere Verbindungen (S) oder eine Mischung umfassend eine oder mehrere Verbindungen (S) zugesetzt werden. Dabei ist besonders überraschend, dass die negativen Auswirkungen der als Reduktionsmittel fungierenden Verbindungen (A) dadurch reduziert werden können, dass der Zusammensetzung eine oder mehrere Verbindungen (S) zugesetzt werden, obwohl diese regelmäßig selbst als Reduktionsmittel fungieren können.

Die erfindungsgemäße Verwendung betrifft auch die Herstellung erfindungsgemäßer Zusammensetzungen (wie vorstehend definiert, vorzugsweise wie vorstehend als bevorzugt bezeichnet).

Besonders bevorzugt ist die erfindungsgemäße Verwendung einer oder mehrerer Verbindungen (S) ausgewählt aus der Gruppe bestehend aus Sulfinsäuren, Salzen der Sulfinsäuren und Salzen bororganischer Verbindungen oder von Mischungen umfassend eine oder mehrere Verbindungen (S) ausgewählt aus der Gruppe bestehend aus Sulfinsäuren, Salzen der Sulfinsäuren und Salzen bororganischer Verbindungen, zur
(i) Erhöhung der Lagerstabilität einer Zusammensetzung umfassend
   - eine oder mehrere Verbindungen (A) ausgewählt aus der Gruppe bestehend aus den Isomeren der Ascorbinsäure, den Salzen der Isomere der Ascorbinsäure, den Estern der Isomere der Ascorbinsäure und den Ethern der Isomere der Ascorbinsäure,
   - ein oder mehrere radikalisch polymerisierbare organische Monomere (M), und
   - eine basische Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C)
   und
(ii) Herstellung einer erfindungsgemäßen Zusammensetzung.

Bevorzugt ist eine erfindungsgemäße Verwendung (vorzugsweise wie vorstehend als bevorzugt bezeichnet),
wobei die eine oder zumindest eine der mehreren Verbindungen (S) ausgewählt ist aus der Gruppe bestehend aus aromatischen Sulfinsäuren, Salzen der aromatischen Sulfinsäuren und Salzen aromatischer bororganischer Verbindungen, vorzugsweise ausgewählt ist aus der Gruppe bestehend aus Toluolsulfinsäure, Natriumtoluolsulfinat, Kaliumtoluolsulfinat, Benzolsulfinsäure, Natriumbenzolsulfinat, Kaliumbenzolsulfinat, Natriumtetraphenylborat und Lithiumtetraphenylborat, besonders bevorzugt ausgewählt ist aus der Gruppe bestehend aus Toluolsulfinsäure, Natriumtoluolsulfinat, Kaliumtoluolsulfinat, Benzolsulfinsäure, Natriumbenzolsulfinat und Kaliumbenzolsulfinat
und/oder
wobei die eine oder zumindest eine der mehreren Verbindungen (A) ausgewählt ist aus der Gruppe bestehend aus Ascorbinsäure, Salzen der Ascorbinsäure, Isoascorbinsäure und Salzen der Isoascorbinsäure, vorzugsweise ausgewählt ist aus der Gruppe bestehend aus Ascorbinsäure, Natriumascorbat, Isoascorbinsäure und Natriumisoascorbat
und/oder
wobei das eine oder zumindest eines der mehreren radikalisch polymerisierbaren organischen Monomere (M)
- ausgewählt ist aus der Gruppe bestehend aus Monomeren mit mindestens zwei ethylenischen Gruppen (M1),
   wobei die Monomere mit mindestens zwei ethylenischen Gruppen (M1) vorzugsweise ausgewählt sind aus der Gruppe bestehend aus Diacrylaten, Triacrylaten, Dimethacrylaten, Trimethacrylaten, besonders vorzugsweise ausgewählt sind aus der Gruppe bestehend aus Ethylenglykoldimethacrylat, 1,6-Hexandioldimethacrylat, Triethylenglykoldimethacrylat, 1,12-Dodecandioldimethacrylat, ethoxyliertes Bisphenol-A-dimethacrylat, Polyethylenglykoldimethacrylat, 7,7,9-Trimethyl-4,13-dioxo-3,14-dioxa-5,12-diazahexadecan-1,16-dioxydimethacrylat, Butandioldimethacrylat, Trimethylolpropantrimethacrylat, Urethandimethacrylat, Glycerin-1,3-dimethacrylat, Tetraethylenglykoldimethacrylat, Neopentylglykoldimethacrylat, 2,2-Bis[4-[3-methacryloyloxy-2-hydroxypropoxy]phenyl]propan und den Di(meth)acrylaten des Dihydroxymethyltricyclo[5.2.1.0^{2,6}]decans
   und/oder
   wobei das eine oder zumindest eines der mehreren oder zumindest ein weiteres der mehreren radikalisch polymerisierbaren organischen Monomere (M)
- ausgewählt ist aus der Gruppe bestehend aus Hydroxylverbindungen mit mindestens einer ethylenischen Gruppe (M2),
   wobei die Hydroxylverbindungen mit mindestens einer ethylenischen Gruppe (M2) vorzugsweise ausgewählt sind aus der Gruppe bestehend aus 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat und 2,2-Bis[4-[3-methacryloyloxy-2-hydroxypropoxy]phenyl]propan.

Wie vorstehend im Zusammenhang mit den entsprechend bevorzugten erfindungsgemäßen Zusammensetzungen erläutert, zeigt sich der überraschende technische Effekt der vorliegenden Erfindung besonders ausgeprägt bei Einsatz der vorstehend definierten Verbindungen und ganz besonders ausgeprägt bei Einsatz der vorstehend als bevorzugt bezeichneten Verbindungen. In eigenen Versuchen hat sich insbesondere gezeigt, dass die erfindungsgemäße Verwendung besonders vorteilhaft ist, wenn mehrere der als bevorzugt offenbarten Verbindungen kombiniert werden.

Besonders bevorzugt ist somit beispielsweise eine erfindungsgemäße Verwendung (vorzugsweise wie vorstehend als bevorzugt bezeichnet),
wobei die eine oder zumindest eine der mehreren Verbindungen (S) ausgewählt ist aus der Gruppe bestehend aus Toluolsulfinsäure, Natriumtoluolsulfinat, Kaliumtoluolsulfinat, Benzolsulfinsäure, Natriumbenzolsulfinat und Kaliumbenzolsulfinat
und
wobei die eine oder zumindest eine der mehreren Verbindungen (A) ausgewählt ist aus der Gruppe bestehend aus Ascorbinsäure, Natriumascorbat, Isoascorbinsäure und Natriumisoascorbat.

Diese Kombination von Verbindungen (S) und (A) ist im Rahmen der vorliegenden Erfindung generell besonders bevorzugt.

Bei der erfindungsgemäßen Verwendung zeigt sich die überraschende Wirkung am besonders deutlich für die Verhältnisse an Massen und Stoffmengen, die vorstehend bereits im Zusammenhang mit erfindungsgemäßen Zusammensetzungen als bevorzugt offenbart wurden. Bevorzugt ist somit eine erfindungsgemäße Verwendung (vorzugsweise wie vorstehend als bevorzugt bezeichnet),
wobei das Verhältnis der Gesamtstoffmenge sämtlicher Verbindungen (A) zu der Gesamtstoffmenge sämtlicher Verbindungen (S)
größer als 0,2, bevorzugt größer als 0,3, besonders bevorzugt größer als 1 ist und/oder
im Bereich von 0,2 bis 50, bevorzugt im Bereich von 0,3 bis 20, besonders bevorzugt im Bereich von 1 bis 10 liegt
und/oder
wobei das Verhältnis der kombinierten Gesamtmasse sämtlicher Verbindungen (A) und sämtlicher Verbindungen (S) zu der Gesamtmasse sämtlicher radikalisch polymerisierbarer organischer Monomere (M) im Bereich von 0,05 bis 10 Gew.-% liegt, vorzugsweise im Bereich von 0,5 bis 7 Gew.-%
und/oder
wobei der Gesamtanteil radikalisch polymerisierbarer organischer Monomere (M) im Bereich von 15 bis 95 Gew.-% liegt, vorzugsweise im Bereich von 25 bis 40 Gew.-%, bezogen auf die Gesamtmasse der Zusammensetzung
und/oder
wobei der kombinierte Gesamtanteil sämtlicher Verbindungen (A) und sämtlicher Verbindungen (S) im Bereich von 0,05 bis 7 Gew.-% liegt, vorzugsweise im Bereich von 0,1 bis 2,5 Gew.-%, bezogen auf die Gesamtmasse der Zusammensetzung.

Eng mit der vorliegenden erfinderischen Verwendung verknüpft, ist ein Verfahren zur Erhöhung der Lagerstabilität einer Zusammensetzung umfassend
- eine oder mehrere Verbindungen (A) ausgewählt aus der Gruppe bestehend aus den Isomeren der Ascorbinsäure, den Salzen der Isomere der Ascorbinsäure, den Estern der Isomere der Ascorbinsäure und den Ethern der Isomere der Ascorbinsäure,
- ein oder mehrere radikalisch polymerisierbare organische Monomere (M) (wie vorstehend im Zusammenhang mit anderen Aspekten der Erfindung definiert, vorzugsweise wie vorstehend als bevorzugt bezeichnet), und
- eine basische Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C) mit folgendem Schritt:
- Vermischen der Zusammensetzung mit einer oder mehreren Verbindungen (S) ausgewählt aus der Gruppe bestehend aus Sulfinsäuren, Salzen der Sulfinsäuren und Salzen bororganischer Verbindungen (wie vorstehend auch im Zusammenhang mit anderen Aspekten der Erfindung definiert, vorzugsweise wie vorstehend als bevorzugt bezeichnet).

Das entsprechende Verfahren ist, wie auch die erfindungsgemäße Verwendung, besonders vorteilhaft, weil damit (Basis-)Zusammensetzungen, deren Lagerstabilität mangelhaft ist, so modifiziert werden können, dass eine Zusammensetzung mit verbesserter Lagerstabilität erhalten wird.

Besonders bevorzugt sind in dem vorstehend offenbarten Verfahren die eine oder zumindest eine der Verbindungen (S) und/oder die eine oder zumindest eine der Verbindungen (A) und/oder das eine oder zumindest eines der radikalisch polymerisierbaren organischen Monomere (M) so definiert, wie es vorstehend für bevorzugte erfindungsgemäße Zusammensetzungen, bevorzugte erfindungsgemäße Kits, bevorzugte erfindungsgemäße Verfahren oder bevorzugte erfindungsgemäße Verwendungen definiert ist.

Offenbart wird auch ein gehärtetes Dentalmaterial umfassend
- ein oder mehr Oxidationsprodukte einer oder mehrerer Verbindungen (A) ausgewählt aus der Gruppe bestehend aus den Isomeren der Ascorbinsäure, den Salzen der Isomere der Ascorbinsäure, den Estern der Isomere der Ascorbinsäure und den Ethern der Isomere der Ascorbinsäure (wie vorstehend im Zusammenhang mit anderen Aspekten der Erfindung definiert, vorzugsweise wie vorstehend als bevorzugt bezeichnet),
- ein oder mehr Oxidationsprodukte einer oder mehrerer Verbindungen (S) ausgewählt aus der Gruppe bestehend aus Sulfinsäuren, Salzen der Sulfinsäuren und Salzen bororganischer Verbindungen (wie vorstehend im Zusammenhang mit anderen Aspekten der Erfindung definiert, vorzugsweise wie vorstehend als bevorzugt bezeichnet),
- ein Polymer umfassend Einheiten von (co-)polymerisierten radikalisch polymerisierbaren organischen Monomeren (M) (wie vorstehend im Zusammenhang mit anderen Aspekten der Erfindung definiert, vorzugsweise wie vorstehend als bevorzugt bezeichnet),
   und
- das Produkt der Vernetzung von ein oder mehreren carbonsäuregruppenhaltigen Polymeren (P) ausgewählt aus der Gruppe bestehend aus Homo- und Copolymeren einer α,β-ungesättigten Carbonsäure (C), mit einer basischen Glaszusammensetzung (G).

Ein gehärtetes Dentalmaterial, d.h. ein Dentalmaterial, in dem die Härtung zumindest teilweise bereits eingesetzt hat, das unter Einsatz einer erfindungsgemäßen Zusammensetzung, aus einem erfindungsgemäßen Kit oder mit Hilfe eines erfindungsgemäßen Verfahrens hergestellt wird, besitzt die vorstehend beschriebenen Merkmale, so dass seine Herstellungshistorie nachvollzogen werden kann. Besonders vorteilhaft ist hierbei, dass die Oxidationsprodukte der Verbindungen (A) und (S), d.h. die chemischen Verbindungen, zu denen die Verbindungen (A) und (S) jeweils umgesetzt werden, wenn sie als Reduktionsmittel in einem Redox-Polymerisationsinitiatorsystem reagieren und somit selbst oxidiert werden, keine negativen Auswirkungen auf die mechanischen Eigenschaften und/oder die Verträglichkeit und/oder das Alterungsverhalten der gehärteten Dentalmaterialien haben.

Aus den bevorzugten Ausführungsformen der erfindungsgemäßen Zusammensetzungen, der erfindungsgemäßen Kits, dem erfindungsgemäßen Verfahren und der erfindungsgemäßen Verwendung folgt, dass gehärtete Dentalmaterialien gemäß der vorstehenden Definition bevorzugt sind,
wobei die eine oder zumindest eine der mehreren Verbindungen (S) ausgewählt ist aus der Gruppe bestehend aus aromatischen Sulfinsäuren, Salzen der aromatischen Sulfinsäuren und Salzen aromatischer bororganischer Verbindungen, vorzugsweise ausgewählt ist aus der Gruppe bestehend aus Toluolsulfinsäure, Natriumtoluolsulfinat, Kaliumtoluolsulfinat, Benzolsulfinsäure, Natriumbenzolsulfinat, Kaliumbenzolsulfinat, Natriumtetraphenylborat und Lithiumtetraphenylborat, besonders bevorzugt ausgewählt ist aus der Gruppe bestehend aus Toluolsulfinsäure, Natriumtoluolsulfinat, Kaliumtoluolsulfinat, Benzolsulfinsäure, Natriumbenzolsulfinat und Kaliumbenzolsulfinat
und/oder
wobei die eine oder zumindest eine der mehreren Verbindungen (A) ausgewählt ist aus der Gruppe bestehend aus Ascorbinsäure, Salzen der Ascorbinsäure, Isoascorbinsäure und Salzen der Isoascorbinsäure, vorzugsweise ausgewählt ist aus der Gruppe bestehend aus Ascorbinsäure, Natriumascorbat, Isoascorbinsäure und Natriumisoascorbat
und/oder
wobei das eine oder zumindest eines der mehreren radikalisch polymerisierbaren organischen Monomere (M)
- ausgewählt ist aus der Gruppe bestehend aus Monomeren mit mindestens zwei ethylenischen Gruppen (M1),
   wobei die Monomere mit mindestens zwei ethylenischen Gruppen (M1) vorzugsweise ausgewählt sind aus der Gruppe bestehend aus Diacrylaten, Triacrylaten, Dimethacrylaten, Trimethacrylaten, besonders vorzugsweise ausgewählt sind aus der Gruppe bestehend aus Ethylenglykoldimethacrylat, 1,6-Hexandioldimethacrylat, Triethylenglykoldimethacrylat, 1,12-Dodecandioldimethacrylat, ethoxyliertes Bisphenol-A-dimethacrylat, Polyethylenglykoldimethacrylat, 7,7,9-Trimethyl-4,13-dioxo-3,14-dioxa-5,12-diazahexadecan-1,16-dioxydimethacrylat, Butandioldimethacrylat, Trimethylolpropantrimethacrylat, Urethandimethacrylat, Glycerin-1,3-dimethacrylat, Tetraethylenglykoldimethacrylat, Neopentylglykoldimethacrylat, 2,2-Bis[4-[3-methacryloyloxy-2-hydroxypropoxy]phenyl]propan und den Di(meth)acrylaten des Dihydroxymethyltricyclo[5.2.1.0^{2,6}]decans
   und/oder
   wobei das eine oder zumindest eines der mehreren oder zumindest ein weiteres der mehreren radikalisch polymerisierbaren organischen Monomere (M)
- ausgewählt ist aus der Gruppe bestehend aus Hydroxylverbindungen mit mindestens einer ethylenischen Gruppe (M2),
   wobei die Hydroxylverbindungen mit mindestens einer ethylenischen Gruppe (M2) vorzugsweise ausgewählt sind aus der Gruppe bestehend aus 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat und 2,2-Bis[4-[3-methacryloyloxy-2-hydroxypropoxy]phenyl]propan.

Aus den bevorzugten Ausführungsformen der erfindungsgemäßen Zusammensetzungen, der erfindungsgemäßen Kits, dem erfindungsgemäßen Verfahren und der erfindungsgemäßen Verwendung folgt zudem, dass gehärtete Dentalmaterialien (vorzugsweise wie vorstehend als bevorzugt bezeichnet) bevorzugt sind,
die das Produkt der Vernetzung von ein oder mehreren carbonsäuregruppenhaltigen Polymeren (P) ausgewählt aus der Gruppe bestehend aus Homo- und Copolymeren einer α,β-ungesättigten Carbonsäure (C), mit einer basischen Glaszusammensetzung (G) umfassen,
wobei die α,β-ungesättigte Carbonsäure (C) ausgewählt ist aus der Gruppe bestehend aus Acrylsäure, Methacrylsäure, Itaconsäure, Fumarsäure, Maleinsäure, Chlormethacrylsäure, Cyanomethacrylsäure, Aconitsäure, Mesaconsäure, Glutaconsäure und Citraconsäure, vorzugsweise ausgewählt ist aus der Gruppe bestehend aus Acrylsäure und Maleinsäure,
wobei das gewichtsmittlere Molekulargewicht Mw der Gesamtmenge der carbonsäuregruppenhaltigen Polymere (P) im Bereich von 1000 bis 150000 g/mol, vorzugsweise zwischen 40000 und 100000 g/mol, liegt,
wobei die basische Glaszusammensetzung (G) vorzugsweise
   ein oder mehrere Kationen von Elementen umfasst, die ausgewählt sind aus der Gruppe bestehend aus den Metallen der Hauptgruppen I, II und III des Periodensystems, vorzugsweise ausgewählt sind aus der Gruppe bestehend aus Natrium, Kalium, Calcium und Aluminium,
      und die besonders bevorzugt vorliegen in Form einer oder mehrerer Verbindungen ausgewählt aus der Gruppe bestehend aus Oxiden, Hydroxiden, Sulfaten, Nitraten, Phosphaten, Carbonaten, Silikaten, Fluoriden und Nitriden
      und/oder
   ein Aluminiumfluorosilikatglas umfasst
      und/oder
   ein oder mehrere Gläser mit organisch modifizierter Oberfläche, vorzugsweise mit silanisierter Oberfläche, umfasst.

Die vorstehend im Zusammenhang mit anderen Aspekten der Erfindung definierten bevorzugten Ausgestaltungen sind insoweit entsprechend bevorzugt, *mutatis mutandis.*

Bevorzugt ist ein vorstehend definiertes gehärtetes Dentalmaterial (vorzugsweise wie vorstehend als bevorzugt bezeichnet), herstellbar gemäß einem erfindungsgemäßen Verfahren.

Im Folgenden wird die Erfindung anhand von Beispielen näher beschrieben, wobei die Beispiele solche erfindungsgemäßen Zusammensetzungen bzw. erfindungsgemäßen zweikomponentigen kunststoffmodifizierten Glasionomerzemente betrifft, die für den Einsatz als Befestigungszemente optimiert sind. Der Fachmann weiß jedoch, wie er eine dentale Grundzusammensetzung für die unterschiedlichen dentalen Anwendungen modifiziert. Die mechanischen Eigenschaften von dentalen Zusammensetzungen müssen in Abhängigkeit von den Indikationen unterschiedlich sein. So benötigt ein Befestigungszement keine niedrigen Abrasionswerte. Umgekehrt braucht ein Füllungsmaterial keine geringe Filmdicke. Beide Systeme brauchen jedoch eine gute Haftung. Der Fachmann ist in der Lage, diese Eigenschaften einzustellen. So würde er für ein Füllungsmaterial einen höheren Füllstoffgehalt mit einer ganz anderen Korngrößenverteilung im Vergleich zu einem Befestigungszement wählen.

### Beispiele

### Herstellung der Ausgangskomponenten:

Als Ausgangspunkt für die durchgeführten Experimente wurden zunächst zwei separate Komponenten in Form von Pasten hergestellt. Diese Komponenten werden nachfolgend als Paste A und Paste B bezeichnet. Die Zusammensetzung der Paste A und der Paste B lässt sich jeweils der nachfolgenden Tabelle 1 entnehmen.

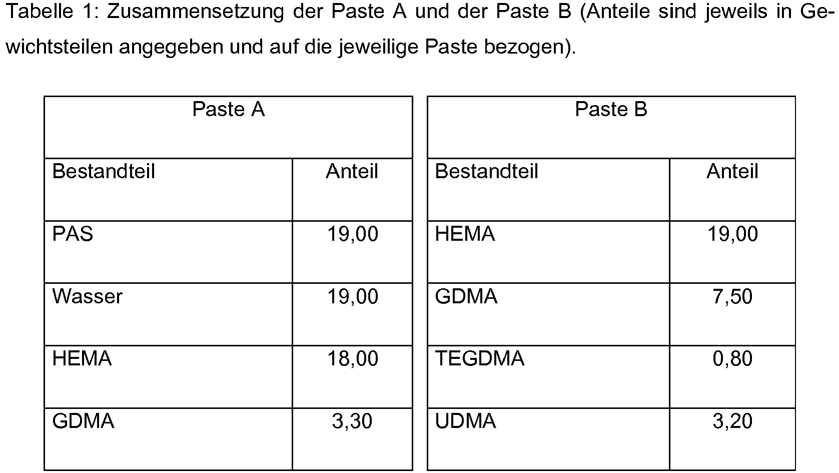

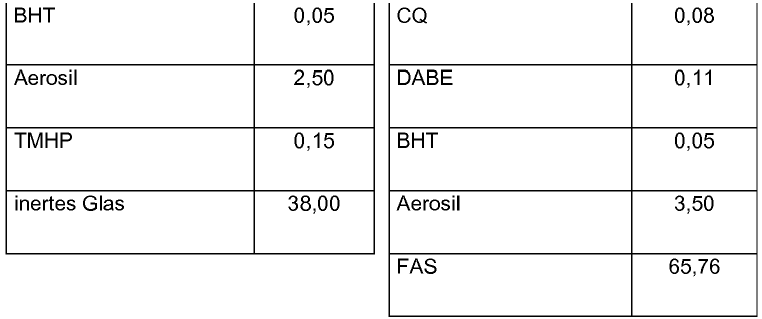

Die in Tabelle 1 verwendeten Abkürzungen sind in Tabelle 2 den entsprechenden chemischen Verbindungen zugeordnet.

**Tabelle 2: Verwendete Abkürzungen.**

| Abkürzung | Verbindung | Funktion |
|---|---|---|
| PAS | Polyacrylsäure | Carbonsäuregruppenhaltiges Polymer (P) |
| Wasser | H₂O (demineralisiert) | |
| HEMA | 2-Hydroxyethylmethacrylat | Monomer (M) |
| GDMA | Glycerin-1,3-dimethacrylat | Monomer (M) |
| TEGDMA | Triethylenglycoldimethacrylat | Monomer (M) |
| UDMA | Urethandimethacrylat | Monomer (M) |
| Aerosil | pyrogene Kieselsäure | Inerter Füllstoff |
| FAS | Aluminiumfluorosilikatglas | Basische Glaszusammensetzung (G) |
| BHT | 2,6-Di(tert-butyl)hydroxytoluol | Inhibitor |
| CQ | DL-Kampferchinon | Photoinitiator |
| DABE | 4-Dimethylaminobenzoesäureethylester | Reduktionsmittel; Verbindung (Z) |
| TMHP | 1,1,3,3-Tetramethylbutylhydroperoxid | Oxidationsmittel (O) |

Für die Herstellung der Pasten A und B wurden die Bestandteile jeweils eingewogen, an einem Mischer (SpeedMixer™ DAC 600.1 VAC-P, Hauschild & Co KG, Hamm, Deutschland) homogenisiert, an einem Dreiwalzenstuhl (Exakt, Norderstedt, Deutschland) gewalzt und anschließend an einem Vakuum-Mischer (SpeedMixer™ DAC 600.1 VAC-P) bei -0,9 bar Vakuum entlüftet.

### Herstellung der untersuchten Proben:

Ausgehend von Paste B wurden durch das Vermischen mit weiteren Bestandteilen anspruchsgemäße Zusammensetzungen in Form von Pasten (z.B. B-E2), d.h. Zusammensetzungen zur Verwendung als Komponente eines mehrkomponentigen kunststoffmodifizierten Glasionomerzements sowie Vergleichszusammensetzungen (z.B. B-V1), die an den Stand der Technik angelehnt sind, hergestellt. Hierfür wurde jeweils eine bestimmte Menge der hergestellten Paste B mit einer oder mehreren weiteren Substanzen vermischt. Die chemische Zusammensetzung der auf diesem Weg hergestellten Zusammensetzungen (modifizierte Paste B) ist in den Tabellen 3 und 4 eingetragen.

Die in den Tabellen 3 und 4 verwendeten Abkürzungen sind in Tabelle 5 den entsprechenden chemischen Verbindungen zugeordnet.

**Tabelle 3: Zusammensetzung der ausgehend von Paste B hergestellten Zusammensetzungen (modifizierte Paste B; erfindungsgemäß; Anteile sind jeweils in Gewichtsteilen angegeben und auf die jeweilige Zusammensetzung bezogen).**

| Bestandteil | B-E1 | B-E2 | B-E3 | B-E4 | B-E5 | B-E6 |
|---|---|---|---|---|---|---|
| Paste B | 99,47 | 99,47 | 99,47 | 99,47 | 99,41 | 99,23 |
| AS | 0,50 | 0,48 | 0,26 | 0,13 | | 0,26 |
| NalA | | | | | 0,32 | |
| NaTS | 0,03 | 0,05 | 0,27 | 0,40 | 0,27 | |
| NaTPB | | | | | | 0,51 |

**Tabelle 4: Zusammensetzung der hergestellten Zusammensetzungen (modifizierte Paste B; Vergleich; Anteile sind jeweils in Gewichtsteilen angegeben und auf die jeweilige Zusammensetzung bezogen).**

| Bestandteil | B-V1 | B-V2 | B-V3 | B-V4 | B-V5 | B-V6 | B-V7 |
|---|---|---|---|---|---|---|---|
| Paste B | 99,47 | 99,46 | 99,55 | 99,54 | 99,47 | 99,46 | 99,45 |
| AS | 0,53 | | 0,26 | | 0,48 | | |
| NaTS | | 0,54 | | 0,27 | | 0,49 | 0,45 |
| OS | | | 0,19 | 0,19 | | | |
| NaDS | | | | | 0,05 | | |
| Fe(II) | | | | | | 0,05 | 0,10 |

**Tabelle 5: Verwendete Abkürzungen.**

| Abkürzung | Verbindung |
|---|---|
| AS | Ascorbinsäure |
| NalA | Natriumisoascorbat |
| NaTS | Natrium-p-toluolsulfinat |
| NaTPB | Natriumtetraphenylborat |
| NaDS | Natriumdisulfit |
| Fe(II) | Eisen(II)chlorid Tetrahydrat |
| OS | Oxalsäure |

Für die Herstellung der Zusammensetzungen wurden die Bestandteile jeweils eingewogen, an einem Mischer (SpeedMixer™ DAC 600.1 VAC-P, Hauschild & Co KG, Hamm, Deutschland) homogenisiert, an einem Dreiwalzenstuhl (Exakt, Norderstedt, Deutschland) gewalzt und anschließend an einem Vakuum-Mischer (SpeedMixer™ DAC 600.1 VAC-P) bei -0,9 bar Vakuum entlüftet.

In den Zusammensetzungen, die neben der Paste B noch mehr als einen weiteren Bestandteil umfassen, sind die Stoffmengenverhältnisse der weiteren Bestandteile in Tabelle 6 zusammengefasst.

**Tabelle 6: Stoffmengenverhältnis der der Paste B bei der Probenherstellung zugesetzten Bestandteile.**

| Probe | Bestandteil 1 | Stoffmengenverhältnis | Bestandteil 2 |
|---|---|---|---|
| B-E1 | AS | 20 : 1 | NaTS |
| B-E2 | AS | 10 : 1 | NaTS |
| B-E3 | AS | 1 : 1 | NaTS |
| B-E4 | AS | 1 : 3 | NaTS |
| B-E5 | NalA | 1 : 1 | NaTS |
| B-E6 | AS | 1 : 1 | NaTPB |
| B-V3 | AS | 1 : 1 | OS |
| B-V4 | NaTS | 1 : 1 | OS |
| B-V5 | AS | 10 : 1 | NaDS |
| B-V6 | NaTS | 10 : 1 | Fe(II) |
| B-V7 | NaTS | 5 : 1 | Fe(II) |

Jede der hergestellten Zusammensetzungen (erfindungsgemäße und Vergleichsbeispiele) ist eine Zusammensetzung zur Verwendung als Komponente eines mehrkomponentigen kunststoffmodifizierten Glasionomerzements.

Jede der hergestellten Zusammensetzungen (erfindungsgemäße und Vergleichsbeispiele) ist jeweils eine Komponente eines zweikomponentigen kunststoffmodifizierten Glasionomerzements und bildet jeweils mit der gleichen Menge der Paste A einen zweikomponentigen kunststoffmodifizierten Glasionomerzement. Dies bedeutet, dass beispielsweise die erfindungsgemäße Zusammensetzung B-E2 zusammen mit der Paste A einen zweikomponentigen kunststoffmodifizierten Glasionomerzement bildet, ebenso wie die Vergleichszusammensetzung B-V5 mit der Paste A einen zweikomponentigen kunststoffmodifizierten Glasionomerzement bildet.

### Versuchsdurchführung:

Die hergestellten zweikomponentigen kunststoffmodifizierten Glasionomerzemente (modifizierte Paste B; Paste A) wurden in Doppelkammerspritzen (5 ml, 1:1, SDL X05-01-52, PED X05-01-SI, Sulzer Mixpac AG, Haag, Schweiz) abgefüllt. Nach dem Abfüllen wurden die Doppelkammerspritzen bei einer Temperatur von 37 °C gelagert.

Nach definierten Intervallen (initial, d.h. direkt nach der Abfüllung sowie nach 1, 2, 4, 6, 8, 10 und 12 Wochen) wurden die Spritzen entnommen und nach Temperierung auf Raumtemperatur jeweils - soweit möglich - die Abbindezeit und die Biegefestigkeit des durch Vermischen aus den Komponenten hergestellten gehärteten kunststoffmodifizierten Glasionomerzements bestimmt (die Bestimmungsmethoden sind weiter unten angegeben). Zum Mischen wurden die passenden, statischen Mischer (ML 2.5-16-S, Sulzer Mixpac AG) auf die Spritzen aufgesetzt und - soweit möglich - die beiden jeweiligen Komponenten der zweikomponentigen kunststoffmodifizierten Glasionomerzemente mit einem Stössel (PLH X05-01-46) ausgedrückt und homogen im Verhältnis 1:1 gemischt.

Die Lagerstabilität einer Zusammensetzung ist primär davon abhängig, ob sie ohne Vermischen mit einer jeweils weiteren Komponente über einen bestimmten Zeitraum hinweg ohne signifikante Polymerisation, d. h. chemisch nahezu unverändert, beständig ist oder nicht. Eine Zusammensetzung (insbesondere Paste), welche unter den vorstehend genannten Lagerbedingungen auch nach zwölf Wochen noch nicht gehärtet (polymerisiert) ist, ist lagerstabiler als eine Zusammensetzung (Paste), welche bereits nach z. B. sechs Wochen so gehärtet (polymerisiert) ist, dass ein Vermischen mit der zugeordneten weiteren Komponente nicht mehr möglich ist.

Sofern eine Zusammensetzung zu einem bestimmten Zeitpunkt mit einer zugeordneten weiteren Komponente vermischt und der resultierende Glasionomerzement gehärtet werden kann, sind die Aushärtezeit und die Biegefestigkeit des gehärteten Glasionomerzements weitere Indikatoren für die Lagerstabilität der untersuchten Zusammensetzung (Paste).

### Bestimmung der Abbindezeit:

Zur Bestimmung der Abbindezeit wurde das zu untersuchende Material ausschließlich chemisch gehärtet. Die Abbindezeit wurde an einem Rheometer (Physica MCR 301, Anton Paar GmbH, Graz, Österreich) bestimmt. Dazu wurde bei 37 °C in einer Oszillationsmessung (Auslenkung/Amplitude= 1%, f = 4 Hz) die Viskosität in Abhängigkeit von der Zeit aufgenommen. Der kunststoffmodifizierte Glasionomerzement wurde wie vorstehend beschrieben angemischt und auf die Messplatte eines Platte/Platte-Systems aufgebracht (D = 12 mm, Spalt = 1 mm). Die Zeit wurde mit Mischbeginn gestartet. Als Abbindezeit wurde die Zeit vom Mischbeginn bis zum Erreichen einer Viskosität von 10⁴ Pa·s definiert.

### Bestimmung der Biegefestigkeit:

Zur Bestimmung der Biegefestigkeit wurde das zu untersuchende Material ausschließlich chemisch gehärtet. Die Bestimmung der Biegefestigkeit erfolgte gemäß ISO 9917-2:2010. Der Glasionomerzement wurde wie vorstehend beschrieben angemischt und jeweils luftblasenfrei in entsprechende Teflonformen gefüllt, mit Folie und Glasplatte bedeckt. Mit einer Schraubzwinge wurden die Überschüsse herausgepresst. Die Probekörper wurden 24 Stunden bei 37 °C im Wasserbad gehärtet. Die Maße des Probekörpers (2 mm x 2 mm) wurden in der Mitte mit einer Messgenauigkeit von 0,01 mm gemessen. Anschließend wurden die Probekörper mit einer Universalprüfmaschine (Zwick GmbH, Ulm, Deutschland) mit einer Vorschubgeschwindigkeit von 0,75 mm/min bis zum Bruch belastet.

### Ergebnisse:

Die Ergebnisse der Experimente sind in den nachfolgenden Tabelle 7 (Biegefestigkeit) und 8 (Abbindezeit) zusammengefasst.

**Tabelle 7: Ergebnisse Biegefestigkeit.**

| | Biegefestigkeit / MPa | | | | | | | | | | | | |
|---|---|---|---|---|---|---|---|---|---|---|---|---|---|
| Lagerdauer | B-E1 | B-E2 | B-E3 | B-E4 | B-E5 | B-E6 | B-V1 | B-V2 | B-V3 | B-V4 | B-V5 | B-V6 | B-V7 |
| Initial | 24,7 | 23,6 | 22,3 | 25,5 | 25,2 | 26,8 | 24,9 | Y | P | Y | 20,2 | P | P |
| 1. Woche | 27,7 | 26,3 | 26,2 | 23,0 | 24,9 | 23,7 | 22,7 | - | - | - | 21,4 | - | - |
| 2. Woche | 25,9 | 27,1 | 26,7 | 18,6 | 23,9 | 27,3 | 25,9 | - | - | - | 24,8 | - | - |
| 4. Woche | 28,3 | 27,2 | 18,3 | 27,0 | 24,9 | 26,9 | 23,6 | - | - | - | 21,9 | - | - |
| 6. Woche | 27,5 | 22,1 | 18,9 | 19,0 | 25,1 | 23,7 | P | - | - | - | P | - | - |
| 8. Woche | 27,1 | 28,7 | 14,9 | 18,3 | 26,8 | 25,1 | - | - | - | - | - | - | - |
| 10. Woche | 21,9 | 23,5 | 9,6 | 12,0 | 25,2 | 23,9 | - | - | - | - | - | - | - |
| 12. Woche | 8,1 | 27,0 | 7,3 | X | 18,9 | 22,3 | - | - | - | - | - | - | - |

**Tabelle 8: Ergebnisse Abbindezeit.**

| | Abbindezeit / s | | | | | | | | | | | | |
|---|---|---|---|---|---|---|---|---|---|---|---|---|---|
| Lagerdauer | B-E1 | B-E2 | B-E3 | B-E4 | B-E5 | B-E6 | B-V1 | B-V2 | B-V3 | B-V4 | B-V5 | B-V6 | B-V7 |
| Initial | 113 | 110 | 120 | 153 | 135 | 73 | 120 | Y | P | Y | 72 | P | P |
| 1. Woche | 107 | 130 | 135 | 175 | 145 | 110 | 114 | - | - | - | 50 | - | - |
| 2. Woche | 104 | 129 | 153 | 162 | 148 | 125 | 117 | - | - | - | 61 | - | - |
| 4. Woche | 116 | 120 | 187 | 196 | 160 | 132 | 90 | - | - | - | 101 | - | - |
| 6. Woche | 107 | 123 | 213 | 189 | 154 | 145 | P | - | - | - | P | - | - |
| 8. Woche | 115 | 116 | 234 | 243 | 146 | 164 | - | - | - | - | - | - | - |
| 10. Woche | 211 | 135 | 259 | 298 | 166 | 162 | - | - | - | - | - | - | - |
| 12. Woche | 281 | 170 | 267 | 326 | 175 | 168 | - | - | - | - | - | - | - |

In den Tabellen 7 und 8 sind besondere experimentelle Befunde mit den Buchstaben "X", "Y" oder "P" kenntlich gemacht.

Der Eintrag "X" bedeutet, dass der entsprechende zweikomponentige kunststoffmodifizierte Glasionomerzement zwar nach dem Vermischen noch eine Viskosität von 10⁴ Pa·s erreichte, so dass eine Abbindezeit bestimmt werden konnte, sich aber kein hinreichend fester Prüfkörper für die Bestimmung der Biegefestigkeit erhalten ließ.

Der Eintrag "P" bedeutet, dass die entsprechende Zusammensetzung während der Lagerung polymerisiert, d.h. gehärtet, ist. Dies bedeutet, dass die entsprechende Zusammensetzung nicht mehr aus den Doppelkammerspritzen ausgedrückt werden konnte und sich somit unter den gewählten experimentellen Bedingungen nicht mit der Paste A vermischen ließ. Der Eintrag "P" kennzeichnet somit den vollständigen Verlust der Lagerstabilität.

Der Eintrag "Y" bedeutet, dass die entsprechende Zusammensetzung durch das Vermischen mit der spezifischen Paste A nicht gehärtet werden konnte; weder eine Abbindezeit noch eine Biegefestigkeit konnten bestimmt werden. Der Eintrag "Y" kennzeichnet somit Proben, deren Bestandteile die Anforderungen an ein Initiatorsystem nicht erfüllen.

Die in den Tabelle 7 und 8 zusammengefassten Versuchsergebnissen belegen die nachfolgend angeführten Effekte.

Die eng an den Stand der Technik angelehnte Zusammensetzung B-V1, die eine Vergleichszusammensetzung von besonderer Bedeutung ist, ist nach 6 Wochen der Lagerung polymerisiert. Dies bedeutet, dass die Zusammensetzung ab diesem Moment unter den gewählten experimentellen Bedingungen unbrauchbar ist; die Lagerstabilität kann als "mittelmäßig" bewertet werden.

Bereits wenn (ausgehend von B-V1) erfindungsgemäß ein kleiner Anteil AS durch NaTS ausgetauscht wird, zeigt sich hingegen eine ausgeprägte Verbesserung der Lagerstabilität und auch nach 12 Wochen können entsprechende erfindungsgemäße Zusammensetzungen noch gehärtet werden (vgl. insbesondere B-E1 bis B-E4).

Hierbei wird vorteilhafterweise die initiale Abbindezeit im Vergleich mit B-V1 durch die Zugabe von NaTS, selbst bei großen Anteilen von NaTS (vgl. bspw. B-E3), nur geringfügig beeinflusst. Dies ist besonders überraschend, da NaTS alleine (siehe B-V2) die Anforderungen an einen Bestandteil eines Initiatorsystems unter den gewählten experimentellen Bedingungen nicht erfüllt und B-V2 durch Mischen mit der zugeordneten Paste A nicht gehärtet werden kann. Erst für sehr hohe Anteile von NaTS (vgl. B-E4) kommt es in erfindungsgemäßen Zusammensetzungen nach sehr langen Lagerzeiten von 12 Wochen zu einer Beeinträchtigung der Leistungsfähigkeit, die zur Folge hat, dass der entsprechende kunststoffmodifizierte Glasionomerzement nicht mehr hinreichend härtet.

Besonders günstige Biegefestigkeiten und Abbindezeiten werden erhalten, wenn das Verhältnis der Gesamtstoffmenge an AS zu der Gesamtstoffmenge an NaTS größer als 1 ist (vgl. Tabelle 6, B-E1 und B-E2) bzw. im Bereich von 1 bis 10 liegt (vgl. B-E2).

Die Ergebnisse für B-E5 und B-E6 zeigen, dass der technische Effekt für sämtliche der erfindungsgemäß einzusetzenden Verbindungen (A) und (S) erzielt werden kann.

Die Vergleichsbeispiele B-V3 und B-V5 zeigen, dass sich der erfindungsgemäß erreichte technische Effekt nicht mit beliebigen Mischungen von üblichen Reduktionsmitteln erreichen lässt. Die Substitution von AS gegen NaDS bzw. OS führt im Vergleich mit (B-V1) nicht zu einer verbesserten Lagerstabilität (vgl. B-V5) bzw. sogar zu einer verschlechterten Lagerstabilität (B-V3).

Entsprechend lassen sich auch durch die Kombination von NaTS mit OS (vgl. B-V4), oder Fe(II) (vgl. B-V6 und B-V7) keine Zusammensetzungen erhalten, die leistungsstark genug sind, die Härtung des kunststoffmodifizierten Glasionomerzements zu bewirken und/oder lagerstabil sind.

Zusammenfassend kann festgestellt werden, dass eine erfindungsgemäße Zusammensetzung zur Verwendung als Komponente eines mehrkomponentigen kunststoffmodifizierten Glasionomerzements sowie die entsprechenden erfindungsgemäßen mehrkomponentigen kunststoffmodifizierten Glasionomerzemente gegenüber aus dem Stand der Technik bekannten Systemen eine höhere Lagerstabilität aufweisen und auch nach mehrwöchiger Lagerung mit nur geringfügig veränderten Abbindezeiten zu Dentalmaterialien gehärtet werden können, die gute mechanische Eigenschaften, insbesondere eine hohe Biegefestigkeit, aufweisen.

Darüber hinaus zeigt sich, dass durch die Verwendung von Verbindungen (S) ausgewählt aus der Gruppe bestehend aus Sulfinsäuren, Salzen der Sulfinsäuren und Salzen bororganischer Verbindungen die Lagerstabilität von Zusammensetzungen umfassend eine oder mehrere Verbindungen (A) ausgewählt aus der Gruppe bestehend aus den Isomeren der Ascorbinsäure, den Salzen der Isomere der Ascorbinsäure, den Estern der Isomere der Ascorbinsäure und den Ethern der Isomere der Ascorbinsäure sowie ein oder mehrere radikalisch polymerisierbare Monomere (M), erhöht werden kann.

## Patentansprüche

1. Zusammensetzung zur Verwendung als Komponente eines mehrkomponentigen kunststoffmodifizierten Glasionomerzements, wobei die Zusammensetzung umfasst
- ein oder mehrere radikalisch polymerisierbare organische Monomere (M) sowie als Bestandteil eines Polymerisationsinitiatorsystems dafür
- eine oder mehrere Verbindungen (A) ausgewählt aus der Gruppe bestehend aus den Isomeren der Ascorbinsäure, den Salzen der Isomere der Ascorbinsäure, den Estern der Isomere der Ascorbinsäure und den Ethern der Isomere der Ascorbinsäure
und
- eine oder mehrere Verbindungen (S) ausgewählt aus der Gruppe bestehend aus Sulfinsäuren, Salzen der Sulfinsäuren und Salzen bororganischer Verbindungen,
zusätzlich umfassend eine basische Glaszusammensetzung (G) als Vernetzer für carbonsäuregruppenhaltige Polymere (P) ausgewählt aus der Gruppe bestehend aus Homo- und Copolymeren einer α,β-ungesättigten Carbonsäure (C).

2. Zusammensetzung nach Anspruch 1,
wobei die basische Glaszusammensetzung (G) ein oder mehrere Kationen von Elementen umfasst, die ausgewählt sind aus der Gruppe bestehend aus den Metallen der Hauptgruppen I, II und III des Periodensystems, vorzugsweise ausgewählt sind aus der Gruppe bestehend aus Natrium, Kalium, Calcium und Aluminium,
und die besonders bevorzugt vorliegen in Form einer oder mehrerer Verbindungen ausgewählt aus der Gruppe bestehend aus Oxiden, Hydroxiden, Sulfaten, Nitraten, Phosphaten, Carbonaten, Silikaten, Fluoriden und Nitriden
und/oder
wobei die basische Glaszusammensetzung (G) ein Aluminiumfluorosilikatglas umfasst
und/oder
wobei die basische Glaszusammensetzung (G) ein oder mehrere Gläser mit organisch modifizierter Oberfläche, vorzugsweise mit silanisierter Oberfläche, umfasst.

3. Zusammensetzung nach einem der vorangehenden Ansprüche,
wobei das eine oder zumindest eines der mehreren radikalisch polymerisierbaren organischen Monomere (M)
- ausgewählt ist aus der Gruppe bestehend aus Monomeren mit mindestens zwei ethylenischen Gruppen (M1),
wobei die Monomere mit mindestens zwei ethylenischen Gruppen (M1) vorzugsweise ausgewählt sind aus der Gruppe bestehend aus Diacrylaten, Triacrylaten, Dimethacrylaten, Trimethacrylaten, besonders vorzugsweise ausgewählt sind aus der Gruppe bestehend aus Ethylenglykoldimethacrylat, 1,6-Hexandioldimethacrylat, Triethylenglykoldimethacrylat, 1,12-Dodecandioldimethacrylat, ethoxyliertes Bisphenol-A-dimethacrylat, Polyethylenglykoldimethacrylat, 7,7,9-Trimethyl-4,13-dioxo-3,14-dioxa-5,12-diazahexadecan-1,16-dioxydimethacrylat, Butandioldimethacrylat, Trimethylolpropantrimethacrylat, Urethandimethacrylat, Glycerin-1,3-dimethacrylat, Tetraethylenglykoldimethacrylat, Neopentylglykoldimethacrylat, 2,2-Bis[4-[3-methacryloyloxy-2-hydroxypropoxy]phenyl]propan und den Di(meth)acrylaten des Dihydroxymethyltricyclo[5.2.1.0^{2,6}]decans
und/oder
wobei das eine oder zumindest eines der mehreren oder zumindest ein weiteres der mehreren radikalisch polymerisierbaren organischen Monomere (M)
- ausgewählt ist aus der Gruppe bestehend aus Hydroxylverbindungen mit mindestens einer ethylenischen Gruppe (M2),
wobei die Hydroxylverbindungen mit mindestens einer ethylenischen Gruppe (M2) vorzugsweise ausgewählt sind aus der Gruppe bestehend aus 2- Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-Bis[4-[3-methacryloyloxy-2-hydroxypropoxy]phenyl]propan
und/oder
wobei die eine oder zumindest eine der mehreren Verbindungen (A) ausgewählt ist aus der Gruppe bestehend aus Ascorbinsäure, Salzen der Ascorbinsäure, Isoascorbinsäure und Salzen der Isoascorbinsäure, vorzugsweise ausgewählt ist aus der Gruppe bestehend aus Ascorbinsäure, Natriumascorbat, Isoascorbinsäure und Natriumisoascorbat
und/oder
wobei die eine oder zumindest eine der mehreren Verbindungen (S) ausgewählt ist aus der Gruppe bestehend aus aromatischen Sulfinsäuren, Salzen der aromatischen Sulfinsäuren und Salzen aromatischer bororganischer Verbindungen, vorzugsweise ausgewählt ist aus der Gruppe bestehend aus Toluolsulfinsäure, Natriumtoluolsulfinat, Kaliumtoluolsulfinat, Benzolsulfinsäure, Natriumbenzolsulfinat, Kaliumbenzolsulfinat, Natriumtetraphenylborat und Lithiumtetraphenylborat, besonders bevorzugt ausgewählt ist aus der Gruppe bestehend aus Toluolsulfinsäure, Natriumtoluolsulfinat, Kaliumtoluolsulfinat, Benzolsulfinsäure, Natriumbenzolsulfinat und Kaliumbenzolsulfinat.

4. Zusammensetzung nach einem der vorangehenden Ansprüche,
wobei das Verhältnis der Gesamtstoffmenge sämtlicher Verbindungen (A) zu der Gesamtstoffmenge sämtlicher Verbindungen (S)
größer als 0,2, bevorzugt größer als 0,3, besonders bevorzugt größer als 1 ist
und/oder
im Bereich von 0,2 bis 50, bevorzugt im Bereich von 0,3 bis 20, besonders bevorzugt im Bereich von 1 bis 10 liegt
und/oder
wobei das Verhältnis der kombinierten Gesamtmasse sämtlicher Verbindungen (A) und sämtlicher Verbindungen (S) zu der Gesamtmasse sämtlicher radikalisch polymerisierbarer organischer Monomere (M) im Bereich von 0,05 bis 10 Gew.-% liegt, vorzugsweise im Bereich von 0,5 bis 7 Gew.-%
und/oder
wobei der Gesamtanteil radikalisch polymerisierbarer organischer Monomere (M) im Bereich von 15 bis 95 Gew.-% liegt, vorzugsweise im Bereich von 25 bis 40 Gew.-%, bezogen auf die Gesamtmasse der Zusammensetzung
und/oder
wobei der kombinierte Gesamtanteil sämtlicher Verbindungen (A) und sämtlicher Verbindungen (S) im Bereich von 0,05 bis 7 Gew.-% liegt, vorzugsweise im Bereich von 0,1 bis 2,5 Gew.-%, bezogen auf die Gesamtmasse der Zusammensetzung
und/oder
wobei die Zusammensetzung
- flüssig oder eine Paste ist
und
- nach einer Lagerung bei 37 °C von 6 Wochen, bevorzugt 8 Wochen, besonders bevorzugt 10 Wochen und ganz besonders bevorzugt 12 Wochen, eine Viskosität besitzt, die kleiner ist als 10000 Pa s, vorzugsweise kleiner ist als 8000 Pa s
und/oder
wobei die Zusammensetzung direkt nach der Herstellung eine Viskosität aufweist, die höchstens um den Faktor 5, bevorzugt höchstens um den Faktor 3, kleiner ist als die Viskosität der entsprechenden Komponente nach 6 Wochen der Lagerung bei 37 °C.

5. Zusammensetzung nach einem der vorangehenden Ansprüche, umfassend
- 0,01 bis 5 Gew.-%, vorzugsweise 0,05 bis 1 Gew.-%, einer oder mehrerer Verbindungen ausgewählt aus der Gruppe bestehend aus Ascorbinsäure, Natriumascorbat, Isoascorbinsäure und Natriumisoascorbat,
- 0,01 bis 5 Gew.-%, vorzugsweise 0,05 bis 1 Gew.-%, einer oder mehrerer Verbindungen ausgewählt aus der Gruppe bestehend aus Toluolsulfinsäure, Natriumtoluolsulfinat, Kaliumtoluolsulfinat, Benzolsulfinsäure, Natriumbenzolsulfinat, Kaliumbenzolsulfinat, Natriumtetraphenylborat und Lithiumtetraphenylborat,
- 2 bis 40 Gew.-%, vorzugsweise 3 bis 30 Gew.-%, einer oder mehrerer Verbindungen ausgewählt aus der Gruppe bestehend aus Ethylenglykoldimethacrylat, 1,6-Hexandioldimethacrylat, Triethylenglykoldimethacrylat, 1,12-Dodecandioldimethacrylat, ethoxyliertes Bisphenol-A-dimethacrylat, Polyethylenglykoldimethacrylat, 7,7,9-Trimethyl-4,13-dioxo-3,14-dioxa-5,12-diazahexadecan-1,16-dioxydimethacrylat, Butandioldimethacrylat, Trimethylolpropantrimethacrylat, Urethandimethacrylat, Glycerin-1,3-dimethacrylat, Tetraethylenglykoldimethacrylat, Neopentylglykoldimethacrylat, 2,2-Bis[4-[3-methacryloyloxy-2-hydroxypropoxy]phenyl]propan und den Di(meth)acrylaten des Dihydroxymethyltricyclo[5.2.1.0^{2,6}]decans
- 3 bis 60 Gew.-%, vorzugsweise 6 bis 40 Gew.-%, einer oder mehrerer Verbindungen ausgewählt aus der Gruppe bestehend aus 2- Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-Bis[4-[3-methacryloyloxy-2-hydroxypropoxy]phenyl]propan und
- 5 bis 85 Gew.-%, vorzugsweise 20 bis 80 Gew.-%, Aluminiumfluorosilikatglas,
vorzugsweise umfassend
- Ascorbinsäure und/oder Natriumisoascorbat in einer Gesamtmenge von 0,1 bis 1 Gew.-%,
- Natriumtoluolsulfinat und/oder Natriumtetraphenylborat in einer Gesamtmenge von 0,01 bis 1 Gew.-%,
- Urethandimethacrylat und/oder Triethylenglycoldimethacrylat und/oder Glycerin-1,3-dimethacrylat in einer Gesamtmenge von 5 bis 20 Gew.-%,
- 2-Hydroxyethylmethacrylat und/oder 2-Hydroxypropylmethacrylat in einer Gesamtmenge von 10 bis 30 Gew.-%,
- 0,01 bis 1 Gew.-% 2,6-Di(tert-butyl)hydroxytoluol und
- 50 bis 75 Gew.-%, Aluminiumfluorosilikatglas,
wobei die Prozentangaben auf die Gesamtmasse der Zusammensetzung bezogen sind.

6. Zusammensetzung nach einem der vorangehenden Ansprüche,
wobei die Zusammensetzung wasserfrei ist
und/oder
wobei die Zusammensetzung kein carbonsäuregruppenhaltiges Polymer (P) ausgewählt aus der Gruppe bestehend aus Homo- und Copolymeren einer α,β-ungesättigten Carbonsäure (C) umfasst
und/oder
wobei die Zusammensetzung kein Oxidationsmittel (O) ausgewählt aus der Gruppe bestehend aus Persulfaten, Permanganaten, Perboraten, Peroxiden und Hydroperoxiden umfasst.

7. Mehrkomponentiger kunststoffmodifizierter Glasionomerzement umfassend
- eine Zusammensetzung nach einem der Ansprüche 1 bis 6 als erste Komponente
und
- in ein oder mehreren weiteren Komponenten zumindest
- ein oder mehrere carbonsäuregruppenhaltige Polymere (P) ausgewählt aus der Gruppe bestehend aus Homo- und Copolymeren einer α,β-ungesättigten Carbonsäure (C),
- Wasser
und
- ein oder mehrere Oxidationsmittel (O) ausgewählt aus der Gruppe bestehend aus Persulfaten, Permanganaten, Perboraten, Peroxiden und Hydroperoxiden,
wobei der mehrkomponentige kunststoffmodifizierte Glasionomerzement
- in der ersten und/oder einer weiteren Komponente eine basische Glaszusammensetzung (G) als Vernetzer für die Polymere (P) umfasst,
mit der Maßgabe, dass die basische Glaszusammensetzung (G) nicht in einer Komponente gemeinsam mit einem carbonsäuregruppenhaltigen Polymer (P) und/oder gemeinsam mit Wasser vorliegt.

8. Mehrkomponentiger kunststoffmodifizierter Glasionomerzement nach Anspruch 7,
wobei die α,β-ungesättigte Carbonsäure (C) ausgewählt ist aus der Gruppe bestehend aus Acrylsäure, Methacrylsäure, Itaconsäure, Fumarsäure, Maleinsäure, Chlormethacrylsäure, Cyanomethacrylsäure, Aconitsäure, Mesaconsäure, Glutaconsäure und Citraconsäure, vorzugsweise ausgewählt ist aus der Gruppe bestehend aus Acrylsäure und Maleinsäure
und/oder
wobei das gewichtsmittlere Molekulargewicht M_{w} der Gesamtmenge der carbonsäuregruppenhaltigen Polymere (P) im Bereich von 1000 bis 150000 g/mol, vorzugsweise zwischen 40000 und 100000 g/mol, liegt
und/oder
wobei die basische Glaszusammensetzung (G) wie in Anspruch 2 definiert ist und/oder
wobei das eine oder zumindest eines der mehreren Oxidationsmittel (O) ausgewählt ist aus der Gruppe bestehend aus Peroxiden und Hydroperoxiden, vorzugsweise ausgewählt ist aus der Gruppe bestehend aus Hydroperoxiden.

9. Verfahren zur Herstellung einer Zusammensetzung nach einem der Ansprüche 1 bis 6 oder eines gehärteten Dentalmaterials, wobei eine Zusammensetzung nach einem der Ansprüche 1 bis 6 als Zwischenprodukt hergestellt wird, umfassend die folgenden Schritte:
| | |
|---|---|
| Schritt (a) | Herstellen oder Bereitstellen eines oder mehrerer radikalisch polymerisierbarer organischer Monomere (M), |
| Schritt (b) | Herstellen oder Bereitstellen einer oder mehrerer Verbindungen (A), wie in Anspruch 1 definiert, |
| Schritt (c) | Herstellen oder Bereitstellen einer oder mehrerer Verbindungen (S), wie in Anspruch 1 definiert, und |
| Schritt (d) | nach den Schritten (a), (b) und (c) Vermischen der Monomere (M) mit den Verbindungen (A) und (S) |
| Schritt (e) | Herstellen oder Bereitstellen einer basischen Glaszusammensetzung (G) wie in Anspruch 1 definiert, |
wobei Schritt (d) das Vermischen der Monomere (M) mit den Verbindungen (A) und (S) sowie mit der in Schritt (e) hergestellten oder bereitgestellten Glaszusammensetzung (G) umfasst.

10. Verfahren nach Anspruch 9,
wobei das eine oder zumindest eines der mehreren radikalisch polymerisierbaren organischen Monomere (M) ausgewählt ist aus den in Anspruch 3 definierten Gruppen
und/oder
wobei die eine oder zumindest eine der mehreren Verbindungen (A) ausgewählt ist aus der in Anspruch 3 definierten Gruppe
und/oder
wobei die eine oder zumindest eine der mehreren Verbindungen (S) ausgewählt ist aus der in Anspruch 3 definierten Gruppe
und/oder
wobei das Verhältnis der Gesamtstoffmenge sämtlicher Verbindungen (A) zu der Gesamtstoffmenge sämtlicher Verbindungen (S) wie in Anspruch 4 definiert ist und/oder
wobei das Verhältnis der kombinierten Gesamtmasse sämtlicher Verbindungen (A) und sämtlicher Verbindungen (S) zu der Gesamtmasse sämtlicher radikalisch polymerisierbarer organischer Monomere (M) wie in Anspruch 4 definiert ist und/oder
wobei der Gesamtanteil radikalisch polymerisierbarer organischer Monomere (M) im Bereich von 15 bis 95 Gew.-% liegt, vorzugsweise im Bereich von 25 bis 40 Gew.-%, bezogen auf die Gesamtmasse der hergestellten Zusammensetzung und/oder
wobei der kombinierte Gesamtanteil sämtlicher Verbindungen (A) und sämtlicher Verbindungen (S) im Bereich von 0,05 bis 7 Gew.-% liegt, vorzugsweise im Bereich von 0,1 bis 2,5 Gew.-%, bezogen auf die Gesamtmasse der hergestellten Zusammensetzung.

11. Verfahren nach einem der Ansprüche 9 oder 10, wobei die basische Glaszusammensetzung (G) wie in Anspruch 2 definiert ist.

12. Verfahren nach einem der Ansprüche 9 bis 11 zur Herstellung eines gehärteten Dentalmaterials, zusätzlich umfassend den Schritt:
| | |
|---|---|
| Schritt (g) | Härten einer die hergestellte Zusammensetzung umfassenden Mischung, wobei das Härten vorzugsweise umfasst: |
| | das Umsetzen der in der Mischung enthaltenen Verbindungen (A) und (S) mit einem oder mehreren Oxidationsmitteln (O) ausgewählt aus der Gruppe bestehend aus Persulfaten, Permanganaten, Perboraten, Peroxiden und Hydroperoxiden |
| | und/oder |
| | das Bestrahlen der Mischung mit Licht einer Wellenlänge im Bereich von 300 bis 700 nm, vorzugsweise im Bereich von 350 bis 600 nm |
| | und/oder |
| | das Umsetzen von in der Mischung enthaltenen carbonsäuregruppenhaltigen Polymeren (P) wie in Anspruch 7 definiert mit einer in der Mischung enthaltenen basischen Glaszusammensetzung (G), wie in Anspruch 1 definiert, |
| | wobei die α,β-ungesättigte Carbonsäure (C) vorzugsweise wie in Anspruch 8 definiert ist |
| | und/oder |
| | wobei das gewichtsmittlere Molekulargewicht M_{w} der Gesamtmenge der carbonsäuregruppenhaltigen Polymere (P) vorzugsweise wie in Anspruch 8 definiert ist. |

13. Verwendung einer oder mehrerer Verbindungen (S) wie in Anspruch 1 definiert oder von Mischungen umfassend eine oder mehrere Verbindungen (S) wie in Anspruch 1 definiert, zur
(i) Erhöhung der Lagerstabilität einer Zusammensetzung umfassend
- eine oder mehrere Verbindungen (A) wie in Anspruch 1 definiert,
- ein oder mehrere radikalisch polymerisierbare organische Monomere (M), und
- eine basische Glaszusammensetzung (G) wie in Anspruch 1 definiert und/oder
(ii) Herstellung einer Zusammensetzung gemäß einem der Ansprüche 1 bis 6.

14. Verwendung nach Anspruch 13,
wobei die eine oder zumindest eine der mehreren Verbindungen (S) ausgewählt ist aus der in Anspruch 3 definierten Gruppe
und/oder
wobei die eine oder zumindest eine der mehreren Verbindungen (A) ausgewählt ist aus der in Anspruch 3 definierten Gruppe
und/oder
wobei das eine oder zumindest eines der mehreren radikalisch polymerisierbaren organischen Monomere (M) ausgewählt ist aus den in Anspruch 3 definierten Gruppen
und/oder
wobei das Verhältnis der Gesamtstoffmenge sämtlicher Verbindungen (A) zu der Gesamtstoffmenge sämtlicher Verbindungen (S) wie in Anspruch 4 definiert ist
und/oder
wobei das Verhältnis der kombinierten Gesamtmasse sämtlicher Verbindungen (A) und sämtlicher Verbindungen (S) zu der Gesamtmasse sämtlicher radikalisch polymerisierbarer organischer Monomere (M) wie in Anspruch 4 definiert ist
und/oder
wobei der Gesamtanteil radikalisch polymerisierbarer organischer Monomere (M) in der Zusammensetzung wie in Anspruch 4 definiert ist
und/oder
wobei der kombinierte Gesamtanteil sämtlicher Verbindungen (A) und sämtlicher Verbindungen (S) in der Zusammensetzung wie in Anspruch 4 definiert ist.

## Claims

1. A composition for use as component of a multicomponent resin-modified glass ionomer cement, the composition comprising
- one or more radically polymerizable organic monomers (M)
and also, as constituent of a polymerization initiator system for it
- one or more compounds (A) selected from the group consisting of the isomers of ascorbic acid, the salts of the isomers of ascorbic acid, the esters of the isomers of ascorbic acid and the ethers of the isomers of ascorbic acid
and
- one or more compounds (S) selected from the group consisting of sulfinic acids, salts of sulfinic acids and salts of organoboron compounds
additionally comprising a basic glass composition (G) as crosslinking agent for polymers containing carboxylic acid groups (P) selected from the group consisting of homo- and copolymers of an α,β-unsaturated carboxylic acid (C).

2. The composition as claimed in claim 1,
wherein the basic glass composition (G) comprises one or more cations of elements which are selected from the group consisting of the metals of Main Groups I, II and III of the Periodic Table, are preferably selected from the group consisting of sodium, potassium, calcium and aluminum,
and which are particularly preferably present in the form of one or more compounds selected from the group consisting of oxides, hydroxides, sulfates, nitrates, phosphates, carbonates, silicates, fluorides and nitrides
and/or
wherein the basic glass composition (G) comprises an aluminum fluorosilicate glass
and/or
wherein the basic glass composition (G) comprises one or more glasses with organically modified surface, preferably with silanized surface.

3. The composition as claimed in one of the preceding claims,
wherein the one or at least one of the several radically polymerizable organic monomers (M)
- is selected from the group consisting of monomers with at least two ethylenic groups (M1),
wherein the monomers with at least two ethylenic groups (M1) are preferably selected from the group consisting of diacrylates, triacrylates, dimethacrylates, trimethacrylates, are particularly preferably selected from the group consisting of ethylene glycol dimethacrylate, 1,6-hexanediol dimethacrylate, triethylene glycol dimethacrylate, 1,12-dodecanediol dimethacrylate, ethoxylated bisphenol A dimethacrylate, polyethylene glycol dimethacrylate, 7,7,9-trimethyl-4,13-dioxo-3,14-dioxa-5,12-diazahexadecane-1,16-diol dimethacrylate, butanediol dimethacrylate, trimethylolpropane trimethacrylate, urethane dimethacrylate, glycerol 1,3-dimethacrylate, tetraethylene glycol dimethacrylate, neopentyl glycol dimethacrylate, 2,2-bis[4-[3-methacryloyloxy-2-hydroxypropoxy]phenyl]propane and the di(meth)acrylates of dihydroxymethyltricyclo[5.2.1.0^{2,6}]decane
and/or
wherein the one or at least one of the several or at least one additional of the several radically polymerizable organic monomers (M)
- is selected from the group consisting of hydroxyl compounds with at least one ethylenic group (M2),
wherein the hydroxyl compounds with at least one ethylenic group (M2) are preferably selected from the group consisting of 2-hydroxyethyl methacrylate, 2-hydroxypropyl methacrylate, 3-hydroxypropyl methacrylate, 1,2-dihydroxypropyl methacrylate, 1,3-dihydroxypropyl methacrylate, 2,3-dihydroxypropyl methacrylate, 2-hydroxypropyl 1,3-dimethacrylate, 3-hydroxypropyl 1,2-dimethacrylate, pentaerythritol dimethacrylate, glycerol dimethacrylate, 2,2-bis[4-[3-methacryloyloxy-2-hydroxypropoxy]phenyl]propane
and/or
wherein the one or at least one of the several compounds (A) is selected from the group consisting of ascorbic acid, salts of ascorbic acid, isoascorbic acid and salts of isoascorbic acid, is preferably selected from the group consisting of ascorbic acid, sodium ascorbate, isoascorbic acid and sodium isoascorbate
and/or
wherein the one or at least one of the several compounds (S) is selected from the group consisting of aromatic sulfinic acids, salts of aromatic sulfinic acids and salts of aromatic organoboron compounds, is preferably selected from the group consisting of toluenesulfinic acid, sodium toluenesulfinate, potassium toluenesulfinate, benzenesulfinic acid, sodium benzenesulfinate, potassium benzenesulfinate, sodium tetraphenylborate and lithium tetraphenylborate, is particularly preferably selected from the group consisting of toluenesulfinic acid, sodium toluenesulfinate, potassium toluenesulfinate, benzenesulfinic acid, sodium benzenesulfinate and potassium benzenesulfinate.

4. The composition as claimed in one of the preceding claims,
wherein the ratio of the total molar amount of all of the compounds (A) to the total molar amount of all of the compounds (S)
is greater than 0.2, preferably greater than 0.3, particularly preferably greater than 1
and/or
is in the range from 0.2 to 50, preferably in the range from 0.3 to 20, particularly preferably in the range from 1 to 10
and/or
wherein the ratio of the combined total weight of all of the compounds (A) and all of the compounds (S) to the total weight of all of the radically polymerizable organic monomers (M) lies in the range from 0.05 to 10% by weight, preferably in the range from 0.5 to 7% by weight
and/or
wherein the total content of radically polymerizable organic monomers (M) lies in the range from 15 to 95% by weight, preferably in the range from 25 to 40% by weight, based on the total weight of the composition
and/or
wherein the combined total content of all of the compounds (A) and all of the compounds (S) lies in the range from 0.05 to 7% by weight, preferably in the range from 0.1 to 2.5% by weight, based on the total weight of the composition
and/or
wherein the composition
- is liquid or a paste
and
- after storing at 37°C for 6 weeks, preferably 8 weeks, particularly preferably 10 weeks and very particularly preferably 12 weeks, has a viscosity which is smaller than 10 000 Pa.s, is preferably smaller than 8000 Pa.s
and/or
wherein the composition exhibits, directly after the manufacturing, a viscosity which is smaller at the most by the factor 5, preferably at the most by the factor 3, than the viscosity of the corresponding component after storing at 37°C for 6 weeks.

5. The composition as claimed in one of the preceding claims, comprising
- 0.01 to 5% by weight, preferably 0.05 to 1% by weight, of one or more compounds selected from the group consisting of ascorbic acid, sodium ascorbate, isoascorbic acid and sodium isoascorbate,
- 0.01 to 5% by weight, preferably 0.05 to 1% by weight, of one or more compounds selected from the group consisting of toluenesulfinic acid, sodium toluenesulfinate, potassium toluenesulfinate, benzenesulfinic acid, sodium benzensulfinate, potassium benzenesulfinate, sodium tetraphenylborate and lithium tetraphenylborate,
- 2 to 40% by weight, preferably 3 to 30% by weight, of one or more compounds selected from the group consisting of ethylene glycol dimethacrylate, 1,6-hexanediol dimethacrylate, triethylene glycol dimethacrylate, 1,12-dodecanediol dimethacrylate, ethoxylated bisphenol A dimethacrylate, polyethylene glycol dimethacrylate, 7,7,9-trimethyl-4,13-dioxo-3,14-dioxa-5,12-diazahexadecane-1,16-diol dimethacrylate, butanediol dimethacrylate, trimethylolpropane trimethacrylate, urethane dimethacrylate, glycerol 1,3-dimethacrylate, tetraethylene glycol dimethacrylate, neopentyl glycol dimethacrylate, 2,2-bis[4-[3-methacryloyloxy-2-hydroxypropoxy]phenyl]propane and the di(meth)acrylates of dihydroxymethyltricyclo[5.2.1.0^{2,6}]decane
- 3 to 60% by weight, preferably 6 to 40% by weight, of one or more compounds selected from the group consisting of 2-hydroxyethyl methacrylate, 2-hydroxypropyl methacrylate, 3-hydroxypropyl methacrylate, 1,2-dihydroxypropyl methacrylate, 1,3-dihydroxypropyl methacrylate, 2,3-dihydroxypropyl methacrylate, 2-hydroxypropyl 1,3-dimethacrylate, 3-hydroxypropyl 1,2-dimethacrylate, pentaerythritol dimethacrylate, glycerol dimethacrylate, 2,2-bis[4-[3-methacryloyloxy-2-hydroxypropoxy]phenyl]propane and
- 5 to 85% by weight, preferably 20 to 80% by weight, of aluminum fluorosilicate glass,
preferably comprising
- ascorbic acid and/or sodium isoascorbate in a total amount of 0.1 to 1% by weight,
- sodium toluenesulfinate and/or sodium tetraphenylborate in a total amount of 0.01 to 1% by weight,
- urethane dimethacrylate and/or triethylene glycol dimethacrylate and/or glycerol 1,3-dimethacrylate in a total amount of 5 to 20% by weight,
- 2-hydroxyethyl methacrylate and/or 2-hydroxypropyl methacrylate in a total amount of 10 to 30% by weight,
- 0.01 to 1% by weight of 2,6-di(tert-butyl)hydroxytoluene and
- 50 to 75% by weight of aluminum fluorosilicate glass,
wherein the percentage values are based on the total weight of the composition.

6. The composition as claimed in one of the preceding claims,
the composition being anhydrous
and/or
the composition not comprising any polymer containing carboxylic acid groups (P) selected from the group consisting of homo- and copolymers of an α,β-unsaturated carboxylic acid (C)
and/or
the composition not comprising any oxidizing agent (0) selected from the group consisting of persulfates, permanganates, perborates, peroxides and hydroperoxides.

7. A multicomponent resin-modified glass ionomer cement comprising
- a composition as claimed in one of claims 1 to 6 as first component
and
- in one or more additional components, at least
- one or more polymers containing carboxylic acid groups (P) selected from the group consisting of homo- and copolymers of an α,β-unsaturated carboxylic acid (C),
- water
and
- one or more oxidizing agents (0) selected from the group consisting of persulfates, permanganates, perborates, peroxides and hydroperoxides,
the multicomponent resin-modified glass ionomer cement
- comprising, in the first and/or an additional component, a basic glass composition (G) as crosslinking agent for the polymers (P),
with the proviso that the basic glass composition (G) is not present in a component together with a polymer containing carboxylic acid groups (P) and/or together with water.

8. The multicomponent resin-modified glass ionomer cement as claimed in claim 7,
wherein the α,β-unsaturated carboxylic acid (C) is selected from the group consisting of acrylic acid, methacrylic acid, itaconic acid, fumaric acid, maleic acid, chloromethacrylic acid, cyanomethacrylic acid, aconitic acid, mesaconic acid, glutaconic acid and citraconic acid, is preferably selected from the group consisting of acrylic acid and maleic acid
and/or
wherein the weight-average molecular weight M_{w} of the total amount of the polymers containing carboxylic acid groups (P) lies in the range from 1000 to 150 000 g/mol, preferably between 40 000 and 100 000 g/mol,
and/or
wherein the basic glass composition (G) is defined as in claim 2 and/or
wherein the one or at least one of the several oxidizing agents (O) is selected from the group consisting of peroxides and hydroperoxides, is preferably selected from the group consisting of hydroperoxides.

9. A process for the manufacturing of a composition as claimed in one of claims 1 to 6 or for the preparation of a cured dental material, wherein a composition as claimed in one of claims 1 to 6 is manufactured as intermediate, comprising the following stages:
| | |
|---|---|
| stage (a) | synthesis or provision of one or more radically polymerizable organic monomers (M), |
| stage (b) | synthesis or provision of one or more compounds (A), as defined in claim 1, |
| stage (c) | synthesis or provision of one or more compounds (S), as defined in claim 1, and |
| stage (d) | after stages (a), (b) and (c), mixing of the monomers (M) with the compounds (A) and (S) |
| stage (e) | production or provision of a basic glass composition (G) as defined in claim 1, |
| | wherein stage (d) comprises the mixing of the monomers (M) with the compounds (A) and (S) and also with the glass composition (G) produced or provided in stage (e). |

10. The process as claimed in claim 9,
wherein the one or at least one of the several radically polymerizable organic monomers (M) is selected from the groups defined in claim 3
and/or
wherein the one or at least one of the several compounds (A) is selected from the group defined in claim 3
and/or
wherein the one or at least one of the several compounds (S) is selected from the group defined in claim 3
and/or
wherein the ratio of the total molar amount of all of the compounds (A) to the total molar amount of all of the compounds (S) is as defined in claim 4
and/or
wherein the ratio of the combined total weight of all of the compounds (A) and of all of the compounds (S) to the total weight of all of the radically polymerizable organic monomers (M) is as defined in claim 4
and/or
wherein the total content of radically polymerizable organic monomers (M) lies in the range from 15 to 95% by weight, preferably in the range from 25 to 40% by weight, based on the total weight of the composition manufactured
and/or
wherein the combined total content of all of the compounds (A) and of all of the compounds (S) lies in the range from 0.05 to 7% by weight, preferably in the range from 0.1 to 2.5% by weight, based on the total weight of the composition manufactured.

11. The process as claimed in either of claims 9 and 10
wherein the basic glass composition (G) is as defined in claim 2.

12. The process as claimed in one of claims 9 to 11 for the preparation of a cured dental material, additionally comprising the stage:
| | |
|---|---|
| stage (g) | curing of the mixture comprising the composition prepared, wherein the curing preferably comprises: |
| | the reaction of the compounds (A) and (S) present in the mixture with one or more oxidizing agents (O) selected from the group consisting of persulfates, permanganates, perborates, peroxides and hydroperoxides |
| | and/or |
| | the irradiation of the mixture with light having a wavelength in the range from 300 to 700 nm, preferably in the range from 350 to 600 nm |
| | and/or |
| | the reaction of polymers containing carboxylic acid groups (P) as defined in claim 7 present in the mixture with a basic glass composition (G) as defined in claim 1 present in the mixture, |
| | wherein the α,β-unsaturated carboxylic acid (C) is preferably as defined in claim 8 |
| | and/or |
| | wherein the weight-average molecular weight M_{w} of the total amount of the polymers containing carboxylic acid groups (P) is preferably as defined in claim 8. |

13. The use of one or more compounds (S) as defined in claim 1 or of mixtures comprising one or more compounds (S) as defined in claim 1, for
(i) increasing the storage stability of a composition comprising
- one or more compounds (A) as defined in claim 1 and
- one or more radically polymerizable organic monomers (M) and
- a basic glass composition (G) as defined in claim 1
and/or
(ii) manufacturing a composition as claimed in one of claims 1 to 6.

14. The use as claimed in claim 13,
wherein the one or at least one of the several compounds (S) is selected from the group defined in claim 3
and/or
wherein the one or at least one of the several compounds (A) is selected from the group defined in claim 3
and/or
wherein the one or at least one of the several radically polymerizable organic monomers (M) is selected from the groups defined in claim 3
and/or
wherein the ratio of the total molar amount of all of the compounds (A) to the total molar amount of all of the compounds (S) is as defined in claim 4
and/or
wherein the ratio of the combined total weight of all of the compounds (A) and of all of the compounds (S) to the total weight of all of the radically polymerizable organic monomers (M) is as defined in claim 4
and/or
wherein the total content of radically polymerizable organic monomers (M) in the composition is as defined in claim 4
and/or
wherein the combined total content of all of the compounds (A) and of all of the compounds (S) in the composition is as defined in claim 4.

## Revendications

1. Composition destinée à être utilisée en tant que composant d'un ciment au verre ionomère à plusieurs composants modifié par une matière synthétique, dans laquelle la composition comprend
- un ou plusieurs monomères (M) organiques radicalement polymérisables,
ainsi qu'en tant que constituant d'un système initiateur de polymérisation associé
- un ou plusieurs composés (A) choisis parmi le groupe constitué des isomères de l'acide ascorbique, des sels des isomères de l'acide ascorbique, des esters des isomères de l'acide ascorbique et des éthers des isomères de l'acide ascorbique
et
- un ou plusieurs composés (S) choisis parmi le groupe constitué d'acides sulfiniques, de sels des acides sulfiniques et des sels de composés organiques de bore,
comprenant en supplément une composition de verre basique (G) en tant qu'agent de réticulation pour des polymères (P) contenant des groupes acides carboxyliques choisis parmi le groupe constitué d'homo-et de copolymères d'un acide carboxylique (C) α,β-insaturé.

2. Composition selon la revendication 1,
dans laquelle la composition de verre basique (G) comprend un ou plusieurs cations d'éléments, qui sont choisis parmi le groupe constitué des métaux des groupes principaux I, II et III du tableau périodique, de préférence qui sont choisis parmi le groupe constitué du sodium, du potassium, du calcium et de l'aluminium,
et qui sont présents de manière particulièrement préférée sous la forme d'un ou de plusieurs composés choisis parmi le groupe constitué d'oxydes, d'hydroxydes, de sulfates, de nitrates, de phosphates, de carbonates, de silicates, de fluorures et de nitrures
et/ou
dans laquelle la composition de verre basique (G) comprend un verre de fluorosilicate d'aluminium
et/ou
dans laquelle la composition de verre basique (G) comprend un ou plusieurs verres avec une surface organiquement modifiée, de préférence avec une surface silanisée.

3. Composition selon l'une quelconque des revendications précédentes,
dans laquelle le ou au moins un des plusieurs monomères (M) organiques radicalement polymérisables
- est choisi parmi le groupe constitué de monomères avec au moins deux groupes éthyléniques (M1), dans laquelle les monomères avec au moins deux groupes éthyléniques (M1) sont choisis de préférence parmi le groupe constitué de diacrylates, de triacrylates, de diméthacrylates, de triméthacrylates, en particulier sont choisis de préférence parmi le groupe constitué de diméthacrylate de glycol éthylénique, de 1,6-diméthacrylate d'hexanediol, de diméthacrylate de glycol triéthylénique, de 1,12-diméthacrylate de dodécandiol, de diméthacrylate de bisphénol A éthoxylé, de diméthacrylate de polyéthylèneglycol, de 7,7,9-triméthyl-4,13-dioxo-3,14-dioxa-5,12-diazahexadecan-1,16-dioxydiméthacrylate, de diméthacrylate de butanediol, de triméthacrylate de triméthylolpropane, de diméthacrylate d'uréthane, de glycérine-1,3-diméthacrylate, de diméthacrylate de tétraéthylèneglycol, de diméthacrylate de néopentylglycol, de 2,2-bis[4-[3-méthacryloyloxy-2-hydroxypropoxy]phényl]propane et des di(méth)acrylates du dihydroxyméthyltricyclo[5.2.1.0^{2,6}]décane
et/ou
dans laquelle le ou au moins un des plusieurs ou au moins un autre des plusieurs monomères (M) organiques radicalement polymérisables
- est choisi parmi le groupe constitué de composés hydroxyles avec au moins un groupe éthylénique (M2),
dans laquelle les composés hydroxyles avec au moins un groupe éthylénique (M2) sont choisis de préférence parmi le groupe constitué de 2-hydroxyéthylméthacrylate, de 2-hydroxypropylméthacrylate, de 3-hydroxypropylméthacrylate, de 1,2-dihydroxypropylméthacrylate, de 1,3-dihydroxypropylméthacrylate, de 2,3-dihydroxypropylméthacrylate, de 2-hydroxypropyl-1,3-diméthacrylate, de 3-hydroxypropyl-1,2-diméthacrylate, de diméthacrylate de pentaérythritol, de diméthacrylate de glycérine, de 2,2-bis[4-[3-méthacryloyloxy-2-hydroxypropoxy]phényl]propane
et/ou
dans laquelle le ou au moins un des plusieurs composés (A) est choisi parmi le groupe constitué de l'acide ascorbique, de sels de l'acide ascorbique, de l'acide isoascorbique et des sels de l'acide isoascorbique, de préférence est choisi parmi le groupe constitué de l'acide ascorbique, de l'ascorbate de sodium, de l'acide isoascorbique et de l'isoascorbate de sodium
et/ou
dans laquelle le ou au moins un des plusieurs composés (S) est choisi parmi le groupe constitué d'acides sulfiniques aromatiques, de sels des acides sulfiniques aromatiques et des sels de composés aromatiques organiques de bore, de préférence est choisi parmi le groupe constitué de l'acide toluènesulfinique, de toluènesulfinate de sodium, de toluènesulfinate de potassium, de l'acide sulfinique de benzène, de sulfinate de benzène de sodium, de sulfinate de benzène de potassium, de tétraphénylborate de sodium et de tétraphénylborate de lithium, est choisi de manière particulièrement préférée parmi le groupe constitué de l'acide toluènesulfinique, de toluènesulfinate de sodium, de toluènesulfinate de potassium, de l'acide sulfinique de benzène, de sulfinate de benzène de sodium, de sulfinate de benzène de potassium.

4. Composition selon l'une quelconque des revendications précédentes,
dans laquelle le rapport entre la quantité de matière totale de tous les composés (A) et la quantité de matière totale de tous les composés (S)
est supérieur à 0,2, de manière préférée supérieur à 0,3, de manière particulièrement préférée supérieur à 1
et/ou
se situe dans la plage de 0,2 à 50, de manière préférée dans la plage de 0,3 à 20, de manière particulièrement préférée dans la plage de 1 à 10
et/ou
dans laquelle le rapport entre le poids total combiné de tous les composés (A) et de tous les composés (S) et le poids total de tous les monomères (M) organiques radicalement polymérisables se situe dans la plage de 0,05 à 10 % en poids, de préférence dans la plage de 0,5 à 7 % en poids,
et/ou
dans laquelle la fraction totale de monomères (M) organiques radicalement polymérisables se situe dans la plage de 15 à 95 % en poids, de préférence dans la plage de 25 à 40 % en poids, par rapport au poids total de la composition
et/ou
dans laquelle la fraction totale combinée de tous les composés (A) et de tous les composés (S) se situe dans la plage de 0,05 à 7 % en poids, de préférence dans la plage de 0,1 à 2,5 % en poids, par rapport au poids total de la composition,
et/ou
dans laquelle la composition
- est liquide ou une pâte
et
- possède, après un stockage à 37 °C de 6 semaines de manière préférée de 8 semaines, de manière particulièrement préférée de 10 semaines et de manière tout particulièrement préférée de 12 semaines, une viscosité, qui est inférieure à 10 000 Pa s, de préférence inférieure à 8000 Pa s
et/ou
dans laquelle la composition présente directement après la fabrication une viscosité, qui est inférieure au maximum d'un facteur 5, de manière préférée au maximum d'un facteur 3, à la viscosité du composant correspondant après 6 semaines de stockage à 37 °C.

5. Composition selon l'une quelconque des revendications précédentes, comprenant
- 0,01 à 5 % en poids, de préférence de 0,05 à 1 % en poids, d'un ou de plusieurs composés choisis parmi le groupe constitué de l'acide ascorbinique, d'ascorbate de sodium, de l'acide isoascorbinique et d'isoascorbate de sodium,
- 0,01 à 5 % en poids, de préférence 0,05 à 1 % en poids d'un ou de plusieurs composés choisis parmi le groupe constitué de l'acide sulfinique de toluène, de toluènesulfinate de sodium, de toluènesulfinate de potassium, de l'acide sulfinique de benzène, de sulfinate de benzène de sodium, de sulfinate de benzène de potassium, de tétraphénylborate de sodium et de tétraphénylborate de lithium,
- 2 à 40 % en poids, de préférence 3 à 30 % en poids, d'un ou de plusieurs composés choisis parmi le groupe constitué de diméthacrylate d'éthylèneglycol, de 1,6-hexandioldiméthacrylate, de diméthacrylate de triéthylèneglycol, de 1,12-dodécandioldiméthacrylate, de diméthacrylate de bisphénol A éthoxylé, de diméthacrylate de polyéthylèneglycol, de 7,7,9-triméthyl-4,13-dioxo-3,14-dioxa-5,12-diazahexadecane-1,16-dioxydiméthacrylate, de butandioldiméthacrylate, de triméthylolpropanetriméthacrylate, de diméthacrylate d'uréthane, de glycérine-1,3-diméthacrylate, de diméthacrylate de tétraéthylèneglycol, de diméthacrylate de néopentylglycol, de 2,2-bis[4-[3-méthacryloyloxy-2-hydroxypropoxy]phényl]propane et des di(méth)acrylates de dihydroxyméthyltricyclo[5.2.1.0^{2,6}]décane,
- 3 à 60 % en poids, de préférence 6 à 40 % en poids, d'un ou de plusieurs composés choisis parmi le groupe constitué de 2-hydroxyéthylméthacrylate, de 2-hydroxypropylméthacrylate, de 3-hydroxypropylméthacrylate, de 1,2-dihydroxypropylméthacrylate, de 1,3-dihydroxypropylméthacrylate, de 2,3-dihydroxypropylméthacarylate, de 2-hydroxypropyl-1,3-diméthacrylate, de 3-hydroxypropyl-1,2-diméthacrylate, de diméthacrylate de pentaérythritol, de diméthacrylate de glycérine, de 2,2-bis[4-[3-méthacryloyloxy-2-hydroxypropoxy]phényle]propane
et
- 5 à 85 % en poids, de préférence 20 à 80 % en poids, de verre de fluorosilicate d'aluminium,
de préférence comprenant
- de l'acide ascorbinique et/ou de l'isoascorbate de sodium en une quantité totale de 0,1 à 1 % en poids,
- du toluènesulfinate de sodium et/ou du tétraphénylborate de sodium en une quantité totale de 0,01 à 1 % en poids,
- du diméthacrylate d'uréthane et/ou du diméthacrylate de triéthylèneglycol et/ou du 1,3-diméthacrylate de glycérine en une quantité totale de 5 à 20 % en poids,
- du 2-hydroxyéthylméthacrylate et/ou du 2-hydroxypropylméthacrylate en une quantité totale de 10 à 30 % en poids,
- 0,01 à 1 % en poids de 2,6-di(tert-butyle)hydroxytoluène,
et
- 50 à 75 % en poids de verre de fluorosilicate d'aluminium,
dans laquelle les indications en pourcentages se rapportent au poids total de la composition.

6. Composition selon l'une quelconque des revendications précédentes,
dans laquelle la composition est exempte d'eau et/ou
dans laquelle la composition ne comprend aucun polymère (P) contenant des groupes acides carboxyliques choisi parmi le groupe constitué d'homo- et de copolymères d'un acide carboxylique (C) α,β-insaturé
et/ou
dans laquelle la composition ne comprend aucun agent d'oxydation (O) choisi parmi le groupe constitué de persulfates, de permanganates, de perborates, de peroxydes et d'hydroperoxydes.

7. Ciment au verre ionomère à plusieurs composants modifié par matière synthétique, comprenant
- une composition selon l'une quelconque des revendications 1 à 6 en tant que premier composant
et
- dans un ou plusieurs autres composants au moins
-- un ou plusieurs polymères (P) contenant des groupes acides carboxyliques choisis parmi le groupe constitué d'homo- et de copolymères d'un acide carboxylique (C) α,β-insaturé,
-- de l'eau
et
-- un ou plusieurs agents d'oxydation (O) choisis parmi le groupe constitué de persulfates, de permanganates, de perborates, de peroxydes et d'hydroperoxydes,
dans laquelle le ciment au verre ionomère à plusieurs composants modifié par matière synthétique
- comprend dans le premier et/ou un autre composant une composition de verre basique (G) en tant qu'agent de réticulation pour les polymères (P),
à condition que la composition de verre basique (G) ne soit pas présente dans un composant conjointement avec un polymère (P) contenant des groupes acides carboxyliques et/ou conjointement avec de l'eau.

8. Ciment au verre ionomère à plusieurs composants modifié par matière synthétique selon la revendication 7,
dans lequel l'acide carboxylique (C) α,β-insaturé est choisi parmi le groupe constitué de l'acide acrylique, de l'acide méthacrylique, de l'acide itaconique, de l'acide fumarique, de l'acide maléique, de l'acide chlorométhacrylique, de l'acide cyanométhacrylique, de l'acide aconitique, de l'acide mésaconique, de l'acide glutaconique, et de l'acide citraconique, de préférence est choisi parmi le groupe constitué de l'acide acrylique et de l'acide maléique,
et/ou
dans lequel le poids moléculaire pondéré M_{w} de la quantité totale des polymères (P) contenant des groupes acides carboxyliques se situe dans la plage de 1000 à 150 000 g/mol, de préférence entre 40 000 et 100 000 g/mol
et/ou
dans lequel la composition de verre basique (G) est définie telle que dans la revendication 2
et/ou
dans lequel le ou au moins un des plusieurs agents d'oxydation (O) est choisi parmi le groupe constitué de peroxydes et d'hydroperoxydes, de préférence est choisi parmi le groupe constitué d'hydroperoxydes.

9. Procédé servant à fabriquer une composition selon l'une quelconque des revendications 1 à 6 ou un matériau dentaire durci, dans lequel une composition selon l'une quelconque des revendications 1 à 6 est fabriquée en tant que produit intermédiaire, comprenant les étapes suivantes :
étape (a) : fabrication ou mise à disposition d'un ou de plusieurs monomères (M) organiques radicalement polymérisables,
étape (b) : fabrication ou mise à disposition d'un ou de plusieurs composés (A), tels que définis dans la revendication 1,
étape (c) : fabrication ou mise à disposition d'un ou de plusieurs composés (S), tels que définis dans la revendication 1, et
étape (d) : après les étapes (a), (b) et (c), mélange des monomères (M) et des composés (A) et (S),
étape (e) : fabrication ou préparation d'une composition de verre basique (G) telle que définie dans la revendication 1,
dans lequel l'étape (d) comprend le mélange des monomères (M) et des composés (A) et (S) ainsi que de la composition de verre (G) fabriquée ou mise à disposition à l'étape (e).

10. Procédé selon la revendication 9,
dans lequel le ou au moins un des plusieurs monomères (M) organiques radicalement polymérisables est choisi parmi les groupes définis dans la revendication 3
et/ou
dans lequel le ou au moins un des plusieurs composés (A) est choisi parmi le groupe défini dans la revendication 3
et/ou
dans lequel le ou au moins un des plusieurs composés (S) est choisi parmi le groupe défini dans la revendication 3
et/ou
dans lequel le rapport entre la quantité de matière totale de tous les composés (A) et la quantité de matière totale de tous les composés (S) est tel que défini dans la revendication 4
et/ou
dans lequel le rapport entre le poids total combiné de tous les composés (A) et de tous les composés (S) et le poids total de tous les monomères (M) organiques radicalement polymérisables est tel que défini dans la revendication 4
et/ou
dans lequel la fraction totale de monomères (M) organiques radicalement polymérisables se situe dans la plage de 15 à 95 % en poids, de préférence dans la plage de 25 à 40 % en poids, par rapport au poids total de la composition fabriquée
et/ou
dans lequel la fraction totale combinée de tous les composés (A) et de tous les composés (S) se situe dans la plage de 0,05 à 7 % en poids, de préférence dans la plage de 0,1 à 2,5 % en poids, par rapport au poids total de la composition fabriquée.

11. Procédé selon l'une quelconque des revendications 9 ou 10, dans lequel la composition de verre basique (G) est telle que définie dans la revendication 2.

12. Procédé selon l'une quelconque des revendications 9 à 11 servant à fabriquer un matériau dentaire durci, comprenant en supplément l'étape suivante :
étage (g) : durcissement d'un mélange comprenant la composition fabriquée, dans lequel le durcissement comprend de préférence :
la mise en réaction des composés (A) et (S) contenus dans le mélange avec un ou plusieurs agents d'oxydation (O) choisis parmi le groupe constitué de persulfates, de permanganates, de perborates, de peroxydes et d'hydroperoxydes
et/ou
l'irradiation du mélange avec de la lumière d'une longueur d'onde dans la plage de 300 à 700 nm, de préférence dans la plage de 350 à 600 nm
et/ou
la mise en réaction de polymères (P) contenant des groupes acides carboxyliques contenus dans le mélange, tels que définis dans la revendication 7 avec une composition de verre basique (G) contenue dans le mélange, telle que définie dans la revendication 1,
dans lequel l'acide carboxylique (C) α,β-insaturé est défini de préférence tel que dans la revendication 8
et/ou
dans lequel le poids moléculaire pondéré M_{w} de la quantité totale des polymères (P) contenant des groupes acides carboxyliques est de préférence défini tel que dans la revendication 8.

13. Utilisation d'un ou de plusieurs composés (S) tels que définis dans la revendication 1 ou de mélanges comprenant un ou plusieurs composés (S) tels que définis dans la revendication 1, pour
(i) augmenter la stabilité au stockage d'une composition comprenant
- un ou plusieurs composés (A) tels que définis dans la revendication 1,
- un ou plusieurs monomères (M) organiques radicalement polymérisables et
- une composition de verre basique (G) telle que définie dans la revendication 1
et/ou
(ii) fabriquer une composition selon l'une quelconque des revendications 1 à 6.

14. Utilisation selon la revendication 13,
dans laquelle le ou au moins un des plusieurs composés (S) est choisi parmi le groupe défini dans la revendication 3
et/ou
dans laquelle le ou au moins un des plusieurs composés (A) est choisi parmi le groupe défini dans la revendication 3
et/ou
dans laquelle le ou au moins un des plusieurs monomères (M) organiques radicalement polymérisables est choisi parmi les groupes définis dans la revendication 3
et/ou
dans laquelle le rapport entre la quantité de matière totale de tous les composés (A) et la quantité de matière totale de tous les composés (S) est défini tel que dans la revendication 4
et/ou
dans laquelle le rapport entre le poids total combiné de tous les composés (A) et de tous les composés (S) et le poids total de tous les monomères (M) organiques radicalement polymérisables est tel que défini dans la revendication 4
et/ou
dans laquelle la fraction totale de monomères (M) organiques radicalement polymérisables dans la composition est telle que définie dans la revendication 4
et/ou
dans laquelle la fraction totale combinée de tous les composés (A) et de tous les composés (S) dans la composition est telle que définie dans la revendication 4.
